# EUROPEAN PATENT APPLICATION

(11) **EP 4 000 690 A1**
(43) Date of publication of application: **25.05.2022**
(21) Application number: 21203828.5
(22) Date of filing: 19.05.2017
(51) Int. Cl.: A61P 27/12

(54) **COMPOSITIONS AND METHODS FOR THE PREVENTION AND TREATMENT OF MITOCHONDRIAL MYOPATHIES**

(30) Priority: 19.05.2016 US 201662338777 P; 16.06.2016 US 201662351100 P; 16.09.2016 US 201662395903 P
(62) Divisional of application: 17800258.0
(71) Applicant: Stealth BioTherapeutics Inc., Needham, MA 02494 (US)
(72) Inventor: WILSON, D. Travis, Newton, 02461 (US)
(74) Representative: Barker Brettell LLP

(57) **Abstract**

The disclosure provides methods of preventing or treating mitochondrial myopathy in a mammalian subject, reducing risk factors associated with mitochondrial myopathy, and/or reducing the likelihood or severity of mitochondrial myopathy. The methods comprise administering to the subject an effective amount of an aromatic-cationic peptide.

## Description

### CROSS-REFERENCE TO RELATED APPLICATIONS

This application claims the benefit of priority to U.S. provisional application numbers 62/338,777, filed on May 19, 2016; 62/351,100, filed on June 16, 2016; and 62/395,903, filed on September 16, 2016. The content of each foregoing application is incorporated herein by reference in its entirety.

### TECHNICAL FIELD

The present technology relates generally to compositions and methods for preventing, ameliorating, or treating mitochondrial myopathies and/or reducing the severity of one or more risk factors, signs, or symptoms associated with mitochondrial myopathies. Additionally, the present technology relates to administering an effective amount of an aromatic-cationic peptide, such as D-Arg-2',6'-Dmt-Lys-Phe-NH₂, or a pharmaceutically acceptable salt thereof, to a subject suffering from or at risk for a mitochondrial myopathy.

### BACKGROUND

The following description is provided to assist the understanding of the reader. None of the information provided or references cited is admitted to be prior art to the compositions and methods disclosed herein.

Mitochondrial myopathies comprise a group of inherited disorders caused by mutations in either mitochondrial or nuclear DNA resulting in defective mitochondrial metabolism. The term mitochondrial myopathy is used as the descriptive term for a group of disorders where muscle disease is an important manifestation, albeit one that is rarely found in isolation. Mitochondrial myopathies may affect multiple organ systems and tissues. Most mitochondrial myopathies involve children who manifest the signs and symptoms of accelerated aging, including neurodegenerative diseases, stroke, blindness, hearing impairment, diabetes mellitus, and heart failure.

The clinical manifestations of mitochondrial myopathies are variable but often include muscle weakness and wasting, rhabdomyolysis, myoglobinuria, and exercise intolerance, including exercise-induced cramps and myalgia. In some individuals, the myopathy is most prominent in muscles that control movements of the eyes and eyelids. Two common consequences are the gradual paralysis of eye movements, called progressive external opthalmoplegia (PEO), which can be mild or result in complete paralysis of extraocular muscles, and drooping of the upper eyelids, called ptosis. Although PEO is a common manifestation, it is not an obligate finding in mitochondrial myopathies caused by mtDNA mutations. Mitochondrial myopathies can also cause weakness and wasting in other muscles of the face and neck, which can lead to slurred speech and difficulty with swallowing (dysphagia). Exercise intolerance, also referred to as exertional fatigue, can vary greatly among affected individuals. While some individuals may experience trouble with athletic activities, others might experience problems with everyday activities. Although the reason for the variability in clinical manifestations of mitochondrial myopathies caused by mtDNA mutations has yet to be uncovered, heteroplasmy, replicative segregation, and threshold effects of the mtDNA mutations are likely important factors in pathogenesis. If a threshold proportion of mitochondria in a cell is defective and if a threshold proportion of such cells within a tissue have defective mitochondria, symptoms of tissue or organ dysfunction can manifest. Segments of muscle cells where the proportion of mutant mtDNA exceeds the threshold level will show enzyme deficiency.

Morphological analyses of muscle biopsies obtained from patients with mitochondrial myopathy reveal certain typical alterations. The hallmark of mitochondrial myopathy is the ragged-red fiber (RRF), which is an abnormal muscle fiber exhibiting an accumulation of mitochondria that are stained red by the modified Gomori trichrome technique. The mitochondria of RRF are usually ultrastructurally abnormal and often contain paracrystalline inclusions. Many patients with ragged-red fibers often exhibit encephalomyopathy. However, the absence of ragged-red fibers in a biopsy does not exclude a mitochondrial etiology.

There is no known cure for mitochondrial myopathy. Very few treatments are available for patients suffering from mitochondrial myopathies. None of the available pharmacological treatments aimed at treating mitochondrial myopathies are able to completely ameliorate the disorder.

### SUMMARY

In one aspect, the present disclosure provides methods for treating or preventing mitochondrial myopathy, and/or treating or preventing the signs or symptoms of mitochondrial myopathy in a subject in need thereof by administering to the subject a therapeutically effective amount of an aromatic-cationic peptide such as D-Arg-2',6'-Dmt-Lys-Phe-NH₂ (MTP-131), or a pharmaceutically acceptable salt thereof, thereby resulting in the prevention or treatment of one or more signs or symptoms of mitochondrial myopathy. In some embodiments of the methods of the present technology, the pharmaceutically acceptable salt comprises acetate, tartrate, trifluoroacetate, or hydrochloride salt.

In some embodiments, the mitochondrial myopathy is selected the diseases and/or associated with the diseases or conditions that follow: Kearns-Sayre syndrome (KSS); Leber's hereditary optic neuropathy (LHON); Leigh syndrome (LS); MEGDEL Syndrome; mitochondrial DNA depletion syndrome (MDS); mitochondrial myopathy, encephalomyopathy, lactic acidosis, and stroke-like episodes (MELAS); myoclonus epilepsy with ragged-red fibers (MERRF); mitochondrial neurogastrointestinal encephalomyopathy (MNGIE); neuropathy, ataxia and retinitis pigmentosa (NARP); *OPA1* mutations; Pearson syndrome; and progressive external ophthalmoplegia (PEO).

Additionally, or alternatively, in some embodiments, mitochondrial myopathies are a result of point mutations in tRNA genes selected from the group consisting of: tRNA^{Leu} (T3250C; A3302G; A12320G, A3288G); tRNA^{Pro} (G15990A; A16002G; G15995A); tRNA^{Phe} (T618C; G622A); tRNA^{Met} (T4409C; T5543C); tRNA^{Ser} (G7497A; A7480G); tRNA^{Asp} (A7526G); tRNA^{Gln} (4366insA); tRNA^{Ala}; tRNA^{Glu} (T14709C); tRNA^{Trp} (G5521A); and tRNA^{Tyr}.

Additionally, or alternatively, in some embodiments, mitochondrial myopathies are a result of one or more point mutations in mtDNA selected from the group consisting of: G15243A, T9185C, G3421A, G10197A, T12148C, and G6570A.

In some embodiments, mitochondrial myopathies can also include myopathies selected from the group consisting of: Swedish type myopathy with exercise intolerance; combined mitochondrial complex deficiency; familial myalgia syndrome; myopathy with abnormal mitochondrial translation; myopathy with extrapyramidal signs; myopathy with focal depletion of mitochondria; mitochondrial DNA breakage syndrome; limb-girdle muscular dystrophy type IH (LGMD1H); and isolated mitochondrial myopathy (IMMD).

In some embodiments of the methods of the present technology, the signs or symptoms of mitochondrial myopathy include one or more of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, muscle atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy.

In some embodiments of the method, the subject displays abnormal levels of one or more energy biomarkers compared to a normal control subject. In some embodiments, the energy biomarker is selected from the group consisting of lactic acid (lactate) levels; pyruvic acid (pyruvate) levels; lactate/pyruvate ratios; phosphocreatine levels; NADH (NADH+H⁺) or NADPH (NADPH+H⁺) levels; NAD or NADP levels; ATP levels; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels; beta-hydroxy butyrate levels; acetoacetate/ beta-hydroxy butyrate ratio; 8-hydroxy-2'-deoxyguanosine (8-OHdG) levels; levels of reactive oxygen species; oxygen consumption (VO₂), carbon dioxide output (VCO₂), respiratory quotient (VCO₂/VO₂), and to modulate exercise intolerance/tolerance and to modulate anaerobic threshold. In some embodiments of the method, the lactate levels of one or more of whole blood, plasma, cerebrospinal fluid, or cerebral ventricular fluid are abnormal compared to a normal control subject. In some embodiments of the method, the pyruvate levels of one or more of whole blood, plasma, cerebrospinal fluid, or cerebral ventricular fluid are abnormal compared to a normal control subject. In some embodiments of the method, the lactate/pyruvate ratios of one or more of whole blood, plasma, cerebrospinal fluid, or cerebral ventricular fluid are abnormal compared to a normal control subject.

In some embodiments of the methods of the present technology, the aromatic-cationic peptide is administered orally, intranasally, intrathecally, intraocularly, intradermally, transmucosally, iontophoretically, topically, systemically, intravenously, subcutaneously, intraperitoneally, or intramuscularly.

In some embodiments of the methods of the present technology, the aromatic-cationic peptide is administered daily for 1 week or more. In some embodiments of the methods of the present technology, the aromatic-cationic peptide is administered daily for 2 weeks or more. In some embodiments of the methods of the present technology, the aromatic-cationic peptide is administered daily for 3 weeks or more. In some embodiments of the methods of the present technology, the aromatic-cationic peptide is administered daily for 4 weeks or more. In some embodiments of the methods of the present technology, the aromatic-cationic peptide is administered daily for 6 weeks or more. In some embodiments of the methods of the present technology, the aromatic-cationic peptide is administered daily for 12 weeks or more.

In some embodiments, in addition to administration of aromatic-cationic peptides, the method further comprises separately, sequentially or simultaneously administering an additional therapeutic agent to the subject, wherein the additional therapeutic agent is selected from the group consisting of: creatine, L-carnitine, coenzyme Q₁₀, L-arginine, biotin, cytochrome c, corticosteroids, idebenone, sodium dichloroacetate, thiamine, thiocitic acid, riboflavin, α-tocopherol, succinate, ascorbate, menadione, naphthoquinone, and nicotinamide.

In some embodiments of the methods of the present technology, the aromatic-cationic peptide is administered orally, intranasally, intrathecally, intraocularly, intradermally, transmucosally, iontophoretically, topically, systemically, intravenously, subcutaneously, intraperitoneally, or intramuscularly.

In one aspect, the disclosure provides a method of treating or preventing mitochondrial myopathy in a mammalian subject, comprising administering to said mammalian subject a therapeutically effective amount of an aromatic-cationic peptide. In some embodiments, the aromatic-cationic peptide is a peptide having:
at least one net positive charge;
a minimum of four amino acids;
a maximum of about twenty amino acids;
a relationship between the minimum number of net positive charges (pₘ) and the total number of amino acid residues (r) wherein 3pₘ is the largest number that is less than or equal to r + 1; and a relationship between the minimum number of aromatic groups (a) and the total number of net positive charges (pₜ) wherein 2a is the largest number that is less than or equal to pₜ + 1, except that when a is 1, pₜ may also be 1. In particular embodiments, the mammalian subject is a human.

In one embodiment, 2pₘ is the largest number that is less than or equal to r+1, and may be equal to pₜ. The aromatic-cationic peptide may be a water-soluble peptide having a minimum of two or a minimum of three positive charges.

In one embodiment, the peptide comprises one or more non-naturally occurring amino acids, for example, one or more D-amino acids. In some embodiments, the C-terminal carboxyl group of the amino acid at the C-terminus is amidated. In certain embodiments, the peptide has a minimum of four amino acids. The peptide may have a maximum of about 6, a maximum of about 9, or a maximum of about 12 amino acids.

In one embodiment, the peptide may have the formula 2',6'-dimethyl-Tyr-D-Arg-Phe-Lys-NH₂, Phe-D-Arg-Phe-Lys-NH₂, D-Arg-2',6'-Dmt-Lys-Phe-NH₂, or 2',6'-Dmp-D-Arg-Phe-Lys-NH₂. In a particular embodiment, the aromatic-cationic peptide has the formula D-Arg-2',6' -Dmt-Lys-Phe-NH₂.

In one embodiment the peptide is defined by Formula I: 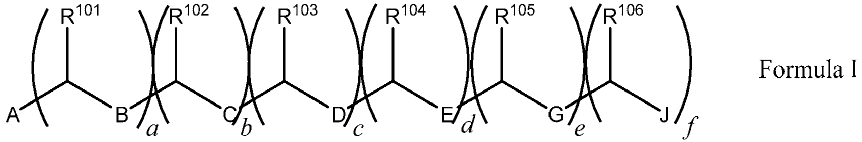 wherein:
one of A and J is 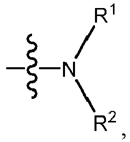
and the other of A and J is 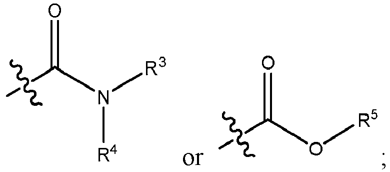
B, C, D, E, and G are each 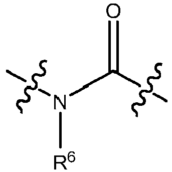 or B, C, D, E, and G are each 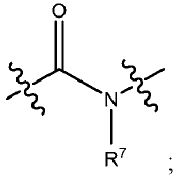 with the proviso that when
   f is 0 and J is not a terminal group, the terminal group is one of G, E, D or C, such that
   one of A and the terminal group is 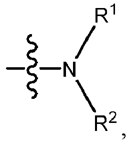 and the other of A and the terminal group is 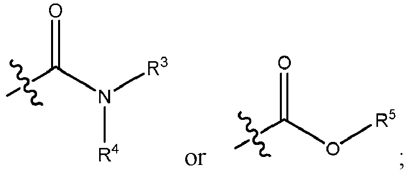
R¹⁰¹ is 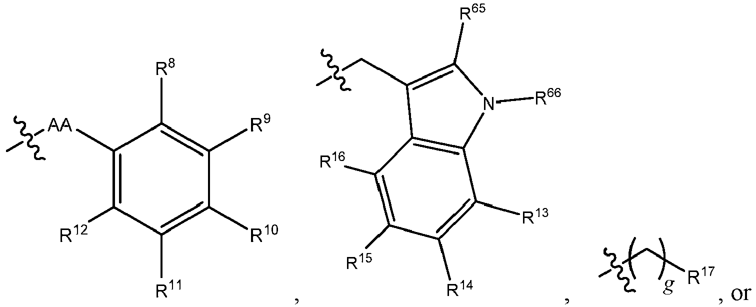 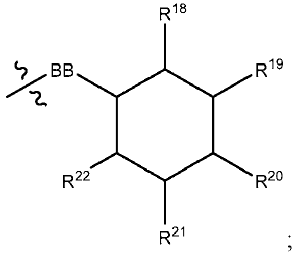
R¹⁰² is 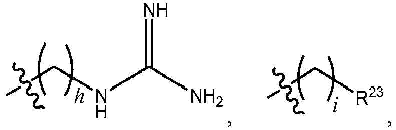 or hydrogen;
R¹⁰³ is 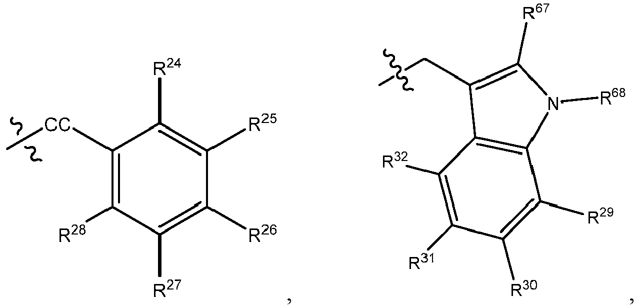 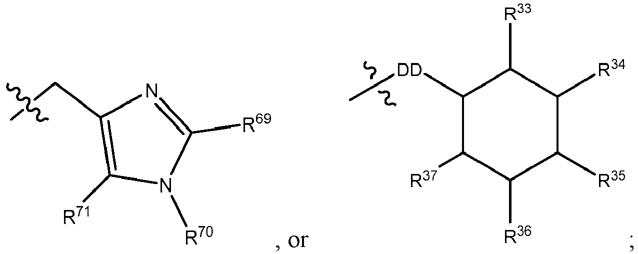
R¹⁰⁴ is 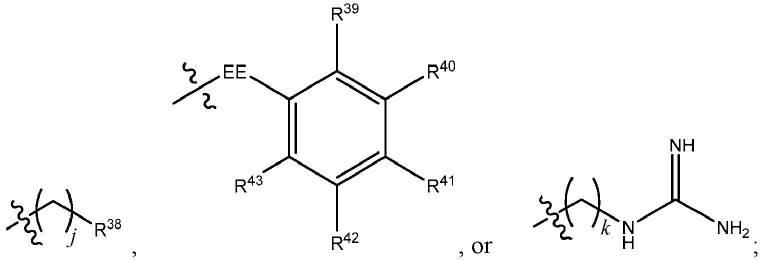
R¹⁰⁵ is 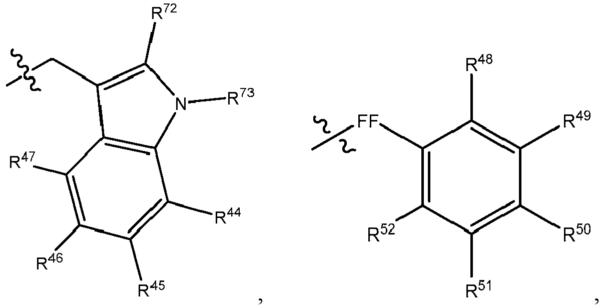 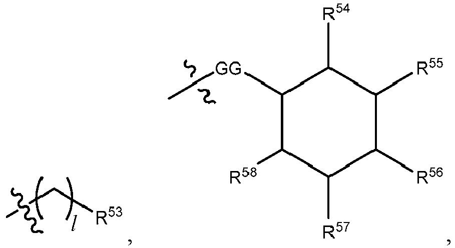 or hydrogen;
R¹⁰⁶ is 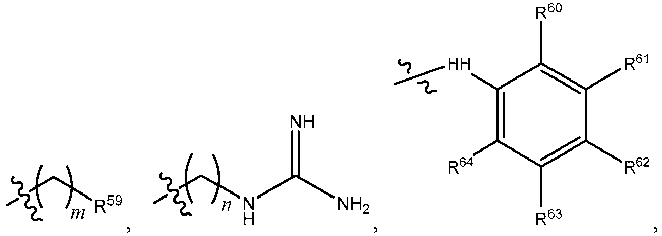 or hydrogen; provided that when R¹⁰², R¹⁰⁴, and R¹⁰⁶ are identical, then R¹⁰¹, R¹⁰³, and R¹⁰⁵ are not identical;
   wherein
   R¹, R², R³, R⁴, and R⁵ are each independently a hydrogen or substituted or unsubstituted C₁-C₆ alkyl, C₂-C₆ alkenyl, C₂-C₆ alkynyl, saturated or unsaturated cycloalkyl, cycloalkylalkyl, aryl, aralkyl, 5- or 6- membered saturated or unsaturated heterocylyl, heteroaryl, or amino protecting group; or R¹ and R² together form a 3, 4, 5, 6, 7, or 8 membered substituted or unsubstituted heterocycyl ring;
   R⁶ and R⁷ at each occurrence are independently a hydrogen or substituted or unsubstituted C₁-C₆ alkyl group;
   R⁸, R⁹, R¹⁰, R¹¹, R¹², R¹³, R¹⁴, R¹⁵, R¹⁶, R¹⁸, R¹⁹, R²⁰, R²¹, R²², R²⁴, R²⁵, R²⁶, R²⁷, R²⁸, R²⁹, R³⁰, R³¹, R³², R³³, R³⁴, R³⁵, R³⁶, R³⁷, R³⁹, R⁴⁰, R⁴¹, R⁴², R⁴³, R⁴⁴, R⁴⁵, R⁴⁶, R⁴⁷, R⁴⁸, R⁴⁹, R⁵⁰, R⁵¹, R⁵², R⁵⁴, R⁵⁵, R⁵⁶, R⁵⁷, R⁵⁸, R⁶⁰, R⁶¹, R⁶², R⁶³, R⁶⁴, R⁶⁵, R⁶⁷, R⁶⁹, R⁷¹, and R⁷² are each independently a hydrogen, amino, amido, -NO₂,-CN, -OR^{a}, -SR^{a}, -NR^{a}R^{a}, -F, -Cl, -Br, -I, or a substituted or unsubstituted C₁-C₆ alkyl, C₁-C₆ alkoxy, -C(O)-alkyl, -C(O)-aryl, - C(O)-aralkyl, -C(O)₂R^{a}, C₁-C₄ alkylamino, C₁-C₄ dialkylamino, or perhaloalkyl group;
   R⁶⁶, R⁶⁸, R⁷⁰, and R⁷³ are each independently a hydrogen or substituted or unsubstituted C₁-C₆ alkyl group;
   R¹⁷, R²³, R³⁸, R⁵³, and R⁵⁹ are each independently a hydrogen, -OR^{a},-SR^{a}, -NR^{a}R^{a}, -NR^{a}R^{b}, -CO₂R^{a}, -(CO)NR^{a}R^{a}, -NR^{a}(CO)R^{a}, -NR^{a}C(NH)NH₂, -NR^{a}-dansyl, or a substituted or unsubstituted alkyl, aryl, or aralkyl group;
   AA, BB, CC, DD, EE, FF, GG, and HH are each independently absent, -NH(CO)-, or -CH₂₋;
   R^{a} at each occurrence is independently a hydrogen or a substituted or unsubstituted C₁-C₆ alkyl group;
   R^{b} at each occurrence is independently a C₁-C₆ alkylene-NR^{a}-dansyl or C₁-C₆ alkylene-NR^{a}-anthraniloyl group;
   *a, b, c, d, e,* and *f* are each independently 0 or 1, with the proviso that *a* + *b* + *c* + *d* + *e* + *f ≥* 2;
   *g, h, k, m,* and *n* are each independently 1, 2, 3, 4, or 5; and
   *i,j,* and *l* are each independently 2, 3, 4, or 5;
   provided that
      when *i* is 4 and R²³ is -SR^{a}, or *j* is 4 and R³⁸ is -SR^{a}, or *l* is 4 and R⁵³ is -SR^{a}, then the R^{a} of the -SR^{a} is a substituted or unsubstituted C₁-C₆ alkyl group;
      when J is -NH₂, *b* and *d* are 0, *a, c, e, f* are 1, then R¹⁰³ is

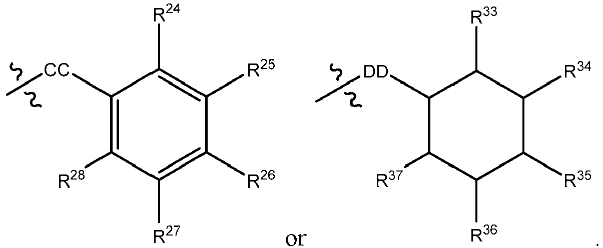

In some embodiments of peptides of Formula I,
R¹, R², R³, R⁴, and R⁵ are each independently a hydrogen or substituted or unsubstituted C₁-C₆ alkyl group;
R⁶ and R⁷ are each independently a hydrogen or methyl group;
R⁸, R¹², R¹⁸, R²², R²⁴, R²⁸, R³³, R³⁷, R³⁹, R⁴³, R⁴⁸, R⁵², R⁵⁴, R⁵⁸, R⁶⁰, and R⁶⁴ are each independently a hydrogen or methyl group;
R¹⁰, R²⁰, R²⁶, R³⁵, R⁴¹, R⁵⁰, R⁵⁶, and R⁶² are each independently a hydrogen or -OR^{a};
R⁹, R¹¹, R¹⁹, R²¹,R²⁵, R²⁷, R³⁴, R³⁶, R⁴⁰, R⁴², R⁴⁹, R⁵¹, R⁵⁵,R⁵⁷, R⁶¹, R⁶³, R⁶⁵, R⁶⁶, R⁶⁷, R⁶⁸, R⁶⁹, R⁷⁰, R⁷¹, R⁷², and R⁷³ are each a hydrogen;
R¹⁷, R²³, R³⁸, R⁵³, and R⁵⁹ are each independently a hydrogen, -OH, -SH, -SCH₃,-NH₂, -NHR^{b}, -CO₂H, -(CO)NH₂, -NH(CO)H, or -NH-dansyl group;
AA, BB, CC, DD, EE, FF, GG, and HH are each independently absent or -CH₂₋;
R^{a} at each occurrence is independently a hydrogen or a substituted or unsubstituted C₁-C₆ alkyl group;
R^{b} at each occurrence is independently an ethylene-NH-dansyl or ethylene-NH-anthraniloyl group.

In certain embodiments of Formula I,
A is 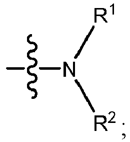
J is 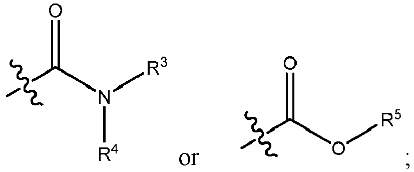
B, C, D, E, and G are each independently absent, 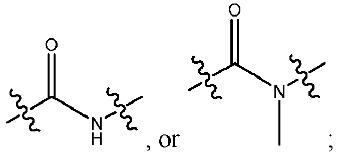
   with the proviso when *f* is 0, G is 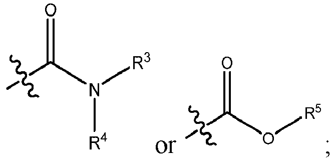 when *e* and *f*
   are 0, E is 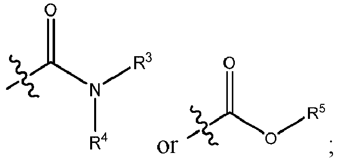 when *d, e,* and *f* are 0, D is 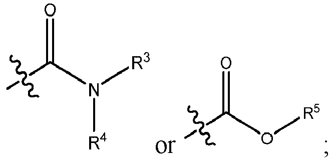 and when c, *d, e,* and *f* are 0, C is

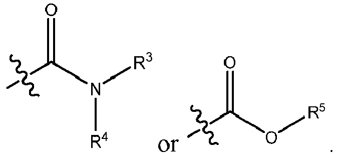

In another embodiment of Formula I,
A is 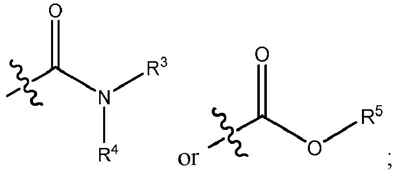
J is 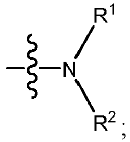
B, C, D, E, and G are each independently absent, 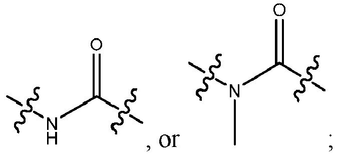
   with the proviso when *f* is 0, G is 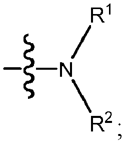 when *e* and *f* are 0, E is 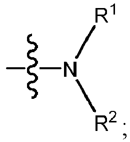 when *d, e,* and *f* are 0, D is 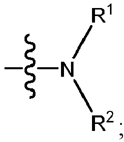 and when *c*, *d, e,* and *f* are
   0, C is

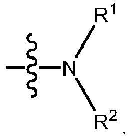

In some embodiments of Formula I, at least one of R¹⁰¹, R¹⁰², R¹⁰⁴, R¹⁰⁵, and R¹⁰⁶ is a basic group, as defined above, and at least one of R¹⁰¹, R¹⁰³, R¹⁰⁴, R¹⁰⁵, and R¹⁰⁶ is a neutral group as defined above. In some such embodiments, the neutral group is an aromatic, heterocyclic or cycloalkyl group as defined above. In some embodiments of Formula I, the peptide contains at least one arginine, such as, but not limited to D-arginine, and at least one 2',6'-dimethyltyrosine, tyrosine, or phenylalanine. In some embodiments of Formula I, R¹⁰¹ is an alkylguanidinium group.

In some embodiments, the aromatic-cationic peptides of the present technology have a core structural motif of alternating aromatic and cationic amino acids. For example, the peptide may be a tetrapeptide defined by any of Formulas A to F set forth below:
Aromatic - Cationic - Aromatic - Cationic (Formula A)
Cationic - Aromatic - Cationic - Aromatic (Formula B)
Aromatic - Aromatic - Cationic - Cationic (Formula C)
Cationic - Cationic - Aromatic - Aromatic (Formula D)
Aromatic - Cationic - Cationic - Aromatic (Formula E)
Cationic - Aromatic - Aromatic - Cationic (Formula F)
wherein, Aromatic is a residue selected from the group consisting of: Phe (F), Tyr (Y), and Trp (W). In some embodiments, the Aromatic residue may be substituted with a saturated analog of an aromatic residue, *e.g*., Cyclohexylalanine (Cha). In some embodiments, Cationic is a residue selected from the group consisting of: Arg (R), Lys (K), and His (H).

The aromatic-cationic peptides may be administered in a variety of ways. In some embodiments, the peptides may be administered orally, intranasally, intrathecally, intraocularly, intradermally, transmucosally, iontophoretically, topically, systemically, intravenously, subcutaneously, intraperitoneally, or intramuscularly.

In another aspect, the disclosure provides methods for improving skeletal muscle function is a subject in need thereof comprising administering to the subject a therapeutically effective amount of the peptide D-Arg-2',6'-Dmt-Lys-Phe-NH₂ or a pharmaceutically acceptable salt thereof, thereby resulting in the improvement skeletal muscle function, wherin the subject is an elderly subject. In some embodiments, the improvement in skeletal muscle function is characterized by an increase in ATP production.

Aspects or embodiments of the invention may also be provided according to the following paragraphs:
1. A method for treating or preventing mitochondrial myopathy in a subject in need thereof, comprising administering to the subject a therapeutically effective amount of the peptide D-Arg-2',6'-Dmt-Lys-Phe-NH₂ or a pharmaceutically acceptable salt thereof, thereby resulting in the prevention or treatment of mitochondrial myopathy.
2. The method of paragraph 1, wherein the peptide is administered daily for 2 weeks or more.
3. The method of paragraph 1, wherein the peptide is administered daily for 12 weeks or more.
4. The method of any one of paragraphs 1-3, wherein the subject has been diagnosed as having mitochondrial myopathy.
5. The method of paragraph 4, wherein the mitochondrial myopathy is selected from the group consisting of Kearns-Sayre syndrome (KSS); MEGDEL Syndrome; mitochondrial DNA depletion syndrome (MDS); mitochondrial myopathy, encephalomyopathy, lactic acidosis, and stroke-like episodes (MELAS); myoclonus epilepsy with ragged-red fibers (MERRF); mitochondrial neurogastrointestinal encephalomyopathy (MNGIE); neuropathy, ataxia and retinitis pigmentosa (NARP); *OPA1* mutations; Pearson syndrome; and progressive external ophthalmoplegia (PEO).
6. The method of paragraph 1, wherein the signs or symptoms of mitochondrial myopathy comprise one or more symptoms selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, muscle atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy.
7. The method of paragraph 1, wherein the subject displays abnormal levels of one or more energy biomarkers compared to a normal control subject.
8. The method of paragraph 7, wherein the energy biomarker is selected from the group consisting of lactic acid (lactate) levels; pyruvic acid (pyruvate) levels; lactate/pyruvate ratios; phosphocreatine levels; NADH (NADH+H⁺) or NADPH (NADPH+H⁺) levels; NAD or NADP levels; ATP levels; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels; beta-hydroxy butyrate levels; acetoacetate/ beta-hydroxy butyrate ratio; 8-hydroxy-2'-deoxyguanosine (8-OHdG) levels; levels of reactive oxygen species; oxygen consumption (VO₂), carbon dioxide output (VCO₂), and respiratory quotient (VCO₂/VO₂).
9. The method of any one of paragraphs 1-8, wherein the subject is human.
10. The method of any one of paragraphs 1-9, wherein the peptide is administered orally, intranasally, intrathecally, intraocularly, intradermally, transmucosally, iontophoretically, topically, systemically, intravenously, subcutaneously, intraperitoneally, or intramuscularly.
11. The method of any one of paragraphs 1-10, further comprising separately, sequentially, or simultaneously administering an additional therapeutic agent to the subj ect.
12. The method of paragraph 11, wherein the additional therapeutic agent is selected from the group consisting of: creatine, L-carnitine, coenzyme Q₁₀, L-arginine, biotin, cytochrome c, corticosteroids, idebenone, sodium dichloroacetate, thiamine, thiocitic acid, riboflavin, α-tocopherol, succinate, ascorbate, menadione, naphthoquinone, and nicotinamide.
13. The method of paragraphs 11 or 12, wherein the combination of peptide and an additional therapeutic agent has a synergistic effect in the prevention or treatment of mitochondrial myopathy.
14. The method of any one of paragraphs 1-13, wherein the pharmaceutically acceptable salt comprises acetate, tartrate, trifluoroacetate, or hydrochloride salt.
15. A method for reducing the risk of mitochondrial myopathy in a subject in need thereof, the method comprising: administering to the subject a therapeutically effective amount of the peptide D-Arg-2',6'-Dmt-Lys-Phe-NH₂ or a pharmaceutically acceptable salt thereof, thereby resulting in the prevention or delayed onset of mitochondrial myopathy.
16. The method of paragraph 15, wherein the peptide is administered daily for 2 weeks or more.
17. The method of paragraph 15, wherein the peptide is administered daily for 12 weeks or more.
18. The method of any one of paragraphs 15-17, wherein the mitochondrial myopathy is selected from the group consisting of: Kearns-Sayre syndrome (KSS); MEGDEL Syndrome; mitochondrial DNA depletion syndrome (MDS); mitochondrial myopathy, encephalomyopathy, lactic acidosis, and stroke-like episodes (MELAS); myoclonus epilepsy with ragged-red fibers (MERRF); mitochondrial neurogastrointestinal encephalomyopathy (MNGIE); neuropathy, ataxia and retinitis pigmentosa (NARP); *OPA1* mutations; Pearson syndrome; and progressive external ophthalmoplegia (PEO).
19. The method of any one of paragraphs 15-18, wherein the subject is human.
20. The method of any one of paragraphs 15-19, wherein the peptide is administered orally, intranasally, intrathecally, intraocularly, intradermally, transmucosally, iontophoretically, topically, systemically, intravenously, subcutaneously, intraperitoneally, or intramuscularly.
21. The method of any one of paragraphs 15-20, further comprising separately, sequentially, or simultaneously administering an additional therapeutic agent to the subj ect.
22. The method of paragraph 21, wherein the additional therapeutic agent is selected from the group consisting of: creatine, L-carnitine, coenzyme Q₁₀, L-arginine, biotin, cytochrome c, corticosteroids, idebenone, sodium dichloroacetate, thiamine, thiocitic acid, riboflavin, α-tocopherol, succinate, ascorbate, menadione, naphthoquinone, and nicotinamide.
23. The method of paragraphs 21 or 22, wherein the combination of peptide and an additional therapeutic agent has a synergistic effect in reducing the risk of mitochondrial myopathy.
24. The method of any one of paragraphs 15-23, wherein the pharmaceutically acceptable salt comprises acetate, tartrate, trifluoroacetate, or hydrochloride salt.
25. A method for improving skeletal muscle function is a subject in need thereof comprising administering to the subject a therapeutically effective amount of the peptide D-Arg-2',6'-Dmt-Lys-Phe-NH₂ or a pharmaceutically acceptable salt thereof, thereby resulting in the improvement skeletal muscle function, wherin the subject is an elderly subject.
26. The method of paragraph 25, wherein the improvement in skeletal muscle function is characterized by an increase in ATP production.

### BRIEF DESCRIPTION OF THE DRAWINGS

Figure 1 is a table showing the subjects from the mitochondrial myopathy study disclosed in Example 1. The table shows the mitochondrial myopathies or the diseases/disorder causing the mitochondrial myopathy of each subject, the genetic mutation causing the mitochondrial myopathy, and the treatment group to which the subject was randomized.
Figure 2 is a graph showing the increase in distance travelled in the 6 Minute Walk Test (6MWT) by subjects treated with 0.25 mg/kg/hr of D-Arg-2',6'-Dmt-Lys-Phe-NH₂ (*i.e.,* Group 3) as compared to the control group (*i.e.,* Group 4, untreated subjects).
Figure 3 is a graph showing that the subjects treated with D-Arg-2',6'-Dmt-Lys-Phe-NH₂ (*i.e*., Groups 1-3) showed a significant dose dependent increase in distance traveled in the 6MWT as compared to the control group (*i.e.,* Group 4, untreated subjects).
Figure 4 shows a heterogeneous slope model for the female subjects enrolled in the trial.
Figure 5 shows the modified Newcastle Mitochondrial Disease Adult Score survey (NMDAS).
Figure 6 is a diagram showing exemplary clinical consequences of mitochondrial dysfunction as related to muscle physiology in the elderly.
Figure 7 is a chart showing the increase of ATPₘₐₓ from baseline in elderly subjects treated with D-Arg-2',6'-Dmt-Lys-Phe-NH₂ (MTP-131) as compared to elderly subjects treated placebo at 2 hours post-infusion and after 7 days post-infusion.
Figure 8 is a chart showing that the increase in ATPₘₐₓ from baseline in elderly subjects treated with D-Arg-2',6'-Dmt-Lys-Phe-NH₂ (MTP-131) as compared to elderly subjects treated placebo at 2 hours post-infusion is similar to the increase in ATPₘₐₓ from baseline in elderly subjects after 6 months of endurance training as compared to control elderly subjects.
Figures 9A-9B are graphs showing that aged mice treated with D-Arg-2',6'-Dmt-Lys-Phe-NH₂ (MTP-131) show increased ATPₘₐₓ (9A) and endurance capacity (9B). ** *p*<0.01 relative to age-matched control. ## *p*<0.01 relative to young control.
Figure 10 shows the Mitochondrial Disease Symptom Assessment survey (MDSA).
Figure 11 is a graph comparing the average distance walked during the 6-Minute Walk Test at the end of treatment between subjects treated with D-Arg-2',6'-Dmt-Lys-Phe-NH₂ treated and subjects treated with placebo.
Figure 12A is a graph showing Total Fatigue as measure by MDSA for D-Arg-2',6'-Dmt-Lys-Phe-NH₂ treated and placebo treated subjects.
Figure 12B is a graph showing Total Fatigue during Activities as measure by MDSA for D-Arg-2',6'-Dmt-Lys-Phe-NH₂ treated and placebo treated subjects.

### DETAILED DESCRIPTION

It is to be appreciated that certain aspects, modes, embodiments, variations and features of the present technology are described below in various levels of detail in order to provide a substantial understanding of the present technology. The definitions of certain terms as used in this specification are provided below. Unless defined otherwise, all technical and scientific terms used herein generally have the same meaning as commonly understood by one of ordinary skill in the art to which this present technology belongs.

As used in this specification and the appended claims, the singular forms "a", "an" and "the" include plural referents unless the content clearly dictates otherwise. For example, reference to "a cell" includes a combination of two or more cells, and the like.

As used herein, the "administration" of an agent, drug, or peptide to a subject includes any route of introducing or delivering to a subject a compound to perform its intended function. Administration can be carried out by any suitable route, including orally, intranasally, intrathecally, intraocularly, intradermally, transmucosally, iontophoretically, topically, systemically, intravenously, subcutaneously, intraperitoneally, or intramuscularly. Administration includes self-administration and the administration by another.

As used herein, the term "amino acid" includes naturally-occurring amino acids and synthetic amino acids, as well as amino acid analogs and amino acid mimetics that function in a manner similar to the naturally-occurring amino acids. Naturally-occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally-occurring amino acid, *i.e.,* an α-carbon that is bound to a hydrogen, a carboxyl group, an amino group, and an R group, *e.g*., homoserine, norleucine, methionine sulfoxide, methionine methyl sulfonium. Such analogs have modified R groups (*e.g*., norleucine) or modified peptide backbones, but retain the same basic chemical structure as a naturally-occurring amino acid. Amino acid mimetics refers to chemical compounds that have a structure that is different from the general chemical structure of an amino acid, but that functions in a manner similar to a naturally-occurring amino acid. Amino acids can be referred to herein by either their commonly known three letter symbols or by the one-letter symbols recommended by the IUPAC-IUB Biochemical Nomenclature Commission.

As used herein, the term "effective amount" refers to a quantity sufficient to achieve a desired therapeutic and/or prophylactic effect, *e.g.,* an amount which results in partial or full amelioration of one or more symptoms of mitochondrial myopathies. In the context of therapeutic or prophylactic applications, in some embodiments, the amount of a composition administered to the subject will depend on the type, degree, and severity of the disease and on the characteristics of the individual, such as general health, age, sex, body weight and tolerance to drugs. The skilled artisan will be able to determine appropriate dosages depending on these and other factors. The compositions can also be administered in combination with one or more additional therapeutic compounds. In the methods described herein, aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, may be administered to a subject having one or more signs, symptoms, or risk factors of mitochondrial myopathy selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, muscle atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy. For example, a "therapeutically effective amount" of the aromatic-cationic peptides includes levels at which the presence, frequency, or severity of one or more signs, symptoms, or risk factors of mitochondrial myopathy are reduced or eliminated. In some embodiments, a therapeutically effective amount reduces or ameliorates the physiological effects of mitochondrial myopathy, and/or the risk factors of mitochondrial myopathy, and/or the likelihood of developing mitochondrial myopathy. In some embodiments, an effective amount of a compound is an amount of the compound sufficient to modulate, normalize, or enhance one or more energy biomarkers (where modulation, normalization, and enhancement are defined herein).

As used herein, the terms "enhancement" of, or to "enhance," energy biomarkers means to improve the level of one or more energy biomarkers in a direction that results in a beneficial or desired physiological outcome in a subject (compared to the values observed in a normal control subject, or the value in the subject prior to treatment with a composition or compound). For example, in a situation where significant energy demands are placed on a subject, it may be desirable to increase the level of ATP in that subject to a level above the ATP level observed in a normal control subject. Enhancement can also be of beneficial effect in a subject suffering from a disease or pathology such as a mitochondrial myopathy, in that normalizing an energy biomarker may not achieve the optimum outcome for the subject; in such cases, enhancement of one or more energy biomarkers can be beneficial, for example, higher-than-normal levels of ATP, or lower-than normal levels of lactic acid (lactate) can be beneficial to such a subject.

As used herein, "isolated" or "purified" polypeptide or peptide refers to a polypeptide or peptide that is substantially free of cellular material or other contaminating polypeptides from the cell or tissue source from which the agent is derived, or substantially free from chemical precursors or other chemicals when chemically synthesized. For example, an isolated aromatic-cationic peptide would be free of materials that would interfere with diagnostic or therapeutic uses of the agent. Such interfering materials may include enzymes, hormones and other proteinaceous and nonproteinaceous solutes.

As used herein, the terms "mitochondrial myopathy" or "mitochondrial myopathies" refer to a group of inherited disorders caused by mutations in either mitochondrial or nuclear DNA resulting in defective mitochondrial metabolism, and are selected from the group consisting of Kearns-Sayre syndrome (KSS); Leber's hereditary optic neuropathy (LHON); Leigh syndrome (LS); MEGDEL Syndrome; mitochondrial DNA depletion syndrome (MDS); mitochondrial myopathy, encephalomyopathy, lactic acidosis, and stroke-like episodes (MELAS); myoclonus epilepsy with ragged-red fibers (MERRF); mitochondrial neurogastrointestinal encephalomyopathy (MNGIE); neuropathy, ataxia and retinitis pigmentosa (NARP); *OPA1* mutations; Pearson syndrome; and progressive external ophthalmoplegia (PEO); Swedish type myopathy with exercise intolerance; combined mitochondrial complex deficiency; familial myalgia syndrome; myopathy with abnormal mitochondrial translation; myopathy with extrapyramidal signs; myopathy with focal depletion of mitochondria; mitochondrial DNA breakage syndrome; limb-girdle muscular dystrophy type IH (LGMD1H); and isolated mitochondrial myopathy (IMMD). In some embodiments, mitochondrial myopathies result from point mutations in tRNA genes selected from the group consisting of: tRNA^{Leu} (T3250C; A3302G; A12320G, A3288G); tRNA^{Pro} (G15990A; A16002G; G15995A); tRNA^{Phe} (T618C; G622A); tRNA^{Met} (T4409C; T5543C); tRNA^{Ser} (G7497A; A7480G); tRNA^{Asp} (A7526G); tRNA^{Gln} (4366insA); tRNA^{Ala}_{;} tRNA^{Glu} (T14709C); tRNA^{Trp} (G5521A); and tRNA^{Tyr}. In some embodiments, mitochondrial myopathies result from one or more point mutations in mtDNA selected from the group consisting of: G15243A, T9185C, G3421A, G10197A, T12148C, and G6570A.

As used herein, the "modulation" of, or to "modulate," an energy biomarker means to change the level of the energy biomarker towards a desired value, or to change the level of the energy biomarker in a desired direction (*e.g*., increase or decrease). Modulation can include, but is not limited to, normalization and enhancement as defined herein.

As used herein, the term "net charge" refers to the balance of the number of positive charges and the number of negative charges carried by the amino acids present in the aromatic-cationic peptides of the present technology. In this specification, it is understood that net charges are measured at physiological pH. The naturally occurring amino acids that are positively charged at physiological pH include L-lysine, L-arginine, and L-histidine. The naturally occurring amino acids that are negatively charged at physiological pH include L-aspartic acid and L-glutamic acid.

As used herein, the term "non-naturally-occurring" refers to a composition which is not found in this form in nature. A non-naturally-occurring composition can be derived from a naturally-occurring composition, *e.g.,* as non-limiting examples, *via* purification, isolation, concentration, chemical modification (*e.g*., addition or removal of a chemical group), and/or, in the case of mixtures, addition or removal of ingredients or compounds. Alternatively, a non-naturally-occurring composition can comprise or be derived from a non-naturally-occurring combination of naturally-occurring compositions. Thus, a non-naturally-occurring composition can comprise a mixture of purified, isolated, modified and/or concentrated naturally-occurring compositions, and/or can comprise a mixture of naturally-occurring compositions in forms, concentrations, ratios and/or levels of purity not found in nature.

As used herein, the terms "normalization" of, or to "normalize," an energy biomarker is defined as changing the level of the energy biomarker from a pathological value towards a normal value, where the normal value of the energy biomarker can be 1) the level of the energy biomarker in a healthy person or subject, or 2) a level of the energy biomarker that alleviates one or more undesirable symptoms in the person or subject. That is, to normalize an energy biomarker which is depressed in a disease state means to increase the level of the energy biomarker towards the normal (healthy) value or towards a value which alleviates an undesirable symptom; to normalize an energy biomarker which is elevated in a disease state means to decrease the level of the energy biomarker towards the normal (healthy) value or towards a value which alleviates an undesirable symptom.

As used herein, the terms "polypeptide," "peptide," and "protein" are used interchangeably herein to mean a polymer comprising two or more amino acids joined to each other by peptide bonds or modified peptide bonds, *i.e.,* peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art.

As used herein, "prevention" or "preventing" of a disorder or condition refers to a compound that, in a statistical sample, reduces the occurrence of the disorder or condition in the treated sample relative to an untreated control sample, or delays the onset of one or more symptoms of the disorder or condition relative to the untreated control sample. As used herein, preventing mitochondrial myopathies includes preventing or delaying the initiation of mitochondrial myopathies. As used herein, prevention of mitochondrial myopathies also includes preventing a recurrence of one or more signs or symptoms of mitochondrial myopathies.

As used herein, the term "separate" therapeutic use refers to an administration of at least two active ingredients at the same time or at substantially the same time by different routes.

As used herein, the term "sequential" therapeutic use refers to administration of at least two active ingredients at different times, the administration route being identical or different. More particularly, sequential use refers to the whole administration of one of the active ingredients before administration of the other or others commences. It is thus possible to administer one of the active ingredients over several minutes, hours, or days before administering the other active ingredient or ingredients. There is no simultaneous treatment in this case.

As used herein, the term "simultaneous" therapeutic use refers to the administration of at least two active ingredients by the same route and at the same time or at substantially the same time.

As used herein, the terms "subject," "individual," or "patient" can be an individual organism, a vertebrate, a mammal, or a human.

In general, "substituted" refers to an organic group as defined below (*e.g.,* an alkyl group) in which one or more bonds to a hydrogen atom contained therein are replaced by a bond to non-hydrogen or non-carbon atoms. Substituted groups also include groups in which one or more bonds to a carbon(s) or hydrogen(s) atom are replaced by one or more bonds, including double or triple bonds, to a heteroatom. Thus, a substituted group is substituted with one or more substituents, unless otherwise specified. In some embodiments, a substituted group is substituted with 1, 2, 3, 4, 5, or 6 substituents. Examples of substituent groups include: halogens (*i.e.,* F, Cl, Br, and I); hydroxyls; alkoxy, alkenoxy, aryloxy, aralkyloxy, heterocyclyl, heterocyclylalkyl, heterocyclyloxy, and heterocyclylalkoxy groups; carbonyls (oxo); carboxylates; esters; urethanes; oximes; hydroxylamines; alkoxyamines; aralkoxyamines; thiols; sulfides; sulfoxides; sulfones; sulfonyls; pentafluorosulfanyl (*i.e.,* SF₅), sulfonamides; amines; N-oxides; hydrazines; hydrazides; hydrazones; azides; amides; ureas; amidines; guanidines; enamines; imides; isocyanates; isothiocyanates; cyanates; thiocyanates; imines; nitro groups; nitriles (*i.e.,* CN); and the like.

Substituted ring groups such as substituted cycloalkyl, aryl, heterocyclyl and heteroaryl groups also include rings and ring systems in which a bond to a hydrogen atom is replaced with a bond to a carbon atom. Therefore, substituted cycloalkyl, aryl, heterocyclyl and heteroaryl groups may also be substituted with substituted or unsubstituted alkyl, alkenyl, and alkynyl groups as defined below.

Alkyl groups include straight chain and branched chain alkyl groups having from 1 to 12 carbon atoms, and typically from 1 to 10 carbons or, in some embodiments, from 1 to 8, 1 to 6, or 1 to 4 carbon atoms. Examples of straight chain alkyl groups include groups such as methyl, ethyl, n-propyl, n-butyl, n-pentyl, n-hexyl, n-heptyl, and n-octyl groups. Examples of branched alkyl groups include, but are not limited to, isopropyl, iso-butyl, sec-butyl, tert-butyl, neopentyl, isopentyl, and 2,2-dimethylpropyl groups. Representative substituted alkyl groups may be substituted one or more times with substituents such as those listed above, and include without limitation haloalkyl (*e.g*., trifluoromethyl), hydroxyalkyl, thioalkyl, aminoalkyl, alkylaminoalkyl, dialkylaminoalkyl, alkoxyalkyl, carboxyalkyl, and the like.

Cycloalkyl groups include mono-, bi- or tricyclic alkyl groups having from 3 to 12 carbon atoms in the ring(s), or, in some embodiments, 3 to 10, 3 to 8, or 3 to 4, 5, or 6 carbon atoms. Exemplary monocyclic cycloalkyl groups include, but not limited to, cyclopropyl, cyclobutyl, cyclopentyl, cyclohexyl, cycloheptyl, and cyclooctyl groups. In some embodiments, the cycloalkyl group has 3 to 8 ring members, whereas in other embodiments the number of ring carbon atoms range from 3 to 5, 3 to 6, or 3 to 7. Bi- and tricyclic ring systems include both bridged cycloalkyl groups and fused rings, such as, but not limited to, bicyclo[2.1.1]hexane, adamantyl, decalinyl, and the like. Substituted cycloalkyl groups may be substituted one or more times with, non-hydrogen and non-carbon groups as defined above. However, substituted cycloalkyl groups also include rings that are substituted with straight or branched chain alkyl groups as defined above. Representative substituted cycloalkyl groups may be mono-substituted or substituted more than once, such as, but not limited to, 2,2-, 2,3-, 2,4- 2,5- or 2,6-disubstituted cyclohexyl groups, which may be substituted with substituents such as those listed above.

Cycloalkylalkyl groups are alkyl groups as defined above in which a hydrogen or carbon bond of an alkyl group is replaced with a bond to a cycloalkyl group as defined above. In some embodiments, cycloalkylalkyl groups have from 4 to 16 carbon atoms, 4 to 12 carbon atoms, and typically 4 to 10 carbon atoms. Substituted cycloalkylalkyl groups may be substituted at the alkyl, the cycloalkyl or both the alkyl and cycloalkyl portions of the group. Representative substituted cycloalkylalkyl groups may be mono-substituted or substituted more than once, such as, but not limited to, mono-, di- or tri-substituted with substituents such as those listed above.

Alkenyl groups include straight and branched chain alkyl groups as defined above, except that at least one double bond exists between two carbon atoms. Alkenyl groups have from 2 to 12 carbon atoms, and typically from 2 to 10 carbons or, in some embodiments, from 2 to 8, 2 to 6, or 2 to 4 carbon atoms. In some embodiments, the alkenyl group has one, two, or three carbon-carbon double bonds. Examples include, but are not limited to vinyl, allyl, -CH=CH(CH₃), -CH=C(CH₃)₂, -C(CH₃)=CH₂, -C(CH₃)=CH(CH₃), -C(CH₂CH₃)=CH₂, among others. Representative substituted alkenyl groups may be mono-substituted or substituted more than once, such as, but not limited to, mono-, di- or tri-substituted with substituents such as those listed above.

Cycloalkenyl groups include cycloalkyl groups as defined above, having at least one double bond between two carbon atoms. In some embodiments the cycloalkenyl group may have one, two or three double bonds but does not include aromatic compounds. Cycloalkenyl groups have from 4 to 14 carbon atoms, or, in some embodiments, 5 to 14 carbon atoms, 5 to 10 carbon atoms, or even 5, 6, 7, or 8 carbon atoms. Examples of cycloalkenyl groups include cyclohexenyl, cyclopentenyl, cyclohexadienyl, cyclobutadienyl, and cyclopentadienyl.

Cycloalkenylalkyl groups are alkyl groups as defined above in which a hydrogen or carbon bond of the alkyl group is replaced with a bond to a cycloalkenyl group as defined above. Substituted cycloalkenylalkyl groups may be substituted at the alkyl, the cycloalkenyl or both the alkyl and cycloalkenyl portions of the group. Representative substituted cycloalkenylalkyl groups may be substituted one or more times with substituents such as those listed above.

Alkynyl groups include straight and branched chain alkyl groups as defined above, except that at least one triple bond exists between two carbon atoms. Alkynyl groups have from 2 to 12 carbon atoms, and typically from 2 to 10 carbons or, in some embodiments, from 2 to 8, 2 to 6, or 2 to 4 carbon atoms. In some embodiments, the alkynyl group has one, two, or three carbon-carbon triple bonds. Examples include, but are not limited to-C=CH, -C=CCH₃, -CH₂C=CCH₃, -C=CCH₂CH(CH₂CH₃)₂, among others. Representative substituted alkynyl groups may be mono-substituted or substituted more than once, such as, but not limited to, mono-, di- or tri-substituted with substituents such as those listed above.

Aryl groups are cyclic aromatic hydrocarbons that do not contain heteroatoms. Aryl groups herein include monocyclic, bicyclic and tricyclic ring systems. Thus, aryl groups include, but are not limited to, phenyl, azulenyl, heptalenyl, biphenyl, fluorenyl, phenanthrenyl, anthracenyl, indenyl, indanyl, pentalenyl, and naphthyl groups. In some embodiments, aryl groups contain 6-14 carbons, and in others from 6 to 12 or even 6-10 carbon atoms in the ring portions of the groups. In some embodiments, the aryl groups are phenyl or naphthyl. Although the phrase "aryl groups" includes groups containing fused rings, such as fused aromatic-aliphatic ring systems (*e.g*., indanyl, tetrahydronaphthyl, and the like), it does not include aryl groups that have other groups, such as alkyl or halo groups, bonded to one of the ring members. Rather, groups such as tolyl are referred to as substituted aryl groups. Representative substituted aryl groups may be mono-substituted or substituted more than once. For example, monosubstituted aryl groups include, but are not limited to, 2-, 3-, 4-, 5-, or 6-substituted phenyl or naphthyl groups, which may be substituted with substituents such as those listed above.

Aralkyl groups are alkyl groups as defined above in which a hydrogen or carbon bond of an alkyl group is replaced with a bond to an aryl group as defined above. In some embodiments, aralkyl groups contain 7 to 16 carbon atoms, 7 to 14 carbon atoms, or 7 to 10 carbon atoms. Substituted aralkyl groups may be substituted at the alkyl, the aryl or both the alkyl and aryl portions of the group. Representative aralkyl groups include but are not limited to benzyl and phenethyl groups and fused (cycloalkylaryl)alkyl groups such as 4-indanylethyl. Representative substituted aralkyl groups may be substituted one or more times with substituents such as those listed above.

Heterocyclyl groups include aromatic (also referred to as heteroaryl) and non-aromatic ring compounds containing 3 or more ring members, of which one or more is a heteroatom such as, but not limited to, N, O, and S. In some embodiments, the heterocyclyl group contains 1, 2, 3 or 4 heteroatoms. In some embodiments, heterocyclyl groups include mono-, bi- and tricyclic rings having 3 to 16 ring members, whereas other such groups have 3 to 6, 3 to 10, 3 to 12, or 3 to 14 ring members. Heterocyclyl groups encompass aromatic, partially unsaturated and saturated ring systems, such as, for example, imidazolyl, imidazolinyl and imidazolidinyl groups. The phrase "heterocyclyl group" includes fused ring species including those comprising fused aromatic and non-aromatic groups, such as, for example, benzotriazolyl, 2,3-dihydrobenzo[1,4]dioxinyl, and benzo[1,3]dioxolyl. The phrase also includes bridged polycyclic ring systems containing a heteroatom such as, but not limited to, quinuclidyl. However, the phrase does not include heterocyclyl groups that have other groups, such as alkyl, oxo or halo groups, bonded to one of the ring members. Rather, these are referred to as "substituted heterocyclyl groups". Heterocyclyl groups include, but are not limited to, aziridinyl, azetidinyl, pyrrolidinyl, imidazolidinyl, pyrazolidinyl, thiazolidinyl, tetrahydrothiophenyl, tetrahydrofuranyl, dioxolyl, furanyl, thiophenyl, pyrrolyl, pyrrolinyl, imidazolyl, imidazolinyl, pyrazolyl, pyrazolinyl, triazolyl, tetrazolyl, oxazolyl, isoxazolyl, thiazolyl, thiazolinyl, isothiazolyl, thiadiazolyl, oxadiazolyl, piperidyl, piperazinyl, morpholinyl, thiomorpholinyl, tetrahydropyranyl, tetrahydrothiopyranyl, oxathiane, dioxyl, dithianyl, pyranyl, pyridyl, pyrimidinyl, pyridazinyl, pyrazinyl, triazinyl, dihydropyridyl, dihydrodithiinyl, dihydrodithionyl, homopiperazinyl, quinuclidyl, indolyl, indolinyl, isoindolyl,azaindolyl (pyrrolopyridyl), indazolyl, indolizinyl, benzotriazolyl, benzimidazolyl, benzofuranyl, benzothiophenyl, benzthiazolyl, benzoxadiazolyl, benzoxazinyl, benzodithiinyl, benzoxathiinyl, benzothiazinyl, benzoxazolyl, benzothiazolyl, benzothiadiazolyl, benzo[1,3]dioxolyl, pyrazolopyridyl, imidazopyridyl (azabenzimidazolyl), triazolopyridyl, isoxazolopyridyl, purinyl, xanthinyl, adeninyl, guaninyl, quinolinyl, isoquinolinyl, quinolizinyl, quinoxalinyl, quinazolinyl, cinnolinyl, phthalazinyl, naphthyridinyl, pteridinyl, thianaphthyl, dihydrobenzothiazinyl, dihydrobenzofuranyl, dihydroindolyl, dihydrobenzodioxinyl, tetrahydroindolyl, tetrahydroindazolyl, tetrahydrobenzimidazolyl, tetrahydrobenzotriazolyl, tetrahydropyrrolopyridyl, tetrahydropyrazolopyridyl, tetrahydroimidazopyridyl, tetrahydrotriazolopyridyl, and tetrahydroquinolinyl groups. Representative substituted heterocyclyl groups may be mono- substituted or substituted more than once, such as, but not limited to, pyridyl or morpholinyl groups, which are 2-, 3-, 4-, 5-, or 6-substituted, or disubstituted with various substituents such as those listed above.

Heteroaryl groups are aromatic ring compounds containing 5 or more ring members, of which, one or more is a heteroatom such as, but not limited to, N, O, and S. Heteroaryl groups include, but are not limited to, groups such as pyrrolyl, pyrazolyl, triazolyl, tetrazolyl, oxazolyl, isoxazolyl, thiazolyl, pyridinyl, pyridazinyl, pyrimidinyl, pyrazinyl, thiophenyl, benzothiophenyl, furanyl, benzofuranyl, indolyl, azaindolyl (pyrrolopyridinyl), indazolyl, benzimidazolyl, imidazopyridinyl (azabenzimidazolyl), pyrazolopyridinyl, triazolopyridinyl, benzotriazolyl, benzoxazolyl, benzothiazolyl, benzothiadiazolyl, imidazopyridinyl, isoxazolopyridinyl, thianaphthyl, purinyl, xanthinyl, adeninyl, guaninyl, quinolinyl, isoquinolinyl, tetrahydroquinolinyl, quinoxalinyl, and quinazolinyl groups. Heteroaryl groups include fused ring compounds in which all rings are aromatic such as indolyl groups and include fused ring compounds in which only one of the rings is aromatic, such as 2,3-dihydro indolyl groups. Although the phrase "heteroaryl groups" includes fused ring compounds, the phrase does not include heteroaryl groups that have other groups bonded to one of the ring members, such as alkyl groups. Rather, heteroaryl groups with such substitution are referred to as "substituted heteroaryl groups." Representative substituted heteroaryl groups may be substituted one or more times with various substituents such as those listed above.

Heterocyclylalkyl groups are alkyl groups as defined above in which a hydrogen or carbon bond of an alkyl group is replaced with a bond to a heterocyclyl group as defined above. Substituted heterocyclylalkyl groups may be substituted at the alkyl, the heterocyclyl or both the alkyl and heterocyclyl portions of the group. Representative heterocyclyl alkyl groups include, but are not limited to, morpholin-4-yl-ethyl, furan-2-yl-methyl, imidazol-4-yl-methyl, pyridin-3-yl-methyl, tetrahydrofuran-2-yl-ethyl, and indol-2-yl-propyl. Representative substituted heterocyclylalkyl groups may be substituted one or more times with substituents such as those listed above.

Heteroaralkyl groups are alkyl groups as defined above in which a hydrogen or carbon bond of an alkyl group is replaced with a bond to a heteroaryl group as defined above. Substituted heteroaralkyl groups may be substituted at the alkyl, the heteroaryl or both the alkyl and heteroaryl portions of the group. Representative substituted heteroaralkyl groups may be substituted one or more times with substituents such as those listed above.

Groups described herein having two or more points of attachment (*i.e.,* divalent, trivalent, or polyvalent) within the compound of the present technology are designated by use of the suffix, "ene." For example, divalent alkyl groups are alkylene groups, divalent aryl groups are arylene groups, divalent heteroaryl groups are divalent heteroarylene groups, and so forth. Substituted groups having a single point of attachment to the compound of the present technology are not referred to using the "ene" designation. Thus, e.g., chloroethyl is not referred to herein as chloroethylene.

Alkoxy groups are hydroxyl groups (-OH) in which the bond to the hydrogen atom is replaced by a bond to a carbon atom of a substituted or unsubstituted alkyl group as defined above. Examples of linear alkoxy groups include but are not limited to methoxy, ethoxy, propoxy, butoxy, pentoxy, hexoxy, and the like. Examples of branched alkoxy groups include but are not limited to isopropoxy, sec-butoxy, tert-butoxy, isopentoxy, isohexoxy, and the like. Examples of cycloalkoxy groups include but are not limited to cyclopropyloxy, cyclobutyloxy, cyclopentyloxy, cyclohexyloxy, and the like. Representative substituted alkoxy groups may be substituted one or more times with substituents such as those listed above.

As used herein, a "synergistic therapeutic effect" refers to a greater-than-additive therapeutic effect which is produced by a combination of at least two agents, and which exceeds that which would otherwise result from the individual administration of the agents. For example, lower doses of one or more agents may be used in treating a disease or disorder, resulting in increased therapeutic efficacy and decreased side-effects.

As used herein, a "therapeutically effective amount" of a compound refers to compound levels in which the physiological effects of a disease or disorder are, at a minimum, ameliorated. A therapeutically effective amount can be given in one or more administrations. The amount of a compound which constitutes a therapeutically effective amount will vary depending on the compound, the disorder and its severity, and the general health, age, sex, body weight and tolerance to drugs of the subject to be treated, but can be determined routinely by one of ordinary skill in the art.

As used herein, the terms "treating" or "treatment" or "alleviation" refers to therapeutic treatment, wherein the object is to reduce, alleviate or slow down the progression or advancement of, and/or reverse the progression of the targeted pathological condition or disorder. A subject is successfully "treated" for mitochondrial myopathy if, after receiving a therapeutic amount of the aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, according to the methods described herein, the subject shows observable and/or measurable reduction in or absence of one or more signs and symptoms of mitochondrial myopathy selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, muscle atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy.

It is also to be appreciated that the various modes of treatment or prevention of medical conditions as described herein are intended to mean "substantial," which includes total but also less than total treatment or prevention, and wherein some biologically or medically relevant result is achieved.

### Aromatic-Cationic Peptides

The aromatic-cationic peptides of the present technology preferably include a minimum of three amino acids, covalently joined by peptide bonds.

The maximum number of amino acids present in the aromatic-cationic peptides of the present technology is about twenty amino acids covalently joined by peptide bonds. In some embodiments, the total number of amino acids is about twelve. In some embodiments, the total number of amino acids is about nine. In some embodiments, the total number of amino acids is about six. In some embodiments, the total number of amino acids is four.

In some aspects, the present technology provides an aromatic-cationic peptide or a pharmaceutically acceptable salt thereof such as acetate salt, tartrate salt, fumarate salt, hydrochloride salt, or trifluoroacetate salt. In some embodiments, the peptide comprises at least one net positive charge; a minimum of three amino acids; a maximum of about twenty amino acids; a relationship between the minimum number of net positive charges (pₘ) and the total number of amino acid residues (r) wherein 3pₘ is the largest number that is less than or equal to r + 1; and a relationship between the minimum number of aromatic groups (a) and the total number of net positive charges (pₜ) wherein 2a is the largest number that is less than or equal to pₜ + 1, except that when a is 1, pₜ may also be 1.

In some embodiments, the peptide is defined by Formula I: 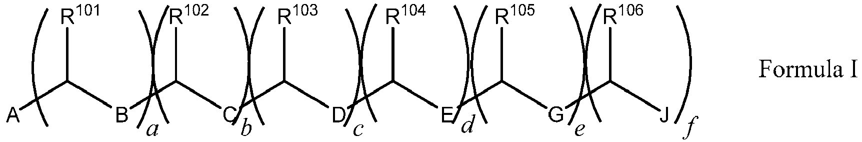 wherein:
one of A and J is 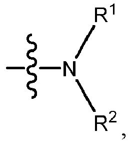
and the other of A and J is 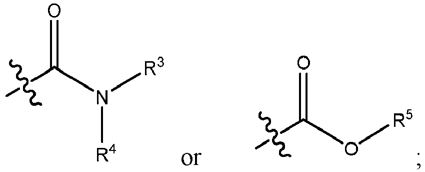
B, C, D, E, and G are each 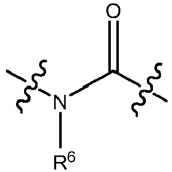 or B, C, D, E, and G are each 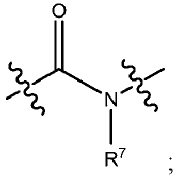 with the proviso that when
   f is 0 and J is not a terminal group, the terminal group is one of G, E, D or C, such that
   one of A and the terminal group is 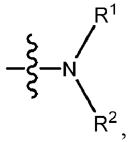 and
   the other of A and the terminal group is 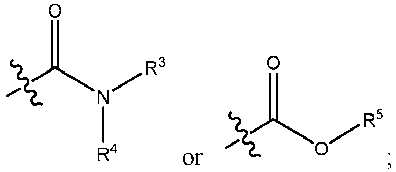
R¹⁰¹ is 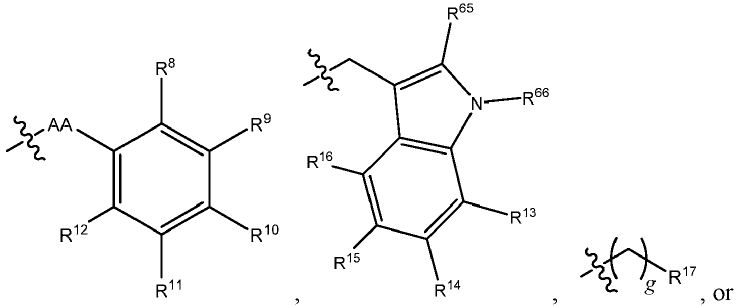 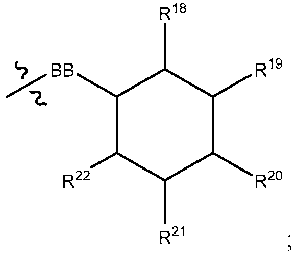 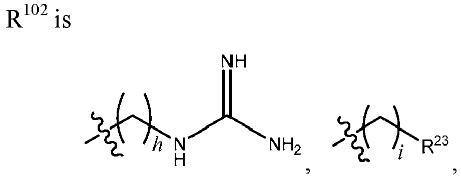 or hydrogen;
R¹⁰³ is 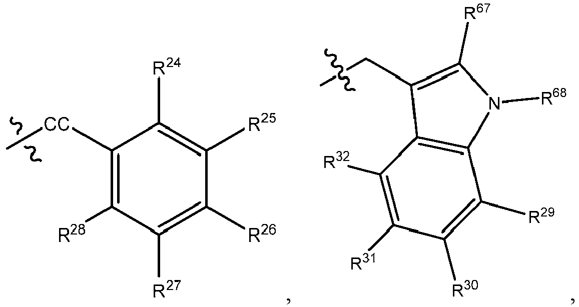 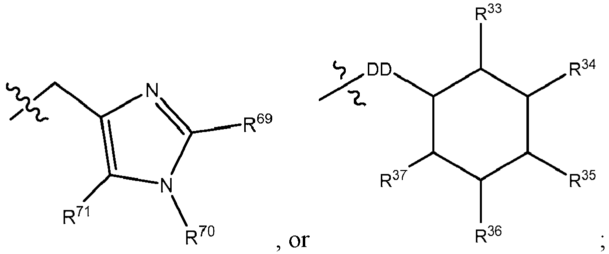
R¹⁰⁴ is 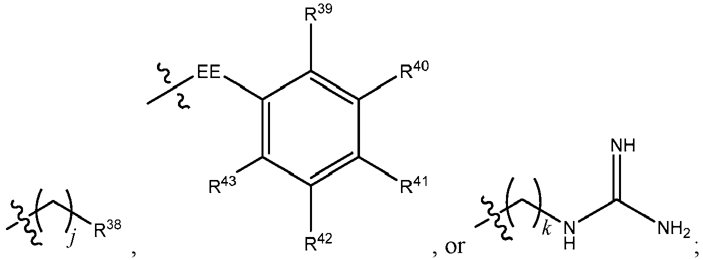
R¹⁰⁵ is 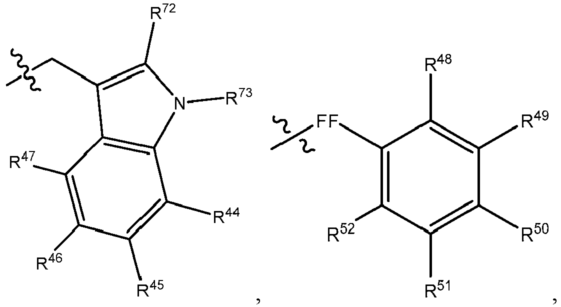 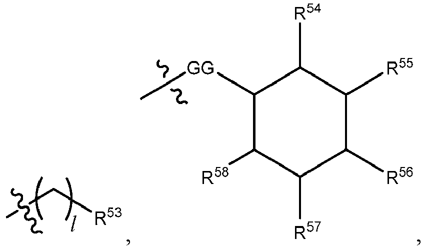 or hydrogen;
R¹⁰⁶ is 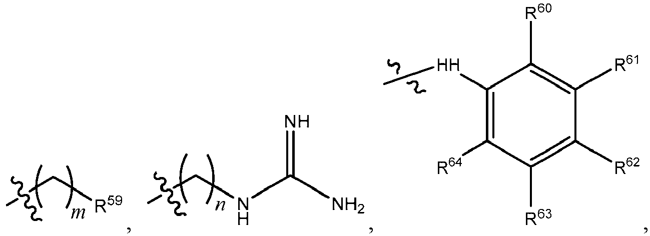 or hydrogen; provided that when R¹⁰², R¹⁰⁴, and R¹⁰⁶ are identical, then R¹⁰¹, R¹⁰³, and R¹⁰⁵ are not identical;
   wherein
   R¹, R², R³, R⁴, and R⁵ are each independently a hydrogen or substituted or unsubstituted C₁-C₆ alkyl, C₂-C₆ alkenyl, C₂-C₆ alkynyl, saturated or unsaturated cycloalkyl, cycloalkylalkyl, aryl, aralkyl, 5- or 6- membered saturated or unsaturated heterocylyl, heteroaryl, or amino protecting group; or R¹ and R² together form a 3, 4, 5, 6, 7, or 8 membered substituted or unsubstituted heterocycyl ring;
   R⁶ and R⁷ at each occurrence are independently a hydrogen or substituted or unsubstituted C₁-C₆ alkyl group;
   R⁸, R⁹, R¹⁰, R¹¹, R¹², R¹³, R¹⁴, R¹⁵, R¹⁶, R¹⁸, R¹⁹, R²⁰,R²¹, R²², R²⁴, R²⁵, R²⁶, R²⁷, R²⁸, R²⁹, R³⁰, R³¹, R³², R³³, R³⁴, R³⁵, R³⁶, R³⁷, R³⁹, R⁴⁰, R⁴¹, R⁴², R⁴³, R⁴⁴, R⁴⁵, R⁴⁶, R⁴⁷, R⁴⁸, R⁴⁹, R⁵⁰, R⁵¹, R⁵², R⁵⁴, R⁵⁵, R⁵⁶, R⁵⁷, R⁵⁸, R⁶⁰, R⁶¹, R⁶², R⁶³, R⁶⁴, R⁶⁵, R⁶⁷, R⁶⁹, R⁷¹, and R⁷² are each independently a hydrogen, amino, amido, -NO₂,-CN, -OR^{a}, -SR^{a}, -NR^{a}R^{a}, -F, -Cl, -Br, -I, or a substituted or unsubstituted C₁-C₆ alkyl, C₁-C₆ alkoxy, -C(O)-alkyl, -C(O)-aryl, - C(O)-aralkyl, -C(O)₂R^{a}, C₁-C₄ alkylamino, C₁-C₄ dialkylamino, or perhaloalkyl group;
   R⁶⁶, R⁶⁸, R⁷⁰, and R⁷³ are each independently a hydrogen or substituted or unsubstituted C₁-C₆ alkyl group;
   R¹⁷, R²³, R³⁸, R⁵³, and R⁵⁹ are each independently a hydrogen, -OR^{a},-SR^{a}, -NR^{a}R^{a}, -NR^{a}R^{b}, -CO₂R^{a}, -(CO)NR^{a}R^{a}, -NR^{a}(CO)R^{a}, -NR^{a}C(NH)NH₂, -NR^{a}-dansyl, or a substituted or unsubstituted alkyl, aryl, or aralkyl group;
   AA, BB, CC, DD, EE, FF, GG, and HH are each independently absent, -NH(CO)-, or -CH₂₋;
   R^{a} at each occurrence is independently a hydrogen or a substituted or unsubstituted C₁-C₆ alkyl group;
   R^{b} at each occurrence is independently a C₁-C₆ alkylene-NR^{a}-dansyl or C₁-C₆ alkylene-NR^{a}-anthraniloyl group;
   *a, b, c, d, e,* and *f* are each independently 0 or 1, with the proviso that *a* + *b* + c + *d* + *e* + *f ≥* 2;
   *g, h, k, m,* and *n* are each independently 1, 2, 3, 4, or 5; and
   *i, j,* and *l* are each independently 2, 3, 4, or 5;
   provided that
      when *i* is 4 and R²³ is -SR^{a}, or *j* is 4 and R³⁸ is -SR^{a}, or *l*is 4 and R⁵³ is -SR^{a}, then the R^{a} of the -SR^{a} is a substituted or unsubstituted C₁-C₆ alkyl group;
      when J is -NH₂, *b* and *d* are 0, *a, c, e, f* are 1, then R¹⁰³ is

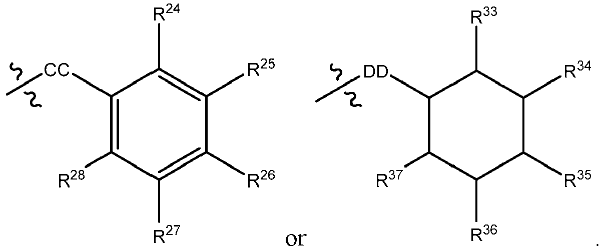

In some embodiments of peptides of Formula I,
R¹, R², R³, R⁴, and R⁵ are each independently a hydrogen or substituted or unsubstituted C₁-C₆ alkyl group;
R⁶ and R⁷ at each occurrence are independently a hydrogen or methyl group;
R⁸, R¹², R¹⁸, R²², R²⁴, R²⁸, R³³, R³⁷, R³⁹, R⁴³, R⁴⁸, R⁵², R⁵⁴, R⁵⁸, R⁶⁰, and R⁶⁴ are each independently a hydrogen or methyl group;
R¹⁰, R²⁰, R²⁶, R³⁵, R⁴¹, R⁵⁰, R⁵⁶, and R⁶² are each independently a hydrogen or -OR^{a};
R⁹, R¹¹, R¹⁹, R²¹,R²⁵, R²⁷, R³⁴, R³⁶, R⁴⁰, R⁴², R⁴⁹, R⁵¹, R⁵⁵, R⁵⁷, R⁶¹, R⁶³, R⁶⁵, R⁶⁶, R⁶⁷, R⁶⁸, R⁶⁹, R⁷⁰, R⁷¹, R⁷², and R⁷³ are each a hydrogen;
R¹⁷, R²³, R³⁸, R⁵³, and R⁵⁹ are each independently a hydrogen, -OH, -SH, -SCH₃,-NH₂, -NHR^{b}, -CO₂H, -(CO)NH₂, -NH(CO)H, or -NH-dansyl group;
AA, BB, CC, DD, EE, FF, GG, and HH are each independently absent or -CH₂₋;
R^{a} at each occurrence is independently a hydrogen or a substituted or unsubstituted C₁-C₆ alkyl group;
R^{b} at each occurrence is independently an ethylene-NH-dansyl or ethylene-NH-anthraniloyl group.

In some embodiments of Formula I,
A is 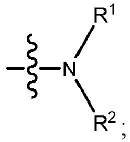
J is 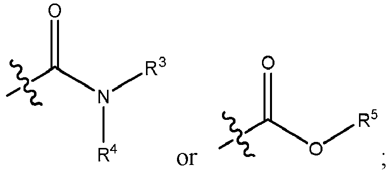
B, C, D, E, and G are each independently 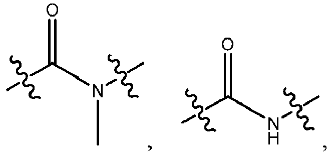 or absent;
   with the proviso when *f* is 0, G is 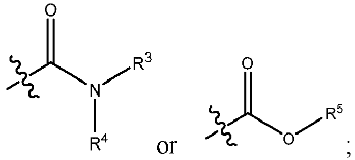
   when *e* and *f* are 0, E is 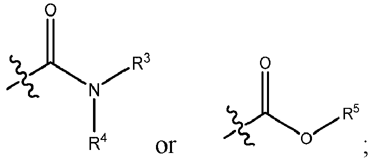
   when *d, e,* and *f* are 0, D is 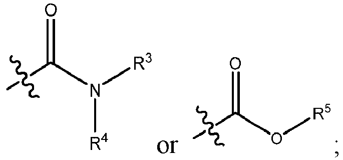 and
   when *c*, *d, e,* and *f* are 0, C is

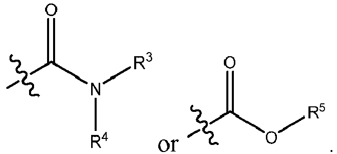

In another embodiment of Formula I,
A is 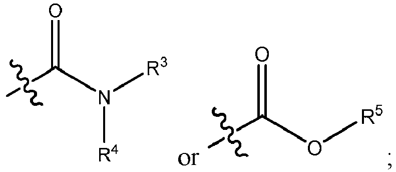
J is 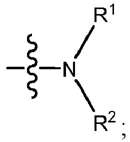
B, C, D, E, and G are each independently 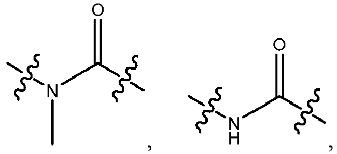 or absent;
   with the proviso when *f* is 0, G is 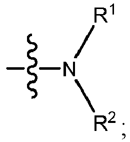
   when *e* and *f* are 0, E is 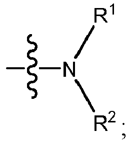
   when *d, e,* and *f* are 0, D is 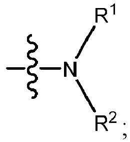 and
   when *c*, *d, e,* and *f* are 0, C is

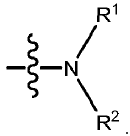

In some embodiments of Formula I, at least one of R¹⁰¹, R¹⁰², R¹⁰⁴, R¹⁰⁵, and R¹⁰⁶ is a basic group, as defined above, and at least one of R¹⁰¹, R¹⁰³, R¹⁰⁴, R¹⁰⁵, and R¹⁰⁶ is a neutral group as defined above. In some such embodiments, the neutral group is an aromatic, heterocyclic or cycloalkyl group as defined above. In some embodiments of Formula I, the peptide contains at least one arginine, such as, but not limited to D-arginine, and at least one 2',6'-dimethyltyrosine, tyrosine, or phenylalanine. In some embodiments of Formula I, R¹⁰¹ is an alkylguanidinium group.

In some embodiments, the peptide of the present technology is selected from the peptides shown in Tables A or B.

| **TABLE A** |
|---|
| Tyr-D-Arg-Phe-Lys-NH₂ |
| D-Arg-Dmt-Lys-Phe-NH₂ |
| D-Arg-Dmt-Phe-Lys-NH₂ |
| D-Arg-Phe-Lys-Dmt-NH₂ |
| D-Arg-Phe-Dmt-Lys-NH₂ |
| D-Arg-Lys-Dmt-Phe-NH₂ |
| D-Arg-Lys-Phe-Dmt-NH₂ |
| D-Arg-Dmt-Lys-Phe-Cys-NH₂ |
| Phe-Lys-Dmt-D-Arg-NH₂ |
| Phe-Lys-D-Arg-Dmt-NH₂ |
| Phe-D-Arg-Phe-Lys-NH₂ |
| Phe-D-Arg-Phe-Lys-Cys-NH₂ |
| Phe-D-Arg-Phe-Lys-Ser-Cys-NH₂ |
| Phe-D-Arg-Phe-Lys-Gly-Cys-NH₂ |
| Phe-D-Arg-Dmt-Lys-NH₂ |
| Phe-D-Arg-Dmt-Lys-Cys-NH₂ |
| Phe-D-Arg-Dmt-Lys-Ser-Cys-NH₂ |
| Phe-D-Arg-Dmt-Lys-Gly-Cys-NH₂ |
| Phe-D-Arg-Lys-Dmt-NH₂ |
| Phe-Dmt-D-Arg-Lys-NH₂ |
| Phe-Dmt-Lys-D-Arg-NH₂ |
| Lys-Phe-D-Arg-Dmt-NH₂ |
| Lys-Phe-Dmt-D-Arg-NH₂ |
| Lys-Dmt-D-Arg-Phe-NH₂ |
| Lys-Dmt-Phe-D-Arg-NH₂ |
| Lys-D-Arg-Phe-Dmt-NH₂ |
| Lys-D-Arg-Dmt-Phe-NH₂ |
| D-Arg-Dmt-D-Arg-Phe-NH₂ |
| D-Arg-Dmt-D-Arg-Dmt-NH₂ |
| D-Arg-Dmt-D-Arg-Tyr-NH₂ |
| D-Arg-Dmt-D-Arg-Trp-NH₂ |
| Trp-D-Arg-Tyr-Lys-NH₂ |
| Trp-D-Arg-Trp-Lys-NH₂ |
| Trp-D-Arg-Dmt-Lys-NH₂ |
| D-Arg-Trp-Lys-Phe-NH₂ |
| D-Arg-Trp-Phe-Lys-NH₂ |
| D-Arg-Trp-Lys-Dmt-NH₂ |
| D-Arg-Trp-Dmt-Lys-NH₂ |
| D-Arg-Lys-Trp-Phe-NH₂ |
| D-Arg-Lys-Trp-Dmt-NH₂ |
| Cha-D-Arg-Phe-Lys-NH₂ |
| Ala-D-Arg-Phe-Lys-NH₂ |
| 2',6'-Dmp-D-Arg-2',6'-Dmt-Lys-NH₂ |
| 2',6'-Dmp-D-Arg-Phe-Lys-NH₂ |
| 2',6'-Dmt-D-Arg-Phe-Orn-NH₂ |
| 26'-Dmt-D-Arg-Phe-Ahp-NH₂ |
| 2',6'-Dmt-D-Arg-Phe-Lys-NH₂ |
| 2',6'-Dmt-D-Cit-Phe-Lys-NH₂ |
| D-Arg-2',6'-Dmt-Lys-Phe-NH₂ |
| D-Tyr-Trp-Lys-NH₂ |
| Lys-D-Arg-Tyr-NH₂ |
| Met-Tyr-D-Arg-Phe-Arg-NH₂ |
| Met- Tyr-D-Lys-Phe-Arg |
| Phe-Arg-D-His-Asp |
| Phe-D-Arg-2',6'-Dmt-Lys-NH₂ |
| Phe-D-Arg-His |
| Trp-D-Lys-Tyr-Arg-NH₂ |
| Tyr-D-Arg-Phe-Lys-Glu-NH₂ |
| Tyr-His-D-Gly-Met |
| D-Arg-Tyr-Lys-Phe-NH₂ |
| D-Arg-*D*-Dmt-Lys-Phe-NH₂ |
| D-Arg-Dmt-*D*-Lys-Phe-NH₂ |
| D-Arg-Dmt-Lys-*D*-Phe-NH₂ |
| D-Arg-*D*-Dmt-*D*-Lys-*D*-Phe-NH₂ |
| Phe-*D*-Arg-*D*-Phe-Lys-NH₂ |
| Phe-*D*-Arg-Phe-*D*-Lys-NH₂ |
| D-Phe-*D*-Arg-*D*-Phe-*D*-Lys-NH₂ |
| Lys-*D*-Phe-Arg-Dmt-NH₂ |
| *D*-Arg-Arg-Dmt-Phe-NH₂ |
| Dmt-*D*-Phe-Arg-Lys-NH₂ |
| Phe-*D*-Dmt-Arg-Lys-NH₂ |
| *D*-Arg-Dmt-Lys-NH₂ |
| Arg-*D*-Dmt-Lys-NH₂ |
| *D*-Arg-Dmt-Phe-NH₂ |
| Arg-D-Dmt-Arg-NH₂ |
| Dmt-D-Arg-NH₂ |
| D-Arg-Dmt-NH₂ |
| D-Dmt-Arg-NH₂ |
| Arg-D-Dmt-NH₂ |
| D-Arg-D-Dmt-NH₂ |
| D-Arg-*D*-Tyr-Lys-Phe-NH₂ |
| D-Arg-Tyr-*D*-Lys-Phe-NH₂ |
| D-Arg-Tyr-Lys-*D*-Phe-NH₂ |
| D-Arg-*D*-Tyr-*D*-Lys-*D*-Phe-NH₂ |
| Lys-*D*-Phe-Arg-Tyr-NH₂ |
| *D*-Arg-Arg-Tyr-Phe-NH₂ |
| Tyr-*D*-Phe-Arg-Lys-NH₂ |
| Phe-*D*-Tyr-Arg-Lys-NH₂ |
| *D*-Arg-Tyr-Lys-NH₂ |
| Arg-*D*-Tyr-Lys-NH₂ |
| *D*-Arg-Tyr-Phe-NH₂ |
| Arg-*D*-Tyr-Arg-NH₂ |
| Tyr-*D*-Arg-NH₂ |
| *D*-Arg-Tyr-NH₂ |
| *D*-Tyr-Arg-NH₂ |
| Arg-*D*-Tyr-NH₂ |
| *D*-Arg-*D*-Tyr-NH₂ |
| Dmt-Lys-Phe-NH₂ |
| Lys-Dmt-*D*-Arg-NH₂ |
| Phe-Lys-Dmt-NH₂ |
| *D*-Arg-Phe-Lys-NH2 |
| *D*-Arg-Cha-Lys-NH₂ |
| *D*-Arg-Trp-Lys-NH₂ |
| Dmt-Lys-*D*-Phe-NH₂ |
| Dmt-Lys-NH₂ |
| Lys-Phe-NH₂ |
| *D*-Arg-Cha-Lys-Cha-NH₂ |
| *D*-Nle-Dmt-Ahp-Phe-NH₂ |
| *D*-Nle-Cha-Ahp-Cha-NH₂ |
| D-Arg-Dmt-D-Lys-NH₂ |
| D-Arg-Dmt-D-Lys-Phe-NH₂ |
| Lys-Trp-D-Arg-NH₂ |
| H-Lys-D-Phe-Arg-Dmt-NH₂ |
| H-D-Arg-Lys-Dmt-Phe-NH₂ |
| H-D-Arg-Lys-Phe-Dmt-NH₂ |
| H-D-Arg-Arg-Dmt-Phe-NH₂ |
| H-D-Arg-Dmt-Phe-Lys-NH₂ |
| H-D-Arg-Phe-Dmt-Lys-NH₂ |
| H-Dmt-D-Phe-Arg-Lys-NH₂ |
| H-Phe-D-Dmt-Arg-Lys-NH₂ |
| H-D-Arg-Dmt-Lys-NH₂ |
| H-D-Arg-Dmt-D-Lys-D-Phe-NH₂ |
| H-D-Arg-D-Dmt-Lys-Phe-NH₂ |
| H-D-Arg-Dmt-Phe-NH₂ |
| H-Dmt-D-Arg-NH₂ |
| H-Phe-D-Arg-D-Phe-Lys-NH₂ |
| H-Phe-D-Arg-Phe-D-Lys-NH₂ |
| H-D-Phe-D-Arg-D-Phe-D-Lys-NH₂ |
| H-D-Arg-D-Dmt-D-Lys-D-Phe-NH₂ |
| H-D-Arg-Cha-Lys-NH₂ |
| H-D-Arg-Cha-Lys-Cha-NH₂ |
| H-Arg-D-Dmt-Lys-NH₂ |
| H-Arg-D-Dmt-Arg-NH₂ |
| H-D-Dmt-Arg-NH₂ |
| H-Arg-D-Dmt-NH₂ |
| H-D-Arg-D-Dmt-NH₂ |
| Arg-Arg-Dmt-Phe |
| Arg-Cha-Lys |
| Arg-Dmt |
| Arg-Dmt-Arg |
| Arg-Dmt-Lys |
| Arg-Dmt-Lys-Phe |
| Arg-Dmt-Lys-Phe-Cys |
| Arg-Dmt-Phe |
| Arg-Dmt-Phe-Lys |
| Arg-Lys-Dmt-Phe |
| Arg-Lys-Phe-Dmt |
| Arg-Phe-Dmt-Lys |
| Arg-Phe-Lys |
| Arg-Trp-Lys |
| Arg-Tyr-Lys |
| Arg-Tyr-Lys-Phe |
| D-Arg-D-Dmt-D-Lys-L-Phe-NH₂ |
| D-Arg-D-Dmt-L-Lys-D-Phe-NH₂ |
| D-Arg-D-Dmt-L-Lys-L-Phe-NH₂ |
| D-Arg-Dmt-D-Lys-NH₂ |
| D-Arg-Dmt-Lys-NH₂ |
| D-Arg-Dmt-Lys-Phe-Cys |
| D-Arg-L-Dmt-D-Lys-D-Phe-NH₂ |
| D-Arg-L-Dmt-D-Lys-L-Phe-NH₂ |
| D-Arg-L-Dmt-L-Lys-D-Phe-NH₂ |
| Dmt-Arg |
| Dmt-Lys |
| Dmt-Lys-Phe |
| Dmt-Phe-Arg-Lys |
| H-Arg-D-Dmt-Lys-Phe-NH₂ |
| H-Arg-Dmt-Lys-Phe-NH₂ |
| H-D-Arg-2,6-dichloro-L-tyrosine-L-Lys-L-Phe-NH₂ |
| H-D-Arg-2,6-dichlorotyrosine-Lys-Phe-NH₂ |
| H-D-Arg-2,6-difluoro-L-tyrosine-L-Lys-L-Phe-NH₂ |
| H-D-Arg-2,6-difluorotyrosine-Lys-Phe-NH₂ |
| H-D-Arg-2,6-dimethyl-L-phenylalanine-L-Lys-L-Phe-NH₂ |
| H-D-Arg-2,6-dimethylphenylalanine-Lys-Phe-NH₂ |
| H-D-Arg-4-methoxy-2,6-dimethyl-L-phenylalanine-L-Lys-L-Phe-NH₂ |
| H-D-Arg-4-methoxy-2,6-dimethylphenylalanine-Lys-Phe-NH₂ |
| H-D-Arg-Dmt-Lys-2,6-dimethylphenylalanine-NH₂ |
| H-D-Arg-Dmt-Lys-3-hydroxyphenylalanine-NH₂ |
| H-D-Arg-Dmt-N6-acetyllysine-Phe-NH₂ |
| H-D-Arg-D-Phe-L-Lys-L-Phe-NH₂ |
| H-D-Arg-D-Trp-L-Lys-L-Phe-NH₂ |
| H-D-Arg-D-Tyr-L-Lys-L-Phe-NH₂ |
| H-D-Arg-L-Dmt-L-Lys-2,6-dimethyl-L-phenylalanine-NH₂ |
| H-D-Arg-L-Dmt-L-Lys-3-hydroxy-L-phenylalanine-NH₂ |
| H-D-Arg-L-Dmt-L-Lys-D-Dmt-NH₂ |
| H-D-Arg-L-Dmt-L-Lys-D-Trp-NH₂ |
| H-D-Arg-L-Dmt-L-Lys-D-Tyr-NH₂ |
| H-D-Arg-L-Dmt-L-Lys-L-Dmt-NH₂ |
| H-D-Arg-L-Dmt-L-Lys-L-Trp-NH₂ |
| H-D-Arg-L-Dmt-L-Lys-L-Tyr-NH₂ |
| H-D-Arg-L-Dmt-L-Phe-L-Lys-NH₂ |
| H-D-Arg-L-Dmt-N6-acetyl-L-lysine-L-Phe-NH₂ |
| H-D-Arg-L-Lys-L-Dmt-L-Phe-NH₂ |
| H-D-Arg-L-Lys-L-Phe-L-Dmt-NH₂ |
| H-D-Arg-L-Phe-L-Dmt-L-Lys-NH₂ |
| H-D-Arg-L-Phe-L-Lys-L-Dmt-NH₂ |
| H-D-Arg-L-Phe-L-Lys-L-Phe-NH₂ |
| H-D-Arg-L-Trp-L-Lys-L-Phe-NH₂ |
| H-D-Arg-L-Tyr-L-Lys-L-Phe-NH₂ |
| H-D-Arg-Phe-Lys-Dmt-NH₂ |
| H-D-Arg-Tyr-Lys-Phe-NH₂ |
| H-D-His-L-Dmt-L-Lys-L-Phe-NH₂ |
| H-D-Lys-L-Dmt-L-Lys-L-Phe-NH₂ |
| H-Dmt-D-Arg-Lys-Phe-NH₂ |
| H-Dmt-D-Arg-Phe-Lys-NH₂ |
| H-Dmt-Lys-D-Arg-Phe-NH₂ |
| H-Dmt-Lys-Phe-D-Arg-NH₂ |
| H-Dmt-Phe-D-Arg-Lys-NH₂ |
| H-Dmt-Phe-Lys-D-Arg-NH₂ |
| H-L-Dmt-D-Arg-L-Lys-L-Phe-NH₂ |
| H-L-Dmt-D-Arg-L-Phe-L-Lys-NH₂ |
| H-L-Dmt-L-Lys-D-Arg-L-Phe-NH₂ |
| H-L-Dmt-L-Lys-L-Phe-D-Arg-NH₂ |
| H-L-Dmt-L-Phe-D-Arg-L-Lys-NH₂ |
| H-L-Dmt-L-Phe-L-Lys-D-Arg-NH₂ |
| H-L-His-L-Dmt-L-Lys-L-Phe-NH₂ |
| H-L-Lys-D-Arg-L-Dmt-L-Phe-NH₂ |
| H-L-Lys-D-Arg-L-Phe-L-Dmt-NH₂ |
| H-L-Lys-L-Dmt-D-Arg-L-Phe-NH₂ |
| H-L-Lys-L-Dmt-L-Lys-L-Phe-NH₂ |
| H-L-Lys-L-Dmt-L-Phe-D-Arg-NH₂ |
| H-L-Lys-L-Phe-D-Arg-L-Dmt-NH₂ |
| H-L-Lys-L-Phe-L-Dmt-D-Arg-NH₂ |
| H-L-Phe-D-Arg-L-Dmt-L-Lys-NH₂ |
| H-L-Phe-D-Arg-L-Lys-L-Dmt-NH₂ |
| H-L-Phe-L-Dmt-D-Arg-L-Lys-NH₂ |
| H-L-Phe-L-Dmt-L-Lys-D-Arg-NH₂ |
| H-L-Phe-L-Lys-D-Arg-L-Dmt-NH₂ |
| H-L-Phe-L-Lys-L-Dmt-D-Arg-NH₂ |
| H-Lys-D-Arg-Dmt-Phe-NH₂ |
| H-Lys-D-Arg-Phe-Dmt-NH₂ |
| H-Lys-Dmt-D-Arg-Phe-NH₂ |
| H-Lys-Dmt-Phe-D-Arg-NH₂ |
| H-Lys-Phe-D-Arg-Dmt-NH₂ |
| H-Lys-Phe-Dmt-D-Arg-NH₂ |
| H-Phe-Arg-Phe-Lys-NH₂ |
| H-Phe-D-Arg-Dmt-Lys-NH₂ |
| H-Phe-D-Arg-Lys-Dmt-NH₂ |
| H-Phe-Dmt-D-Arg-Lys-NH₂ |
| H-Phe-Dmt-Lys-D-Arg-NH₂ |
| H-Phe-Lys-D-Arg-Dmt-NH₂ |
| H-Phe-Lys-Dmt-D-Arg-NH₂ |
| L-Arg-D-Dmt-D-Lys-D-Phe-NH₂ |
| L-Arg-D-Dmt-D-Lys-L-Phe-NH₂ |
| L-Arg-D-Dmt-L-Lys-D-Phe-NH₂ |
| L-Arg-D-Dmt-L-Lys-L-Phe-NH₂ |
| L-Arg-L-Dmt-D-Lys-D-Phe-NH₂ |
| L-Arg-L-Dmt-D-Lys-L-Phe-NH₂ |
| L-Arg-L-Dmt-L-Lys-D-Phe-NH₂ |
| L-Arg-L-Dmt-L-Lys-L-Phe-NH₂ |
| Lys-Dmt-Arg |
| Lys-Phe |
| Lys-Phe-Arg-Dmt |
| Lys-Trp-Arg |
| Phe-Arg-Dmt-Lys |
| Phe-Arg-Phe-Lys |
| Phe-Dmt-Arg-Lys |
| Phe-Lys-Dmt |
| Arg-Dmt-Lys-Phe-NH₂ |
| Phe-Dmt-Arg-Lys-NH₂ |
| Phe-Lys-Dmt-Arg-NH₂ |
| Dmt-Arg-Lys-Phe-NH₂ |
| Lys-Dmt-Arg-Phe-NH₂ |
| Phe-Dmt-Lys-Arg-NH₂ |
| Arg-Lys-Dmt-Phe-NH₂ |
| Arg-Dmt-Phe-Lys-NH₂ |
| D-Arg-Dmt-Lys-Phe-NH₂ |
| Dmt-D-Arg-Phe-Lys-NH₂ |
| H-Phe-D-Arg Phe-Lys-Cys-NH₂ |
| D-Arg-Dmt-Lys-Trp-NH₂ |
| D-Arg-Trp-Lys-Trp-NH₂ |
| H-D-Arg-Dmt-Lys-Phe(*N*Me)-NH₂ |
| H-D-Arg-Dmt-Lys(*N*^{α}Me)-Phe(*N*Me)-NH₂ |
| H-D-Arg(*N*^{α}Me)-Dmt(*N*Me)-Lys(*N*^{α}Me)-Phe(*N*Me)-NH₂ |
| D-Arg-2'6'Dmt-Lys-Phe-NH₂ |
| H-Phe-D-Arg-Phe-Lys-Cys-NH₂ |
| D-Arg-Dmt-Lys-Phe-Ser-Cys-NH₂ |
| D-Arg-Dmt-Lys-Phe-Gly-Cys-NH₂ |
| Gly-D-Phe-Lys-His-D-Arg-Tyr-NH₂ |
| D-Arg-Dmt-Lys-Phe-Met-NH₂ |
| D-Arg-Dmt-Lys-Phe-Lys-Trp-NH₂ |
| D-Arg-Dmt-Lys-Dmt-Lys-Trp-NH₂ |
| D-Arg-Dmt-Lys-Phe-Lys-Met-NH₂ |
| D-Arg-Dmt-Lys-Dmt-Lys-Met-NH₂ |
| H-D-Arg-Dmt-Lys-OH |
| H-D-Arg-Dmt-OH |
| H-D-Arg-Dmt-Lys-Phe-OH |

| TABLE B | | | | |
|---|---|---|---|---|
| Amino Acid Position 1 | Amino Acid Position 2 | Amino Acid Position 3 | Amino Acid Position 4 | C-Terminal Modification |
| Tyr | D-Arg | Phe | Orn | NH₂ |
| Tyr | D-Arg | Phe | Dab | NH₂ |
| Tyr | D-Arg | Phe | Dap | NH₂ |
| 2'6'Dmt | D-Arg | Phe | Lys-NH(CH₂)₂-NH-dns | NH₂ |
| 2'6'Dmt | D-Arg | Phe | Lys-NH(CH₂)₂-NH-atn | NH₂ |
| 2'6'Dmt | D-Arg | Phe | dnsLys | NH₂ |
| 2'6'Dmt | D-Cit | Phe | Ahp | NH₂ |
| 2'6'Dmt | D-Arg | Phe | Dab | NH₂ |
| 2'6'Dmt | D-Arg | Phe | Dap | NH₂ |
| 3'5'Dmt | D-Arg | Phe | Lys | NH₂ |
| 3'5'Dmt | D-Arg | Phe | Orn | NH₂ |
| 3'5'Dmt | D-Arg | Phe | Dab | NH₂ |
| 3'5'Dmt | D-Arg | Phe | Dap | NH₂ |
| Tyr | D-Arg | Tyr | Lys | NH₂ |
| Tyr | D-Arg | Tyr | Orn | NH₂ |
| Tyr | D-Arg | Tyr | Dab | NH₂ |
| Tyr | D-Arg | Tyr | Dap | NH₂ |
| 2'6'Dmt | D-Arg | Tyr | Lys | NH₂ |

| Amino Acid Position 1 | Amino Acid Position 2 | Amino Acid Position | Amino Acid Position 4 | C-Terminal Modification |
|---|---|---|---|---|
| 2'6'Dmt | D-Arg | Tyr | Orn | NH₂ |
| 2'6'Dmt | D-Arg | Tyr | Dab | NH₂ |
| 2'6'Dmt | D-Arg | Tyr | Dap | NH₂ |
| 2'6'Dmt | D-Arg | 2'6'Dmt | Lys | NH₂ |
| 2'6'Dmt | D-Arg | 2'6'Dmt | Orn | NH₂ |
| 2'6'Dmt | D-Arg | 2'6'Dmt | Dab | NH₂ |
| 2'6'Dmt | D-Arg | 2'6'Dmt | Dap | NH₂ |
| 3'5'Dmt | D-Arg | 3'5'Dmt | Arg | NH₂ |
| 3'5'Dmt | D-Arg | 3'5'Dmt | Lys | NH₂ |
| 3'5'Dmt | D-Arg | 3'5'Dmt | Orn | NH₂ |
| 3'5'Dmt | D-Arg | 3'5'Dmt | Dab | NH₂ |
| Tyr | D-Lys | Phe | Dap | NH₂ |
| Tyr | D-Lys | Phe | Arg | NH₂ |
| Tyr | D-Lys | Phe | Lys | NH₂ |
| Tyr | D-Lys | Phe | Orn | NH₂ |
| 2'6'Dmt | D-Lys | Phe | Dab | NH₂ |
| 2'6'Dmt | D-Lys | Phe | Dap | NH₂ |
| 2'6'Dmt | D-Lys | Phe | Arg | NH₂ |
| 2'6'Dmt | D-Lys | Phe | Lys | NH₂ |
| 3'5'Dmt | D-Lys | Phe | Orn | NH₂ |
| 3'5'Dmt | D-Lys | Phe | Dab | NH₂ |
| 3'5'Dmt | D-Lys | Phe | Dap | NH₂ |
| 3'5'Dmt | D-Lys | Phe | Arg | NH₂ |
| Tyr | D-Lys | Tyr | Lys | NH₂ |
| Tyr | D-Lys | Tyr | Orn | NH₂ |
| Tyr | D-Lys | Tyr | Dab | NH₂ |
| Tyr | D-Lys | Tyr | Dap | NH₂ |
| 2'6'Dmt | D-Lys | Tyr | Lys | NH₂ |
| 2'6'Dmt | D-Lys | Tyr | Orn | NH₂ |
| 2'6'Dmt | D-Lys | Tyr | Dab | NH₂ |
| 2'6'Dmt | D-Lys | Tyr | Dap | NH₂ |
| 2'6'Dmt | D-Lys | 2'6'Dmt | Lys | NH₂ |
| 2'6'Dmt | D-Lys | 2'6'Dmt | Orn | NH₂ |
| 2'6'Dmt | D-Lys | 2'6'Dmt | Dab | NH₂ |
| 2'6'Dmt | D-Lys | 2'6'Dmt | Dap | NH₂ |
| 3'5'Dmt | D-Lys | 3'5'Dmt | Lys | NH₂ |
| 3'5'Dmt | D-Lys | 3'5'Dmt | Orn | NH₂ |
| 3'5'Dmt | D-Lys | 3'5'Dmt | Dab | NH₂ |
| 3'5'Dmt | D-Lys | 3'5'Dmt | Dap | NH₂ |
| Tyr | D-Lys | Phe | Arg | NH₂ |
| Tyr | D-Orn | Phe | Arg | NH₂ |
| Tyr | D-Dab | Phe | Arg | NH₂ |
| Tyr | D-Dap | Phe | Arg | NH₂ |
| 2'6'Dmt | D-Arg | Phe | Arg | NH₂ |
| 2'6'Dmt | D-Lys | Phe | Arg | NH₂ |
| 2'6'Dmt | D-Orn | Phe | Arg | NH₂ |
| 2'6'Dmt | D-Dab | Phe | Arg | NH₂ |
| 3'5'Dmt | D-Dap | Phe | Arg | NH₂ |
| 3'5'Dmt | D-Arg | Phe | Arg | NH₂ |
| 3'5'Dmt | D-Lys | Phe | Arg | NH₂ |
| 3'5'Dmt | D-Orn | Phe | Arg | NH₂ |
| Tyr | D-Lys | Tyr | Arg | NH₂ |
| Tyr | D-Orn | Tyr | Arg | NH₂ |
| Tyr | D-Dab | Tyr | Arg | NH₂ |
| Tyr | D-Dap | Tyr | Arg | NH₂ |
| 2'6'Dmt | D-Arg | 2'6'Dmt | Arg | NH₂ |
| 2'6'Dmt | D-Lys | 2'6'Dmt | Arg | NH₂ |
| 2'6'Dmt | D-Orn | 2'6'Dmt | Arg | NH₂ |
| 2'6'Dmt | D-Dab | 2'6'Dmt | Arg | NH₂ |
| 3'5'Dmt | D-Dap | 3'5'Dmt | Arg | NH₂ |
| 3'5'Dmt | D-Arg | 3'5'Dmt | Arg | NH₂ |
| 3'5'Dmt | D-Lys | 3'5'Dmt | Arg | NH₂ |
| 3'5'Dmt | D-Orn | 3'5'Dmt | Arg | NH₂ |
| Mmt | D-Arg | Phe | Lys | NH₂ |
| Mmt | D-Arg | Phe | Orn | NH₂ |
| Mmt | D-Arg | Phe | Dab | NH₂ |
| Mmt | D-Arg | Phe | Dap | NH₂ |
| Tmt | D-Arg | Phe | Lys | NH₂ |
| Tmt | D-Arg | Phe | Orn | NH₂ |
| Tmt | D-Arg | Phe | Dab | NH₂ |
| Tmt | D-Arg | Phe | Dap | NH₂ |
| Hmt | D-Arg | Phe | Lys | NH₂ |
| Hmt | D-Arg | Phe | Orn | NH₂ |
| Hmt | D-Arg | Phe | Dab | NH₂ |
| Hmt | D-Arg | Phe | Dap | NH₂ |
| Mmt | D-Lys | Phe | Lys | NH₂ |
| Mmt | D-Lys | Phe | Orn | NH₂ |
| Mmt | D-Lys | Phe | Dab | NH₂ |
| Mmt | D-Lys | Phe | Dap | NH₂ |
| Mmt | D-Lys | Phe | Arg | NH₂ |
| Tmt | D-Lys | Phe | Lys | NH₂ |
| Tmt | D-Lys | Phe | Orn | NH₂ |
| Tmt | D-Lys | Phe | Dab | NH₂ |
| Tmt | D-Lys | Phe | Dap | NH₂ |
| Tmt | D-Lys | Phe | Arg | NH₂ |
| Hmt | D-Lys | Phe | Lys | NH₂ |
| Hmt | D-Lys | Phe | Orn | NH₂ |
| Hmt | D-Lys | Phe | Dab | NH₂ |
| Hmt | D-Lys | Phe | Dap | NH₂ |
| Hmt | D-Lys | Phe | Arg | NH₂ |
| Mmt | D-Lys | Phe | Arg | NH₂ |
| Mmt | D-Orn | Phe | Arg | NH₂ |
| Mmt | D-Dab | Phe | Arg | NH₂ |
| Mmt | D-Dap | Phe | Arg | NH₂ |
| Mmt | D-Arg | Phe | Arg | NH₂ |
| Tmt | D-Lys | Phe | Arg | NH₂ |
| Tmt | D-Orn | Phe | Arg | NH₂ |
| Tmt | D-Dab | Phe | Arg | NH₂ |
| Tmt | D-Dap | Phe | Arg | NH₂ |
| Tmt | D-Arg | Phe | Arg | NH₂ |
| Hmt | D-Lys | Phe | Arg | NH₂ |
| Hmt | D-Orn | Phe | Arg | NH₂ |
| Hmt | D-Dab | Phe | Arg | NH₂ |
| Hmt | D-Dap | Phe | Arg | NH₂ |
| Hmt | D-Arg | Phe | Arg | NH₂ |
| Trp | D-Arg | Phe | Lys | NH₂ |

2'-methyltyrosine (Mmt); Dimethyltyrosine (Dmt); 2',6'-dimethyltyrosine (2'6'-Dmt); 3',5'-dimethyltyrosine (3'5'Dmt); N,2',6'-trimethyltyrosine (Tmt); 2'-hydroxy-6'-methyltyrosine (Hmt); 2'-methylphenylalanine (Mmp); dimethylphenylalanine (Dmp) 2',6'-dimethylphenylalanine (2',6'-Dmp); N,2',6'-trimethylphenylalanine (Tmp); 2'-hydroxy-6'-methylphenylalanine (Hmp); cyclohexylalanine (Cha); diaminobutyric (Dab); diaminopropionic acid (Dap); β-dansyl-L-α,β -diaminopropionic acid (dnsDap); β-anthraniloyl-L-α,β-diaminopropionic acid (atnDap); biotin (bio); norleucine (Nle); 2-aminohepantoic acid (Ahp); β-(6'-dimethylamino-2'-naphthoyl)alanine (Ald); Sarcosine (Sar)

In another embodiment, the peptide is defined by Formula II: 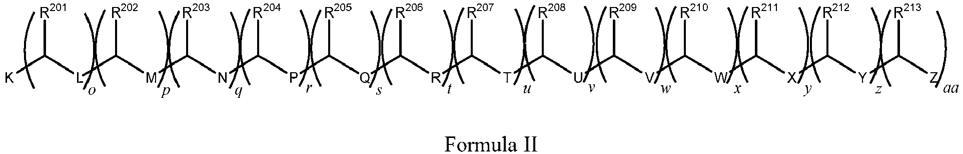 wherein:
one of K and Z is 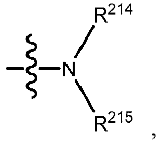
and the other of K and Z is 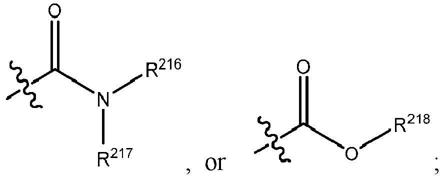
L, M, N, P, Q, R, T, U, V, W, X, and Y are each 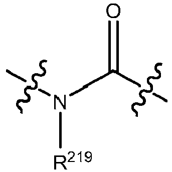 or L, M, N, P, Q, R, T, U, V, W, X, and Y are each 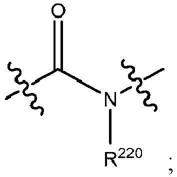 with the proviso that when
   *aa* is 0 and Z is not a terminal group, the terminal group is one of L, M, N, P, Q, R, T, U, V, W, X, or Y, such that one of K and the terminal group is 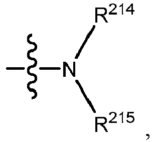
   and the other of K and the terminal group is selected from 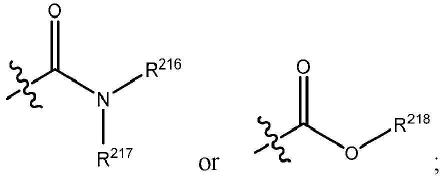
R²⁰¹ is 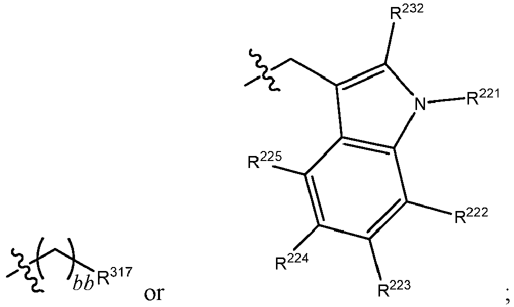
R²⁰² is 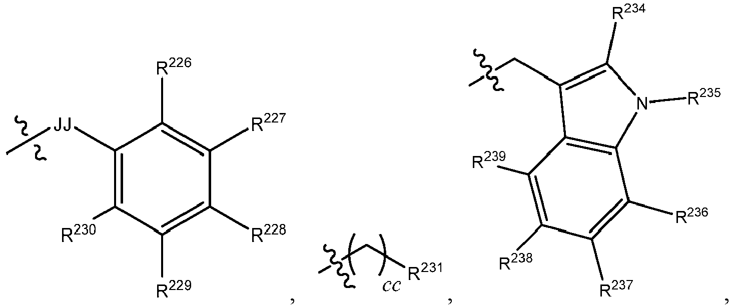 or 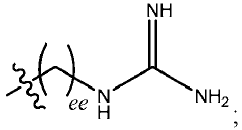
R²⁰³ is 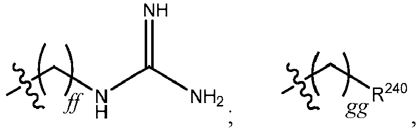 or hydrogen;
R²⁰⁴ is 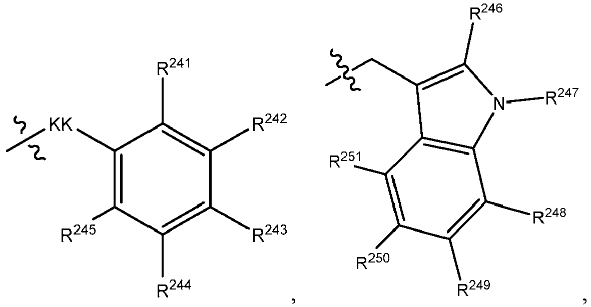 or 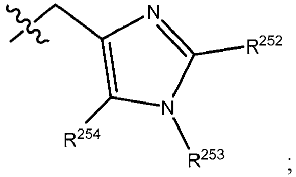
R²⁰⁵ is 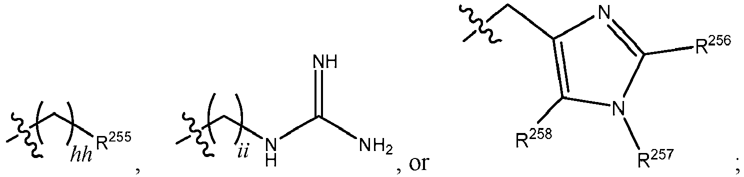
R²⁰⁶ is 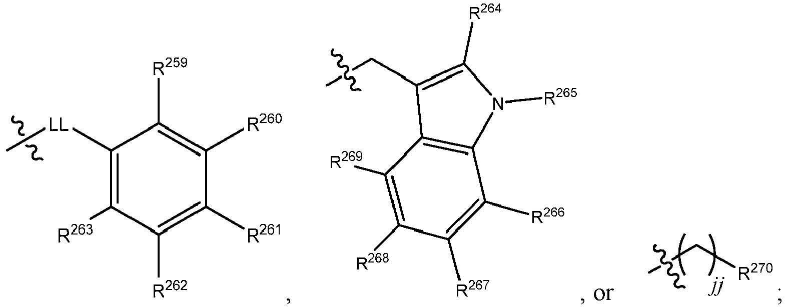
R²⁰⁷ is 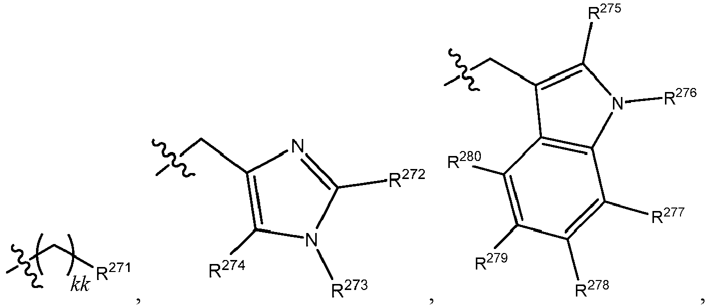 or hydrogen;
R²⁰⁸ is 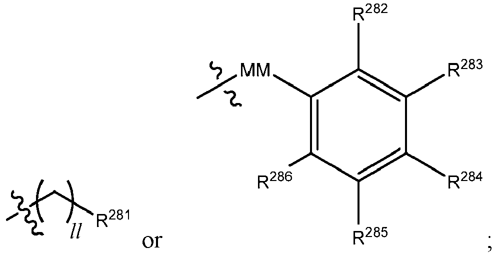
R²⁰⁹ is 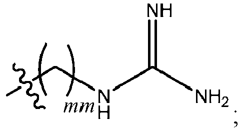
R²¹⁰ is 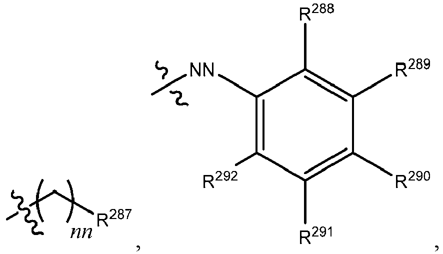 or hydrogen;
R²¹¹ is 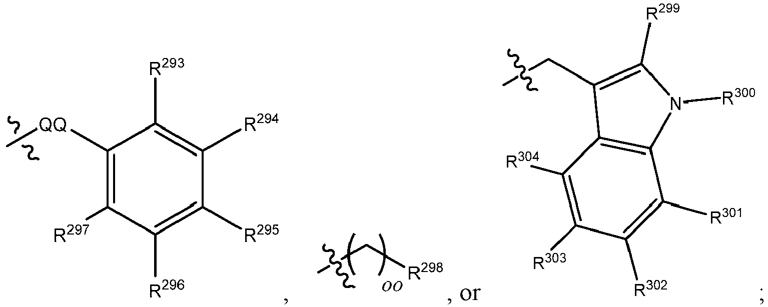
R²¹² is 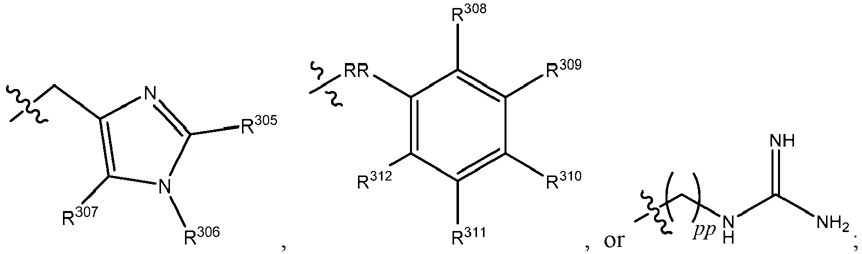 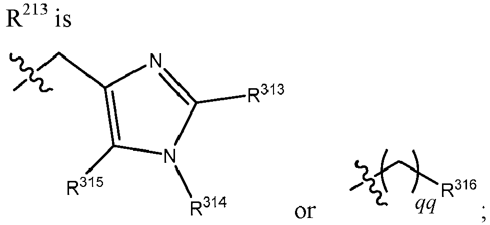 wherein
   R²¹⁴, R²¹⁵, R²¹⁶, R²¹⁷, and R²¹⁸ are each independently a hydrogen or substituted or unsubstituted C₁-C₆ alkyl, C₂-C₆ alkenyl, C₂-C₆ alkynyl, saturated or unsaturated cycloalkyl, cycloalkylalkyl, aryl, aralkyl, 5- or 6- membered saturated or unsaturated heterocylyl, heteroaryl, or amino protecting group; or R²¹⁴ and R²¹⁵ together form a 3, 4, 5, 6, 7, or 8 membered substituted or unsubstituted heterocycyl ring;
   R²¹⁹ and R²²⁰ are, at each occurrence, independently a hydrogen or substituted or unsubstituted C₁-C₆ alkyl group;
   R²²², R²²³, R²²⁴, R²²⁵, R²²⁶, R²²⁷, R²²⁸, R²²⁹, R²³⁰, R²³², R²³⁴, R²³⁶, R²³⁷, R²³⁸, R²³⁹, R²⁴¹, R²⁴², R²⁴³, R²⁴⁴, R²⁴⁵, R²⁴⁶, R²⁴⁸, R²⁴⁹, R²⁵⁰, R²⁵¹, R²⁵², R²⁵⁴, R²⁵⁶, R²⁵⁸, R²⁵⁹, R²⁶⁰, R²⁶¹, R²⁶², R²⁶³, R²⁶⁴, R²⁶⁶, R²⁶⁷, R²⁶⁸, R²⁶⁹, R²⁷², R²⁷⁴, R²⁷⁵, R²⁷⁷, R²⁷⁸, R²⁷⁹, R²⁸⁰, R²⁸², R²⁸³, R²⁸⁴, R²⁸⁵, R²⁸⁶, R²⁸⁸, R²⁸⁹, R²⁹⁰, R²⁹¹, R²⁹², R²⁹³, R²⁹⁴, R²⁹⁵, R²⁹⁶, R²⁹⁷, R²⁹⁹, R³⁰¹, R³⁰², R³⁰³, R³⁰⁴, R³⁰⁵, R³⁰⁷, R³⁰⁸, R³⁰⁹, R³¹⁰, R³¹¹, R³¹², R³¹³, and R³¹⁵ are each independently a hydrogen, amino, amido, -NO₂, -CN, -OR^{c},-SR^{c}, -NR^{c}R^{c}, -F, -Cl, -Br, -I, or a substituted or unsubstituted C₁-C₆ alkyl, C₁-C₆ alkoxy, -C(O)-alkyl, -C(O)-aryl, -C(O)-aralkyl, -C(O)₂R^{c}, C₁-C₄ alkylamino, C₁-C₄ dialkylamino, or perhaloalkyl group;
   R²²¹, R²³⁵, R²⁴⁷, R²⁵³, R²⁵⁷, R²⁶⁵, R²⁷³, R²⁷⁶, R³⁰⁰, R³⁰⁶, and R³¹⁴ are each independently a hydrogen or substituted or unsubstituted C₁-C₆ alkyl group;
   R²³¹, R²⁴⁰, R²⁵⁵, R²⁷⁰, R²⁷¹, R²⁸¹, R²⁸⁷, R²⁹⁸, R³¹⁶, and R³¹⁷ are each independently a hydrogen, -OR^{c}, -SR^{c}, -NR^{c}R^{c}, -NR^{c}R^{d}, -CO₂R^{c}, -(CO)NR^{c}R^{c}, -NR^{c}(CO)R^{c}, -NR^{c}C(NH)NH₂, -NR^{c}-dansyl, or a substituted or unsubstituted alkyl, aryl, or aralkyl group;
   JJ, KK, LL, MM, NN, QQ, and RR are each independently absent, -NH(CO)-, or -CH₂-;
   R^{c} at each occurrence is independently a hydrogen or a substituted or unsubstituted C₁-C₆ alkyl group;
   R^{d} at each occurrence is independently a C₁-C₆ alkylene-NR^{c}-dansyl or C₁-C₆ alkylene-NR^{c}-anthraniloyl group;
   *o, p, q, r, s, t, u, v, w, x, y, z,* and *aa* are each independently 0 or 1, with the proviso that *o* + *p* + *q* + *r* + *s* + *t* + *u* + *v* + *w* + *x* + *y + z* + *aa* equals 6, 7, 8, 9, 10, or 11;
   cc is 0, 1, 2, 3, 4, or 5; and
   *bb, cc, ee, ff*, *gg, hh, ii, jj*, *kk, ll*, *mm, nn, oo, pp,* and *qq* are each independently 1, 2, 3, 4, or 5.

In some embodiments of peptides of Formula II,
R²¹⁴, R²¹⁵, R²¹⁶, R²¹⁷, and R²¹⁸ are each independently a hydrogen or substituted or unsubstituted C₁-C₆ alkyl group;
R²¹⁹ and R²²⁰ are, at each occurrence, independently a hydrogen or methyl group;
R²²², R²²³, R²²⁴, R²²⁵, R²²⁶, R²²⁷, R²²⁸, R²²⁹, R²³⁰, R²³², R²³⁴, R²³⁶, R²³⁷, R²³⁸, R²³⁹, R²⁴¹, R²⁴², R²⁴³, R²⁴⁴, R²⁴⁵, R²⁴⁶, R²⁴⁸, R²⁴⁹, R²⁵⁰, R²⁵¹, R²⁵², R²⁵⁴, R²⁵⁶, R²⁵⁸, R²⁵⁹, R²⁶⁰, R²⁶¹, R²⁶², R²⁶³, R²⁶⁴, R²⁶⁶, R²⁶⁷, R²⁶⁸, R²⁶⁹, R²⁷², R²⁷⁴, R²⁷⁵, R²⁷⁷, R²⁷⁸, R²⁷⁹, R²⁸⁰, R²⁸², R²⁸³, R²⁸⁴, R²⁸⁵, R²⁸⁶, R²⁸⁸, R²⁸⁹, R²⁹⁰, R²⁹¹, R²⁹², R²⁹³, R²⁹⁴, R²⁹⁵, R²⁹⁶, R²⁹⁷, R²⁹⁹, R³⁰¹, R³⁰², R³⁰³, R³⁰⁴, R³⁰⁵, R³⁰⁷, R³⁰⁸, R³⁰⁹, R³¹⁰, R³¹¹, R³¹², R³¹³, and R³¹⁵ are each independently a hydrogen, methyl, or -OR^{c} group;
R²²¹, R²³⁵, R²⁴⁷, R²⁵³, R²⁵⁷, R²⁶⁵, R²⁷³, R²⁷⁶, R³⁰⁰, R³⁰⁶, and R³¹⁴ are each independently a hydrogen or substituted or unsubstituted C₁-C₆ alkyl group;
R²³¹ is -(CO)NR^{c}R^{c}, -OR^{c}, or a C₁-C₆ alkyl group, optionally substituted with a hydroxyl or methyl group;
R²⁴⁰ and R²⁵⁵ are each independently -CO₂R^{c} or -NR^{c}R^{c};
R²⁷⁰ and R²⁷¹ are each independently -CO₂R^{c};
R²⁸¹ is -SR^{c} or -NR^{c}R^{c};
R²⁸⁷ -(CO)NR^{c}R^{c} or -OR^{c};
R²⁹⁸ -NR^{c}R^{c}, -CO₂R^{c}, or -SR^{c};
R³¹⁶ is -NR^{c}R^{c};
R³¹⁷ is hydrogen or -NR^{c}R^{c};
JJ, KK, LL, MM, NN, QQ, and RR are each independently absent or -CH₂-;
R^{c} at each occurrence is independently a hydrogen or a substituted or unsubstituted C₁-C₆ alkyl group;
R^{d} at each occurrence is independently a C₁-C₆ alkylene-NR^{c}-dansyl or C₁-C₆ alkylene-NR^{c}-anthraniloyl group;
*o, p, q, r, s, t, u, v, w, x, y, z,* and *aa* are each independently 0 or 1, with the proviso that *o* + *p* + *q* + *r* + *s* + *t* + *u* + *v* + *w* + *x* + *y* + *z* + *aa* equals 6, 7, 8, 9, 10, or 11;
cc is 0, 1, 2, 3, 4, or 5; and
*bb, cc, dd, ee, ff, gg, hh, ii, jj, kk, ll, mm, nn, oo, pp,* and *qq* are each independently 1, 2, 3, 4, or 5.

In some embodiments of peptides of Formula II,
R²²¹, R²²², R²²³, R²²⁴, R²²⁵, R²²⁶, R²²⁷, R²²⁸, R²²⁹, R²³⁰, R²³², R²³⁴, R²³⁵, R²³⁶, R²³⁷, R²³⁸, R²³⁹, R²⁴², R²⁴⁴, R²⁴⁶, R²⁴⁷, R²⁴⁸, R²⁴⁹, R²⁵⁰, R²⁵¹, R²⁵², R²⁵³, R²⁵⁴, R²⁵⁶, R²⁵⁷, R²⁵⁸, R²⁵⁹, R²⁶⁰, R²⁶², R²⁶³, R²⁶⁴, R²⁶⁵, R²⁶⁶, R²⁶⁷, R²⁶⁸, R²⁶⁹, R²⁷², R²⁷³, R²⁷⁴, R²⁷⁵, R²⁷⁶, R²⁷⁷, R²⁷⁸, R²⁷⁹, R²⁸⁰, R²⁸², R²⁸³, R²⁸⁵, R²⁸⁶, R²⁸⁸, R²⁸⁹, R²⁹¹, R²⁹², R²⁹³, R²⁹⁴, R²⁹⁶, R²⁹⁷, R²⁹⁹, R³⁰⁰, R³⁰¹, R³⁰², R³⁰³, R³⁰⁴, R³⁰⁵, R³⁰⁶, R³⁰⁷, R³⁰⁸, R³⁰⁹, R³¹¹, R³¹², R³¹³, R³¹⁴, and R³¹⁵ are each hydrogen;
R²⁴¹ and R²⁴⁵ are each independently a hydrogen or methyl group;
R²⁴³, R²⁶¹, R²⁸⁴, R²⁹⁰, R²⁹⁵, R³¹⁰ are each independently a hydrogen or OH;
R²³¹ is -(CO)NH₂, an ethyl group substituted with a hydroxyl group, or an isopropyl group;
R²⁴⁰ and R²⁵⁵ are each independently -CO₂H or -NH₂;
R²⁷⁰ and R²⁷¹ are each independently -CO₂H;
R²⁸¹ is -SH or -NH₂;
R²⁸⁷ is -(CO)NH₂ or -OH;
R²⁹⁸ is -NH₂, -CO₂H, or -SH;
R³¹⁶ is -NH₂;
R³¹⁷ is hydrogen or -NH₂;
JJ, KK, LL, MM, NN, QQ, and RR are each independently -CH₂₋;
*o, p, q, r, s, t, u, v, w, x, y, z,* and *aa* are each independently 0 or 1, with the proviso that *o* + *p* + *q* + *r* + *s* + *t* + *u* + *v* + *w* + *x* + *y* + *z* + *aa* equals 6, 7, 8, 9, 10, or 11;
cc is 0, 1, 2, 3, 4, or 5; and
*bb, cc, dd, ee, ff, gg, hh, ii, jj, kk, ll, mm, nn, oo, pp,* and *qq* are each independently 1, 2, 3, 4, or 5.

In certain embodiments of Formula II,
K is 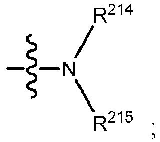
Z is 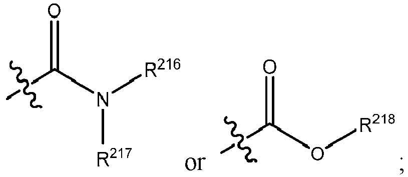
L, M, N, P, Q, R, T, U, V, W, X, and Y are each independently 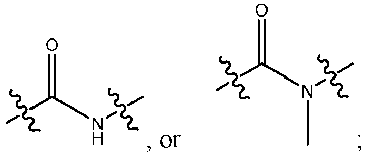 with the proviso that when
   *aa* is 0 and Z is not a terminal group, the terminal group is one of L, M, N, P, Q, R, T, U, V, W, X, or Y, such that one of L, M, N, P, Q, R, T, U, V, W, X, or Y, is

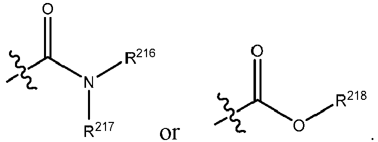

In another embodiment of Formula II,
K is 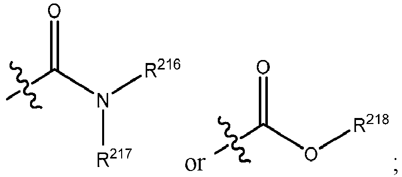
Z is 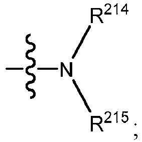
L, M, N, P, Q, R, T, U, V, W, X, and Y are each independently 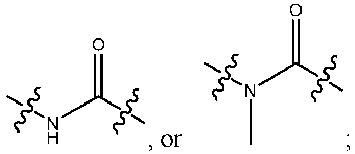 with the proviso that when
   *aa* is 0 and Z is not a terminal group, the terminal group is one of L, M, N, P, Q, R, T, U, V, W, X, or Y, such that one of L, M, N, P, Q, R, T, U, V, W, X, or Y, is

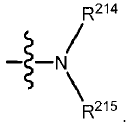

In some embodiments, the peptide of Formula II is selected from the peptides shown in Table C.

| **TABLE C** |
|---|
| D-Arg-Dmt-Lys-Phe-Glu-Cys-Gly-NH₂ |
| Phe-D-Arg-Phe-Lys-Glu-Cys-Gly-NH₂ |
| Phe-D-Arg-Dmt-Lys-Glu-Cys-Gly-NH₂ |
| Ala-D-Phe-D-Arg-Tyr-Lys-D-Trp-His-D-Tyr-Gly-Phe |
| Asp-D-Trp-Lys-Tyr-D-His-Phe-Arg-D-Gly-Lys-NH₂ |
| D-His-Glu-Lys-Tyr-D-Phe-Arg |
| D-His-Lys-Tyr-D-Phe-Glu-D-Asp-D-Asp-D-His-D-Lys-Arg-Trp-NH₂ |
| Lys-D-Gln-Tyr-Arg-D-Phe-Trp-NH₂ |
| Lys-Trp-D-Tyr-Arg-Asn-Phe-Tyr-D-His-NH₂ |
| Phe-D-Arg-Lys-Trp-Tyr-D-Arg-His |
| Thr-Gly-Tyr-Arg-D-His-Phe-Trp-D-His-Lys |
| Trp-Lys-Phe-D-Asp-Arg-Tyr-D-His-Lys |
| Val-D-Lys-His-Tyr-D-Phe-Ser-Tyr-Arg-NH₂ |
| Gly-D-Phe-Lys-Tyr-His-D-Arg-Tyr-NH₂ |
| Asp-D-Trp-Lys-Tyr-D-His-Phe-Arg-D-Gly-Lys-NH₂ |
| D-His-Lys-Tyr-D-Phe-Glu-D-Asp-D-His-D-Lys-Arg-Trp-NH₂ |
| H-Phe-D-Arg-Phe-Lys-Glu-Cys-Gly-NH₂ |
| Phe-Arg-Phe-Lys-Glu-Cys-Gly |
| H-D-Arg-Dmt-Lys-Phe-Sar-Gly-Cys-NH₂ |

In another embodiment the peptide is defined by Formula III: 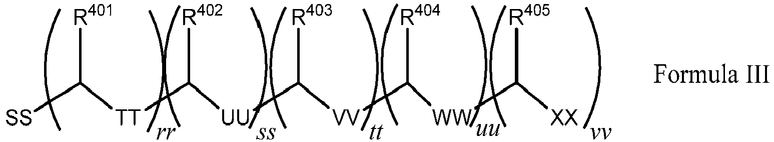 wherein:
one of SS and XX is 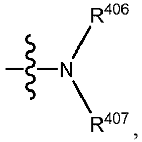
and the other is 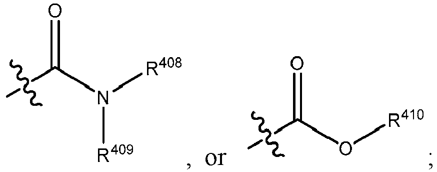
TT, UU, VV, and WW are each 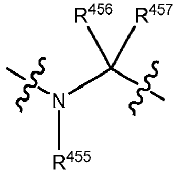 or TT, UU, VV, and WW are each 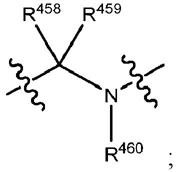
with the proviso when vv is 0 and *uu* is 1, one of SS and WW is 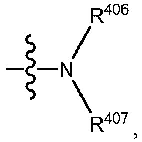
and the other of SS and WW is 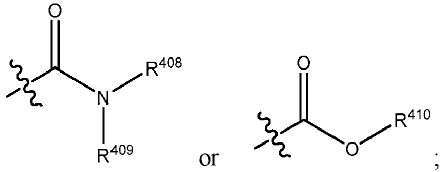
R⁴⁰¹ is 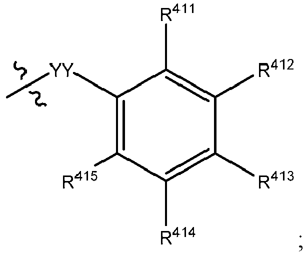
R⁴⁰² is 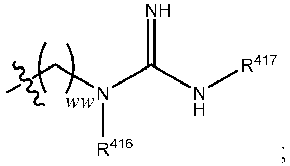
R⁴⁰³ is 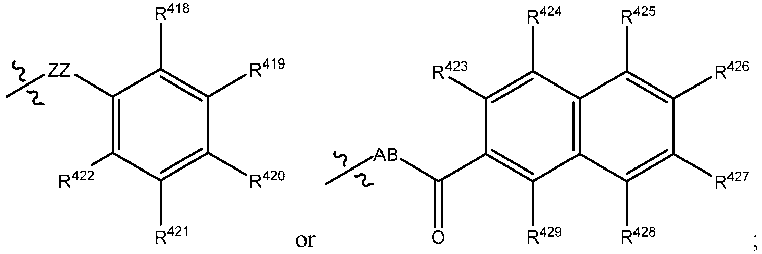
R⁴⁰⁴ is 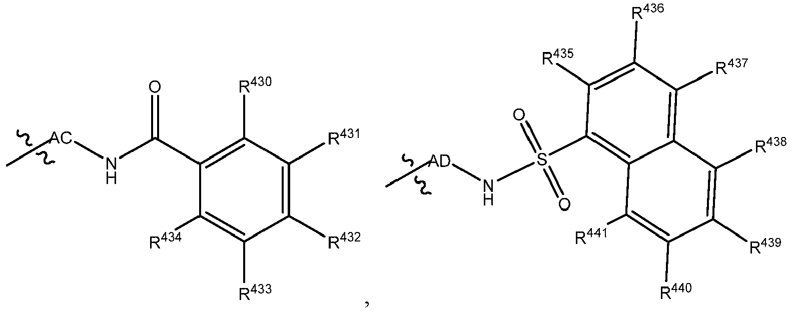 or 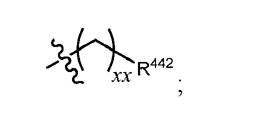 ;
R⁴⁰⁵ is 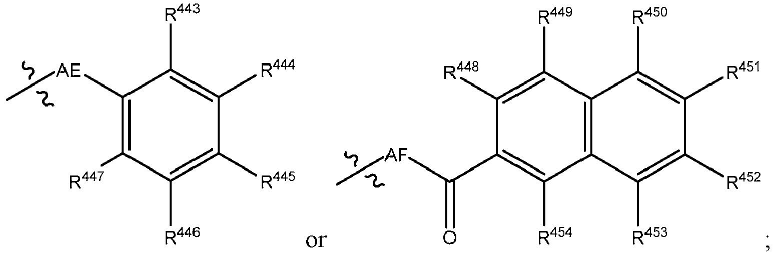 wherein
   R⁴⁰⁶, R⁴⁰⁷, R⁴⁰⁸, R⁴⁰⁹ and R⁴¹⁰ are each independently a hydrogen or substituted or unsubstituted C₁-C₆ alkyl, C₂-C₆ alkenyl, C₂-C₆ alkynyl, saturated or unsaturated cycloalkyl, cycloalkylalkyl, aryl, aralkyl, 5- or 6- membered saturated or unsaturated heterocylyl, heterobicycyl, heteroaryl, or amino protecting group; or R⁴⁰⁶ and R⁴⁰⁷ together form a 3-, 4-, 5-, 6-, 7-, or 8-member substituted or unsubstituted heterocycyl ring;
   R⁴⁵⁵ and R⁴⁶⁰ are at each occurrence independently a hydrogen,-C(O)R^{e}, or an unsubstituted C₁-C₆ alkyl group;
   R⁴⁵⁶ and R⁴⁵⁷ are each independently a hydrogen or substituted or unsubstituted C₁-C₆ alkyl group; or together R⁴⁵⁶ and R⁴⁵⁷ are C=O;
   R⁴⁵⁸ and R⁴⁵⁹ are each independently a hydrogen or substituted or unsubstituted C₁-C₆ alkyl group; or together R⁴⁵⁸ and R⁴⁵⁹ are C=O;
   R⁴¹¹, R⁴¹², R⁴¹³, R⁴¹⁴, R⁴¹⁵, R⁴¹⁸, R⁴¹⁹, R⁴²⁰, R⁴²¹, R⁴²², R⁴²³, R⁴²⁴, R⁴²⁵, R⁴²⁶, R⁴²⁷, R⁴²⁸, R⁴²⁹, R⁴³⁰, R⁴³¹, R⁴³², R⁴³³, R⁴³⁴, R⁴³⁵, R⁴³⁶, R⁴³⁷, R⁴³⁸, R⁴³⁹, R⁴⁴⁰, R⁴⁴¹, R⁴⁴³, R⁴⁴⁴, R⁴⁴⁵, R⁴⁴⁶, R⁴⁴⁷, R⁴⁴⁸, R⁴⁴⁹, R⁴⁵⁰, R⁴⁵¹, R⁴⁵², R⁴⁵³, and R⁴⁵⁴ are each independently a hydrogen, deuterium, amino, amido, -NO₂, -CN, -OR^{e}, -SR^{e},-NR^{e}R^{e}, -F, -Cl, -Br, -I, or a substituted or unsubstituted C₁-C₆ alkyl, C₁-C₆ alkoxy, -C(O)-alkyl, -C(O)-aryl, -C(O)-aralkyl,
   -C(O)₂R^{e}, C₁-C₄ alkylamino, C₁-C₄ dialkylamino, or perhaloalkyl group;
   R⁴¹⁶ and R⁴¹⁷ are each independently a hydrogen, -C(O)R^{e}, or a substituted or unsubstituted C₁-C₆ alkyl;
   R⁴⁴² is a hydrogen, -OR^{e}, -SR^{e}, -NR^{e}R^{e}, -NR^{e}R^{f}, -CO₂R^{e}, -C(O)NR^{e}R^{e}, -NR^{e}C(O)R^{e}, -NR^{e}C(NH)NH₂, -NR^{e}-dansyl, or a substituted or unsubstituted alkyl, aryl, or aralkyl group;
   YY, ZZ, and AE are each independently absent, -NH(CO)-, or -CH₂-; AB, AC, AD, and AF are each independently absent or C₁-C₆ alkylene group;
   R^{e} at each occurrence is independently a hydrogen or a substituted or unsubstituted C₁-C₆ alkyl group;
   R^{f} at each occurrence is independently a C₁-C₆ alkylene-NR^{e}-dansyl or C₁-C₆ alkylene-NR^{e}-anthraniloyl group;
   *rr, ss,* and vv are each independently 0 or 1; *tt* and *uu* are each 1 with the proviso that *rr* + *ss* + *tt* + *uu* + vv equals 4 or 5; and
   ww and xx are each independently 1, 2, 3, 4, or 5.

In some embodiments of peptides of Formula III,
R⁴⁰⁶ is a hydrogen, substituted or unsubstituted C₁-C₆ alkyl group, 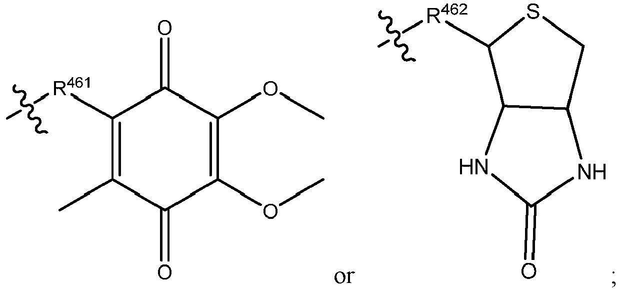 wherein R⁴⁶¹ is a -C₁-C₁₀ alkylene-CO₂- or -CO₂-C₁-C₁₀ alkylene-CO₂-; and R⁴⁶² is C₁-C₁₀ alkylene or C₁-C₁₀ alkylene-CO₂-;
R⁴⁰⁷, R⁴⁰⁸, R⁴⁰⁹, and R⁴¹⁰ are each independently a hydrogen or substituted or unsubstituted C₁-C₆ alkyl group;
R⁴⁵⁵ and R⁴⁶⁰ are each independently a hydrogen, -C(O)-C₁-C₆ alkyl, or methyl group;
R⁴⁵⁶ and R⁴⁵⁷ are each a hydrogen or together R⁴⁵⁶ and R⁴⁵⁷ are C=O;
R⁴⁵⁸ and R⁴⁵⁹ are each a hydrogen or together R⁴⁵⁸ and R⁴⁵⁹ are C=O;
R⁴¹⁶ and R⁴¹⁷ are each independently a hydrogen or -C(O)R^{e};
R⁴¹¹, R⁴¹², R⁴¹³, R⁴¹⁴, R⁴¹⁵, R⁴¹⁸, R⁴¹⁹, R⁴²⁰, R⁴²¹, R⁴²², R⁴⁴³, R⁴⁴⁴, R⁴⁴⁵, R⁴⁴⁶, and R⁴⁴⁷ are each independently a hydrogen, deuterium, methyl, or -OR^{e} group;
R⁴²³, R⁴²⁴, R⁴²⁵, R⁴²⁶, R⁴²⁷, R⁴²⁸, R⁴²⁹, R⁴³⁰, R⁴³¹, R⁴³², R⁴³³, R⁴³⁴, R⁴³⁵, R⁴³⁶, R⁴³⁷, R⁴³⁸, R⁴³⁹, R⁴⁴⁰, R⁴⁴¹, R⁴⁴⁸, R⁴⁴⁹, R⁴⁵⁰, R⁴⁵¹, R⁴⁵², R⁴⁵³, and R⁴⁵⁴ are each independently a hydrogen, NR^{e}R^{e}, or substituted or unsubstituted C₁-C₆ alkyl group;
R⁴⁴² is a -NR^{e}R^{e};
YY, ZZ, and AE are each independently absent or -CH₂-;
AB, AC, AD, and AF are each independently absent or C₁-C₄ alkylene group;
R^{e} at each occurrence is independently a hydrogen or a substituted or unsubstituted C₁-C₆ alkyl group;
*rr, ss,* and vv are each independently 0 or 1; *tt* and *uu* are each 1 with the proviso that *rr* + *ss* + *tt* + *uu* + vv equals 4 or 5; and
ww and xx are each independently 1, 2, 3, 4, or 5.

In some embodiments of peptides of Formula III,
R⁴⁰⁶ is 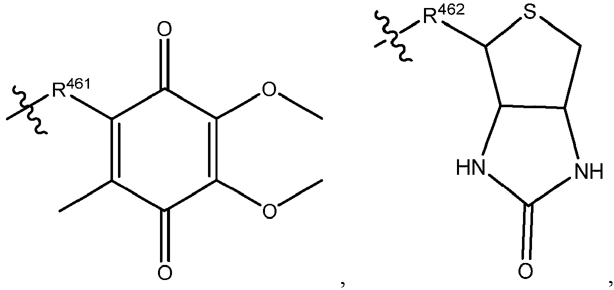 hydrogen, or methyl,
   wherein R⁴⁶¹ is a -(CH₂)₃-CO₂-, -(CH₂)₉-CO₂-, or -CO₂-(CH₂)₂-CO2- and R⁴⁶² is-(CH₂)₄-CO₂-;
   R⁴⁰⁷, R⁴⁰⁸, R⁴⁰⁹, and R⁴¹⁰ are each a hydrogen or methyl group;
   R⁴⁵⁵ and R⁴⁶⁰ are each independently a hydrogen, -C(O)CH₃, or methyl group;
   R⁴⁵⁶ and R⁴⁵⁷ are each a hydrogen or together R⁴⁵⁶ and R⁴⁵⁷ are C=O;
   R⁴⁵⁸ and R⁴⁵⁹ are each a hydrogen or together R⁴⁵⁸ and R⁴⁵⁹ are C=O;
   R⁴¹⁶ and R⁴¹⁷ are each independently a hydrogen or -C(O)CH₃;
   R⁴²⁶, R⁴³⁸, and R⁴⁵¹ are each -N(CH₃)₂;
   R⁴³⁴ and R⁴⁴² are each -NH₂;
   R⁴²³, R⁴²⁴, R⁴²⁵, R⁴²⁷, R⁴²⁸, R⁴²⁹, R⁴³⁰, R⁴³¹, R⁴³², R⁴³³, R⁴³⁵, R⁴³⁶, R⁴³⁷, R⁴³⁹, R⁴⁴⁰, R⁴⁴¹, R⁴⁴³, R⁴⁴⁴, R⁴⁴⁵, R⁴⁴⁶, R⁴⁴⁷, R⁴⁴⁸, R⁴⁴⁹, R⁴⁵⁰, R⁴⁵², R⁴⁵³, and R⁴⁵⁴ are each hydrogen;
   R⁴¹², R⁴¹⁴, R⁴¹⁹, and R⁴²¹ are each independently hydrogen or deuterium;
   R⁴¹¹, R⁴¹⁵, R⁴¹⁸, and R⁴²² are each independently hydrogen, deuterium, or methyl;
   R⁴¹³ and R⁴²⁰ are each independently hydrogen, deuterium, or OR^{e};
   YY, ZZ, and AE are each independently -CH₂-;
   AB, AC, AD, and AF are each -CH₂- or a butylene group;
   R^{e} at each occurrence is independently a hydrogen or a substituted or unsubstituted C₁-C₆ alkyl group;
   *rr, ss,* and vv are each independently 0 or 1; *tt* and *uu* are each 1 with the proviso that *rr* + *ss* + *tt* + *uu* + *vv* equals 4 or 5; and
   ww and xx are each independently 3 or 4.

In certain embodiments of Formula III,
SS is 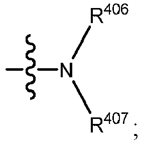
XX is 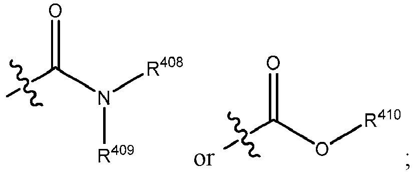
TT, UU, VV, and WW are each independently 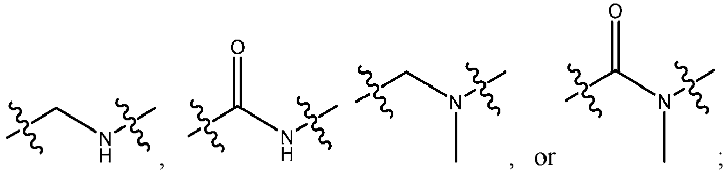 with the proviso when vv is 0 and *uu* is 1, WW is

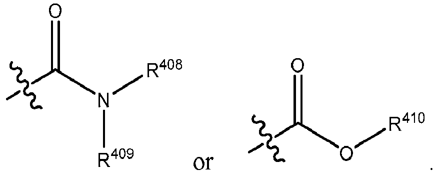

In some embodiments, the peptide of Formula III is selected from the peptides shown in Table D.

| **TABLE D** |
|---|
| 6-Butyric acid CoQ0-Phe-D-Arg-Phe-Lys-NH₂ |
| 6-Decanoic acid CoQ0-Phe-D-Arg-Phe-Lys-NH₂ |
| H-D-N2-acetylarginine-Dmt-Lys-Phe-NH₂ |
| H-D-N8-acetylarginine-Dmt-Lys-Phe-NH₂ |
| H-N2-acetyl-D-arginine-L-Dmt-L-Lys-L-Phe-NH₂ |
| H-N7-acetyl-D-arginine-Dmt-Lys-Phe-NH₂ |
| H-Phe(d5)-D-Arg-Phe(d5)-Lys-NH₂ |
| Succinic monoester CoQ0-Phe-D-Arg-Phe-Lys-HN₂ |
| Dmt-D-Arg-Phe-(atn)Dap-NH₂ |
| Dmt-D-Arg-Phe-(dns)Dap-NH₂ |
| Dmt-D-Arg-Ald-Lys-NH₂ |
| Dmt-D-Arg-Phe-Lys-Ald-NH₂ |
| Bio-2'6'Dmt-D-Arg-Phe-Lys-NH₂ |
| 2'6'Dmt-D-Arg-Phe-dnsDap-NH₂ |
| 2'6'Dmt-D-Arg-Phe-atnDap-NH₂ |
| H-D-Arg-Ψ[CH₂-NH]Dmt-Lys-Phe-NH₂ |
| H-D-Arg-Dmt-Ψ[CH₂-NH]Lys-Phe-NH₂ |
| H-D-Arg-Dmt-LysΨ[CH₂-NH]Phe-NH₂ |
| H-D-Arg-Dmt-Ψ[CH₂-NH]Lys-Ψ[CH₂-NH]Phe-NH₂ |

In some embodiments, the peptide is selected from the peptides shown in Table E.

| **TABLE E** |
|---|
| Arg-D-Leu-D-Tyr-Phe-Lys-Glu-D-Lys-Arg-D-Trp-Lys-D-Phe-Tyr-D-Arg-Gly |
| Asp-Arg-D-Phe-Cys-Phe-D-Arg-D-Lys-Tyr-Arg-D-Tyr-Trp-D-His- Tyr-D-Phe-Lys-Phe |
| D-Glu-Asp-Lys-D-Arg-D-His-Phe-Phe-D-Val-Tyr-Arg-Tyr-D-Tyr-Arg-His-Phe-NH₂ |
| Glu-Arg-D-Lys-Tyr-D-Val-Phe-D-His-Trp-Arg-D-Gly-Tyr-Arg-D-Met-NH₂ |
| Gly-Ala-Lys-Phe-D-Lys-Glu-Arg-Tyr-His-D-Arg-D-Arg-Asp-Tyr-Trp-D-His-Trp-His-D-Lys-Asp |
| His-Tyr-D-Arg-Trp-Lys-Phe-D-Asp-Ala-Arg-Cys-D-Tyr-His-Phe-D-Lys-Tyr-His-Ser-NH₂ |
| Phe-Phe-D-Tyr-Arg-Glu-Asp-D-Lys-Arg-D-Arg-His-Phe-NH₂ |
| Phe-Tyr-Lys-D-Arg-Trp-His-D-Lys-D-Lys-Glu-Arg-D-Tyr-Thr |
| Thr-Tyr-Arg-D-Lys-Trp-Tyr-Glu-Asp-D-Lys-D-Arg-His-Phe-D-Tyr-Gly-Val-Ile-D-His-Arg-Tyr-Lys-NH₂ |
| Tyr-Asp-D-Lys-Tyr-Phe-D-Lys-D-Arg-Phe-Pro-D-Tyr-His-Lys |
| Tyr-D-His-Phe-D-Arg-Asp-Lys-D-Arg-His-Trp-D-His-Phe |
| Phe-Tyr-Lys-D-Arg-Trp-His-D-Lys-D-Lys-Glu-Arg-D-Tyr-Thr |
| Tyr-Asp-D-Lys-Tyr-Phe- D-Lys- D-Arg-Phe-Pro-D-Tyr-His-Lys |
| Glu-Arg-D-Lys-Tyr- D-Val-Phe- D-His-Trp-Arg-D-Gly-Tyr-Arg-D-Met-NH₂ |
| Arg-D-Leu-D-Tyr-Phe-Lys-Glu- D-Lys-Arg-D-Trp-Lys- D-Phe-Tyr-D-Arg-Gly |
| Gly-Ala-Lys-Phe-D-Lys-Glu-Arg-Tyr-His-D-Arg-D-Arg-Asp-Tyr-Trp-D-His-Trp-His-D-Lys-Asp |
| Gly-Ala-Lys-Phe-D-Lys-Glu-Arg-Tyr-His-D-Arg-D-Arg-Asp-Tyr-Trp-D-His-Trp-His-D-Lys-Asp |

In one embodiment, the aromatic-cationic peptides of the present technology have a core structural motif of alternating aromatic and cationic amino acids. For example, the peptide may be a tetrapeptide defined by any of Formulas A to F set forth below:
Aromatic - Cationic - Aromatic - Cationic (Formula A)
Cationic - Aromatic - Cationic - Aromatic (Formula B)
Aromatic - Aromatic - Cationic - Cationic (Formula C)
Cationic - Cationic - Aromatic - Aromatic (Formula D)
Aromatic - Cationic - Cationic - Aromatic (Formula E)
Cationic - Aromatic - Aromatic - Cationic (Formula F)
wherein, Aromatic is a residue selected from the group consisting of: Phe (F), Tyr (Y), and Trp (W). In some embodiments, the Aromatic residue may be substituted with a saturated analog of an aromatic residue, *e.g.,* Cyclohexylalanine (Cha). In some embodiments, Cationic is a residue selected from the group consisting of: Arg (R), Lys (K), and His (H).

The amino acids of the aromatic-cationic peptides of the present technology can be any amino acid. As used herein, the term "amino acid" is used to refer to any organic molecule that contains at least one amino group and at least one carboxyl group. In some embodiments, at least one amino group is at the α position relative to the carboxyl group.

The amino acids may be naturally occurring. Naturally occurring amino acids include, for example, the twenty most common levorotatory (L,) amino acids normally found in mammalian proteins, *i.e.,* alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan, (Trp), tyrosine (Tyr), and valine (Val).

Other naturally occurring amino acids include, for example, amino acids that are synthesized in metabolic processes not associated with protein synthesis. For example, the amino acids ornithine and citrulline are synthesized in mammalian metabolism during the production of urea.

The peptides useful in the present technology can contain one or more non-naturally occurring amino acids. The non-naturally occurring amino acids may be (L-), dextrorotatory (D-), or mixtures thereof. In some embodiments, the peptide has no amino acids that are naturally occurring.

Non-naturally occurring amino acids are those amino acids that typically are not synthesized in normal metabolic processes in living organisms, and do not naturally occur in proteins. In certain embodiments, the non-naturally occurring amino acids useful in the present technology are also not recognized by common proteases.

The non-naturally occurring amino acid can be present at any position in the peptide. For example, the non-naturally occurring amino acid can be at the N terminus, the C-terminus, or at any position between the N-terminus and the C-terminus.

The non-natural amino acids may, for example, comprise alkyl, aryl, or alkylaryl groups. Some examples of alkyl amino acids include α-aminobutyric acid, β-aminobutyric acid, γ-aminobutyric acid, δ-aminovaleric acid, and ε-aminocaproic acid. Some examples of aryl amino acids include ortho-, meta, and para-aminobenzoic acid. Some examples of alkylaryl amino acids include ortho-, meta-, and para-aminophenyl acetic acid, and γ-phenyl-β-aminobutyric acid.

Non-naturally occurring amino acids also include derivatives of naturally occurring amino acids. The derivatives of naturally occurring amino acids may, for example, include the addition of one or more chemical groups to the naturally occurring amino acid.

For example, one or more chemical groups can be added to one or more of the 2', 3', 4', 5', or 6' position of the aromatic ring of a phenylalanine or tyrosine residue, or the 4', 5', 6', or 7' position of the benzo ring of a tryptophan residue. The group can be any chemical group that can be added to an aromatic ring. Some examples of such groups include branched or unbranched C₁-C₄ alkyl, such as methyl, ethyl, n-propyl, isopropyl, butyl, isobutyl, or t-butyl, C₁-C₄ alkyloxy *(i.e.,* alkoxy), amino, C₁-C₄ alkylamino and C₁-C₄ dialkylamino *(e.g.,* methylamino, dimethylamino), nitro, hydroxyl, halo *(i.e.,* fluoro, chloro, bromo, or iodo). Some specific examples of non-naturally occurring derivatives of naturally occurring amino acids include norvaline (Nva), norleucine (Nle), and hydroxyproline (Hyp).

Another example of a modification of an amino acid in a peptide useful in the present methods is the derivatization of a carboxyl group of an aspartic acid or a glutamic acid residue of the peptide. One example of derivatization is amidation with ammonia or with a primary or secondary amine, *e.g.,* methylamine, ethylamine, dimethylamine or diethylamine. Another example of derivatization includes esterification with, for example, methyl or ethyl alcohol.

Another such modification includes derivatization of an amino group of a lysine, arginine, or histidine residue. For example, such amino groups can be alkylated or acylated. Some suitable acyl groups include, for example, a benzoyl group or an alkanoyl group comprising any of the C₁-C₄ alkyl groups mentioned above, such as an acetyl or propionyl group.

In some embodiments, the non-naturally occurring amino acids are resistant, and in some embodiments insensitive, to common proteases. Examples of non-naturally occurring amino acids that are resistant or insensitive to proteases include the dextrorotatory (D-) form of any of the above-mentioned naturally occurring L-amino acids, as well as L- and/or D non-naturally occurring amino acids. The D-amino acids do not normally occur in proteins, although they are found in certain peptide antibiotics that are synthesized by means other than the normal ribosomal protein synthetic machinery of the cell, as used herein, the D-amino acids are considered to be non-naturally occurring amino acids.

In order to minimize protease sensitivity, the peptides useful in the methods of the present technology should have less than five, less than four, less than three, or less than two contiguous L-amino acids recognized by common proteases, irrespective of whether the amino acids are naturally or non-naturally occurring. In some embodiments, the peptide has only D-amino acids, and no L-amino acids.

If the peptide contains protease sensitive sequences of amino acids, at least one of the amino acids is a non-naturally-occurring D-amino acid, thereby conferring protease resistance. An example of a protease sensitive sequence includes two or more contiguous basic amino acids that are readily cleaved by common proteases, such as endopeptidases and trypsin. Examples of basic amino acids include arginine, lysine and histidine. In some embodiments, at least one of the amides in the peptide backbone are alkylated, thereby conferring protease resistance.

It is important that the aromatic-cationic peptides have a minimum number of net positive charges at physiological pH in comparison to the total number of amino acid residues in the peptide. The minimum number of net positive charges at physiological pH is referred to below as (pₘ). The total number of amino acid residues in the peptide is referred to below as (r).

The minimum number of net positive charges discussed below are all at physiological pH. The term "physiological pH" as used herein refers to the normal pH in the cells of the tissues and organs of the mammalian body. For instance, the physiological pH of a human is normally approximately 7.4, but normal physiological pH in mammals may be any pH from about 7.0 to about 7.8.

Typically, a peptide has a positively charged N-terminal amino group and a negatively charged C-terminal carboxyl group. The charges cancel each other out at physiological pH. As an example of calculating net charge, the peptide Tyr-Arg-Phe-Lys-Glu-His-Trp-Arg has one negatively charged amino acid (*i.e.,* Glu) and four positively charged amino acids (*i.e.,* two Arg residues, one Lys, and one His). Therefore, the above peptide has a net positive charge of three.

In one embodiment, the aromatic-cationic peptides have a relationship between the minimum number of net positive charges at physiological pH (pₘ) and the total number of amino acid residues (r) wherein 3pₘ is the largest number that is less than or equal to r + 1. In this embodiment, the relationship between the minimum number of net positive charges (pₘ) and the total number of amino acid residues (r) is as follows:

| **TABLE 1. Amino acid number and net positive charges (3pₘ ≤ p+1)** | | | | | | | | | | | | | | | | | | |
|---|---|---|---|---|---|---|---|---|---|---|---|---|---|---|---|---|---|---|
| (r) | 3 | 4 | 5 | 6 | 7 | 8 | 9 | 10 | 11 | 12 | 13 | 14 | 15 | 16 | 17 | 18 | 19 | 20 |
| (pₘ) | 1 | 1 | 2 | 2 | 2 | 3 | 3 | 3 | 4 | 4 | 4 | 5 | 5 | 5 | 6 | 6 | 6 | 7 |

In another embodiment, the aromatic-cationic peptides have a relationship between the minimum number of net positive charges (pₘ) and the total number of amino acid residues (r) wherein 2pₘ is the largest number that is less than or equal to r + 1. In this embodiment, the relationship between the minimum number of net positive charges (pₘ) and the total number of amino acid residues (r) is as follows:

| **TABLE 2. Amino acid number and net positive charges (2pₘ ≤ p+1)** | | | | | | | | | | | | | | | | | | |
|---|---|---|---|---|---|---|---|---|---|---|---|---|---|---|---|---|---|---|
| (r) | 3 | 4 | 5 | 6 | 7 | 8 | 9 | 10 | 11 | 12 | 13 | 14 | 15 | 16 | 17 | 18 | 19 | 20 |
| (pₘ) | 2 | 2 | 3 | 3 | 4 | 4 | 5 | 5 | 6 | 6 | 7 | 7 | 8 | 8 | 9 | 9 | 10 | 10 |

In one embodiment, the minimum number of net positive charges (pₘ) and the total number of amino acid residues (r) are equal. In another embodiment, the peptides have three or four amino acid residues and a minimum of one net positive charge, or a minimum of two net positive charges, or a minimum of three net positive charges.

It is also important that the aromatic-cationic peptides have a minimum number of aromatic groups in comparison to the total number of net positive charges (pₜ). The minimum number of aromatic groups will be referred to below as (a). Naturally-occurring amino acids that have an aromatic group include the amino acids histidine, tryptophan, tyrosine, and phenylalanine. For example, the hexapeptide Lys-Gln-Tyr-D-Arg-Phe-Trp has a net positive charge of two (contributed by the lysine and arginine residues) and three aromatic groups (contributed by tyrosine, phenylalanine and tryptophan residues).

The aromatic-cationic peptides should also have a relationship between the minimum number of aromatic groups (a) and the total number of net positive charges at physiological pH (pₜ) wherein 3a is the largest number that is less than or equal to pₜ + 1, except that when pₜ is 1, a may also be 1. In this embodiment, the relationship between the minimum number of aromatic groups (a) and the total number of net positive charges (pₜ) is as follows:

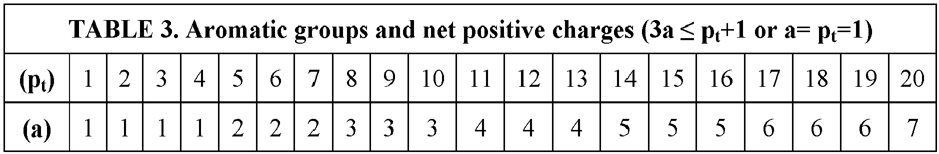

In another embodiment, the aromatic-cationic peptides have a relationship between the minimum number of aromatic groups (a) and the total number of net positive charges (pₜ) wherein 2a is the largest number that is less than or equal to pₜ + 1. In this embodiment, the relationship between the minimum number of aromatic amino acid residues (a) and the total number of net positive charges (pₜ) is as follows:

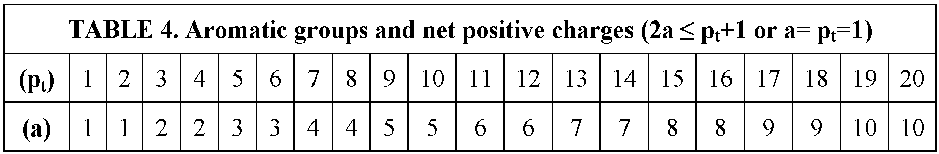

In another embodiment, the number of aromatic groups (a) and the total number of net positive charges (pt) are equal.

In some embodiments, carboxyl groups, especially the terminal carboxyl group of a C-terminal amino acid, are amidated with, for example, ammonia to form the C-terminal amide. Alternatively, the terminal carboxyl group of the C-terminal amino acid may be amidated with any primary or secondary amine. The primary or secondary amine may, for example, be an alkyl, especially a branched or unbranched C₁-C₄ alkyl, or an aryl amine. Accordingly, the amino acid at the C-terminus of the peptide may be converted to an amido, N-methylamido, N-ethylamido, N,N-dimethylamido, N,N-diethyl amido, N-methyl-N-ethylamido, N-phenylamido or N-phenyl-N-ethylamido group.

The free carboxylate groups of the asparagine, glutamine, aspartic acid, and glutamic acid residues not occurring at the C-terminus of the aromatic-cationic peptides of the present technology may also be amidated wherever they occur within the peptide. The amidation at these internal positions may be with ammonia or any of the primary or secondary amines described herein.

In one embodiment, the aromatic-cationic peptide useful in the methods of the present technology is a tripeptide having two net positive charges and at least one aromatic amino acid. In a particular embodiment, the aromatic-cationic peptide useful in the methods of the present technology is a tripeptide having two net positive charges and two aromatic amino acids.

In some embodiments, the aromatic-cationic peptide is a peptide having:
at least one net positive charge;
a minimum of four amino acids;
a maximum of about twenty amino acids;
a relationship between the minimum number of net positive charges (pₘ) and the total number of amino acid residues (r) wherein 3pₘ is the largest number that is less than or equal to r + 1; and a relationship between the minimum number of aromatic groups (a) and the total number of net positive charges (pₜ) wherein 2a is the largest number that is less than or equal to pₜ + 1, except that when a is 1, pₜ may also be 1.

In one embodiment, 2pₘ is the largest number that is less than or equal to r+1, and a may be equal to pₜ. The aromatic-cationic peptide may be a water-soluble peptide having a minimum of two or a minimum of three positive charges.

In one embodiment, the peptide comprises one or more non-naturally occurring amino acids, for example, one or more D-amino acids. In some embodiments, the C-terminal carboxyl group of the amino acid at the C-terminus is amidated. In certain embodiments, the peptide has a minimum of four amino acids. The peptide may have a total of about 6, a total of about 9, or a total of about 12 amino acids.

In one embodiment, the peptides have a tyrosine residue or a tyrosine derivative at the N-terminus *(i.e.,* the first amino acid position). Suitable derivatives of tyrosine include 2'-methyltyrosine (Mmt); 2',6'-dimethyltyrosine (2'6'-Dmt); 3',5'-dimethyltyrosine (3'5'Dmt); N,2',6'-trimethyltyrosine (Tmt); and 2'-hydroxy-6'-methyltyrosine (Hmt).

In one embodiment, a peptide has the formula Tyr-D-Arg-Phe-Lys-NH₂. Tyr-D-Arg-Phe-Lys-NH₂ has a net positive charge of three, contributed by the amino acids tyrosine, arginine, and lysine and has two aromatic groups contributed by the amino acids phenylalanine and tyrosine. The tyrosine of Tyr-D-Arg-Phe-Lys-NH₂ can be a modified derivative of tyrosine such as in 2',6'-dimethyltyrosine to produce the compound having the formula 2',6'-Dmt-D-Arg-Phe-Lys-NH₂. 2',6'-Dmt-D-Arg-Phe-Lys-NH₂ has a molecular weight of 640 and carries a net three positive charge at physiological pH. 2',6'-Dmt-D-Arg-Phe-Lys-NH₂ readily penetrates the plasma membrane of several mammalian cell types in an energy-independent manner (Zhao et al., J. Pharmacol Exp Ther., 304:425-432, 2003).

Alternatively, in some embodiments, the aromatic-cationic peptide does not have a tyrosine residue or a derivative of tyrosine at the N-terminus (*i.e*., amino acid position 1). The amino acid at the N-terminus can be any naturally-occurring or non-naturally-occurring amino acid other than tyrosine. In one embodiment, the amino acid at the N-terminus is phenylalanine or its derivative. Exemplary derivatives of phenylalanine include 2'-methylphenylalanine (Mmp), 2',6'-dimethylphenylalanine (2',6'-Dmp), N,2',6'-trimethylphenylalanine (Tmp), and 2'-hydroxy-6'-methylphenylalanine (Hmp).

An example of an aromatic-cationic peptide that does not have a tyrosine residue or a derivative of tyrosine at the N-terminus is a peptide with the formula Phe-D-Arg-Phe-Lys-NH₂. Alternatively, the N-terminal phenylalanine can be a derivative of phenylalanine such as 2',6'-dimethylphenylalanine (2'6'-Dmp). In one embodiment, the amino acid sequence of 2',6'-Dmt-D-Arg-Phe-Lys-NH₂ is rearranged such that Dmt is not at the N-terminus. An example of such an aromatic-cationic peptide is a peptide having the formula of D-Arg-2'6'-Dmt-Lys-Phe-NH₂.

Suitable substitution variants of the peptides listed herein include conservative amino acid substitutions. Amino acids may be grouped according to their physicochemical characteristics as follows:
(a) Non-polar amino acids: Ala(A) Ser(S) Thr(T) Pro(P) Gly(G) Cys (C);
(b) Acidic amino acids: Asn(N) Asp(D) Glu(E) Gln(Q);
(c) Basic amino acids: His(H) Arg(R) Lys(K);
(d) Hydrophobic amino acids: Met(M) Leu(L) Ile(I) Val(V); and
(e) Aromatic amino acids: Phe(F) Tyr(Y) Trp(W) .

Substitutions of an amino acid in a peptide by another amino acid in the same group are referred to as a conservative substitution and may preserve the physicochemical characteristics of the original peptide. In contrast, substitutions of an amino acid in a peptide by another amino acid in a different group are generally more likely to alter the characteristics of the original peptide.

The amino acids of the peptides disclosed herein may be in either the L- or the D-configuration.

### Treating or Preventing Mitochondrial Myopathy

In one aspect of the present technology, the aromatic-cationic peptide disclosed herein, such as D-Arg-2',6'-Dmt-Lys-Phe-NH₂, or a pharmaceutically acceptable salt thereof, are useful for treating or preventing mitochondrial myopathy and/or treating or preventing the signs or symptoms of mitochondrial myopathy in a subject in need thereof.

Mitochondrial myopathies comprise a group of inherited disorders caused by mutations in either mitochondrial or nuclear DNA resulting in defective mitochondrial metabolism. The term mitochondrial myopathy is used as the descriptive term for a group of disorders where muscle disease is an important manifestation, albeit one that is rarely found in isolation. Mitochondrial myopathies may affect multiple organ systems and tissues including the brain and eye.

The clinical manifestations of mitochondrial myopathies are variable but often include muscle weakness, exercise intolerance, including exercise-induced cramps and myalgia, ptosis and progressive external opthalmoplegia (PEO), which can be mild or result in complete paralysis of extraocular muscles, rhabdomyolysis, and myoglobiniuria. Although PEO is a common manifestation, it is not an obligate finding in mitochondrial myopathies caused by mtDNA mutations. The reason for the variability in clinical manifestations of mitochondrial myopathies caused by mtDNA mutations has yet to be uncovered; however, heteroplasmy, replicative segregation, and threshold effects of the mtDNA mutations are likely important factors in pathogenesis. Defects of oxidative phosphorylation caused by nuclear DNA mutations typically show a generalized reduction in the activity of the affected respiratory chain enzyme complex, whereas mutations of mtDNA result in in a mosaic pattern of enzyme deficiency where muscle cells with normal enzyme activity are mixed with muscle cells with defective activity. Segments of muscle cells where the proportion of mutant mtDNA exceeds the threshold level will show enzyme deficiency.

Morphological analyses of muscle biopsies obtained from patients with mitochondrial myopathy can reveal certain typical alterations. An indication of mitochondrial myopathy is the ragged-red fiber (RRF), which is an abnormal muscle fiber exhibiting an excessive accumulation of mitochondria that are stained red by the modified Gomori trichrome technique. The mitochondria of RRF are usually ultrastructurally abnormal and often contain paracrystalline inclusions. Many patients with ragged-red fibers often exhibit encephalomyopathy. However, the absence of ragged-red fibers in a biopsy does not exclude a mitochondrial etiology.

Several readily measurable clinical markers or energy biomarkers, described in more detail below, are used to assess the metabolic state of patients with mitochondrial myopathy. These clinical markers can also be used as indicators of the efficacy of a given therapy, as the level of a marker is moved from the pathological value to the healthy value. These clinical markers include, but are not limited to, one or more of lactic acid (lactate) levels, either in whole blood, plasma, cerebrospinal fluid, or cerebral ventricular fluid; pyruvic acid (pyruvate) levels, either in whole blood, plasma, cerebrospinal fluid, or cerebral ventricular fluid; lactate/pyruvate ratios, either in whole blood, plasma, cerebrospinal fluid, or cerebral ventricular fluid; phosphocreatine levels, NADH (NADH+H⁺) or NADPH (NADPH+H⁺) levels; NAD or NADP levels; ATP levels; anaerobic threshold; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels, beta-hydroxy butyrate levels, acetoacetate/beta-hydroxy butyrate ratio, 8-hydroxy-2'-deoxyguanosine (8-OHdG) levels; levels of reactive oxygen species; and levels of oxygen consumption (VO₂), levels of carbon dioxide output (VCO₂), and respiratory quotient (VCO₂/VO₂). Several of these clinical markers are measured routinely in exercise physiology laboratories, and provide convenient assessments of the metabolic state of a subject.

Lactic acid levels: Mitochondrial dysfunction typically results in abnormal levels of lactic acid, as pyruvate levels increase and pyruvate is converted to lactate to maintain capacity for glycolysis. Lactate levels can be measured by taking samples of appropriate bodily fluids such as whole blood, plasma, or cerebrospinal fluid. Using magnetic resonance, lactate levels can be measured in virtually any volume of the body desired, such as the brain.

NAD, NADP, NADH and NADPH levels: Mitochondrial dysfunction can also result in abnormal levels of NADH+H⁺, NADPH+H⁺, NAD, or NADP, as the reduced nicotinamide adenine dinucleotides are not efficiently processed by the respiratory chain. NAD, NADP, NADH (NADH+H⁺) or NADPH (NADPH+H⁺) can be measured by a variety of fluorescent, enzymatic, or electrochemical techniques.

Oxygen consumption (VO₂), carbon dioxide output (VCO₂), and respiratory quotient (VCO₂/VO₂): VO₂ is usually measured either while resting (resting VO₂) or at maximal exercise intensity (VO₂ max). Optimally, both values will be measured. However, for severely disabled patients, measurement of VO₂ max may be impractical. Measurement of both forms of VO₂ is readily accomplished using standard equipment from a variety of vendors, e.g., Korr Medical Technologies, Inc. (Salt Lake City, Utah). VCO₂ can also be readily measured, and the ratio of VCO₂ to VO₂ under the same conditions (VCO₂/VO₂, either resting or at maximal exercise intensity) provides the respiratory quotient (RQ).

Oxidized Cytochrome C, reduced Cytochrome C, and ratio of oxidized Cytochrome C to reduced Cytochrome C: Cytochrome C parameters, such as oxidized cytochrome C levels (Cyt C^{ox}), reduced cytochrome C levels (Cyt C^{red}), and the ratio of oxidized cytochrome C/reduced cytochrome C ratio (Cyt C^{ox})/(Cyt C^{red}), can be measured by *in vivo* near infrared spectroscopy.

Exercise testing is particularly helpful as an evaluation and screening tool in mitochondrial myopathies. One of the hallmark characteristics of mitochondrial myopathies is a reduction in maximal whole body oxygen consumption (VO₂ max). Given that VO₂ max is determined by cardiac output (Qc) and peripheral oxygen extraction (arterial-venous total oxygen content) difference, some mitochondrial myopathies affect cardiac function where delivery can be altered; however, most mitochondrial myopathies show a characteristic deficit in peripheral oxygen extraction (A-V O₂ difference) and an enhanced oxygen delivery (hyperkinetic circulation). This can be demonstrated by a lack of exercise-induced deoxygenation of venous blood with direct AV balance measurements and non-invasively by near infrared spectroscopy. The six minute walk test (6MWT) is commonly used as an exercise tolereance test to assess mitochondrial myopathy.

Mitochondrial myopathies can be caused by either mtDNA mutations or nuclear DNA mutations. As described in more detail below, there are several autosomal dominant or recessive inherited mitochondrial myopathies with multiple deletions of mtDNA. Mitochondrial myopathies caused by mtDNA point mutations are usually maternally inherited, whereas large-scale mtDNA deletions are typically sporadic and not maternally inherited. Accordingly, for some subjects, diagnosis can be confirmed by identification of a mtDNA mutation on molecular genetic testing of DNA extracted from a blood sample. In some cases, and a more structured approach is needed, including family history, blood and/or CSF lactate concentration measurements, neuroimaging, cardiac evaluation, and molecular genetic testing for a mtDNA or nuclear gene mutation.

In some embodiments, mitochondrial myopathy is selected from the diseases and/or associated with the diseases or conditions below:

### Kearns-Sayre Syndrome

Kearns-Sayre Syndrome (KSS), also known as oculocranisomatic disorder or oculocraniosomatic neuromuscular disorder with ragged-red fibers, is a mitochondrial myopathy that is caused by various mitochondrial deletions. Single large mtDNA deletions (2 to 8 kb) account for 80% of KSS mutations. The mtDNA deletions that cause KSS result in the impairment of oxidative phosphorylation and a decrease in cellular energy production. In most instances, KSS arises from sporadic somatic mutations occurring after conception. Rarely, the mutation is transmitted through maternal inheritance.

KSS is characterized by a triad of features including: (1) typical onset in persons younger than age 20 years; (2) chronic, progressive, external ophthalmoplegia (PEO); and (3) pigmentary degeneration of the retina (retinitis pigmentosa). Additional clinical features include heart block, limitation or absence of movement in all fields of gaze, ptosis, cataracts, dysphagia, weight loss, weakness, occasional fatigue or pain on exertion, sensory-motor polyneuropathy, stroke, reduced respiratory drive, bilateral sensorineural deafness, ataxia, dementia, or impaired intellect, spasticity, growth hormone deficiency, increased tendon reflexes, endocrinopathies, glucose intolerance, hypothyroidism, hypoparathyroidism, short stature, ragged-red fibers, variation in muscle fiber size, lactic acidosis, high CSF protein levels *(e.g.,* >100 mg/dL), low 5-methyltetrahydrofolate (5-MTHF) in CSF, high homovanillic acid (HVA) in CSF, abnormal choroid plexus function, basal ganglia calcifications, cerebral and cerebellar atrophy, status spongiosis in gray and white matter, and proximal renal tubular acidosis.

Another variant syndrome related to KSS, or other disorders having a single large mtDNA deletion include 2-oxoadipic aciduria and 2-aminoadipic aciduria. Affected patients exhibit episodes of ketosis and acidosis, and may experience coma.

### Leber's hereditary optic neuropathy (LHON)

Leber's hereditary optic neuropathy (LHON) is a maternally inherited blinding disease with variable penetrance. LHON is usually due to one of three pathogenic mitochondrial DNA (mtDNA) point mutations. These mutations are at nucleotide positions 11778 G to A, 3460 G to A and 14484 T to C, respectively in the MTND4, MTND1 and MTND6 subunit genes of complex I of the oxidative phosphorylation chain in mitochondria. Reduced efficiency of ATP synthesis and increased oxidative stress are believed to sensitize the retinal ganglion cells to apoptosis.

Leber's hereditary optic neuropathy (LHON) is characterized by severe visual loss, which usually does not manifest until young adulthood. Maternal transmission is due to a mitochondrial DNA (mtDNA) mutation affecting nucleotide positions (nps) 11778/ND4, 14484/ ND6, or 3460/ND1. These three mutations, affecting respiratory complex I, account for about 95% of LHON cases. Patients inherit multicopy mtDNA entirely from the mother (via the oocyte). The mitochondria may carry only wild-type or only LHON mutant mtDNA (homoplasmy), or a mixture of mutant and wild-type mtDNA (heteroplasmy). Only high loads of mutant heteroplasmy or, most frequently, homoplasmic mutant mtDNA in the target tissue put the subject at risk for blindness from LHON. Except for patients carrying the 14484/ND6 mutation (who present with a more benign disease course), most patients remain legally blind. Typically, a subject in his second or third decade of life will present with abrupt and profound loss of vision in one eye, followed weeks to months later by similar loss of vision in the other eye. LHON may occur later in life and affects both men and women. Environmental factors may trigger the visual loss but do not fully explain why only certain individuals within a family become symptomatic. Additional symptoms include disc microangiopathy, pseudo disc edema, vascular tortuosity, optic atrophy, cardiac conduction defects, spastic paraparesis, sexual and urinary disturbances, Sudden Infant Death Syndrome, abnormal visual evoked potentials, spastic dystonia and encephalopathy.

Disruptions in ND4 can also lead to spastic paraparesis which is associated with leg stiffness, abnormal visual evoked potentials and sexual and urinary disturbances.

### Leigh Syndrome

Leigh syndrome (LS), also referred to as subacute necrotizing encephalopathy, is a rare, inherited, early-onset progressive neurodometabolic disorder with a characteristic neuropathology consisting of focal, bilateral lesions in one or more areas of the central nervous system, including the brainstem, thalamus, basal ganglia, cerebellum, and spinal cord. The lesions are areas of demyelination, gliosis, necrosis, spongiosis, or capillary proliferation. A common laboratory finding is lactic acidemia and an increase of the lactate/pyruvate ratio in blood, urine, and cerebrospinal fluid. Clinical features also include hypotonia, ataxia, vomiting, choreoathetosis, hyperventilation, encephalopathy, loss verbal milestones, motor spasticity, abnormal breathing rhythm, hearing loss, nystagmus, dystonia, vision loss, ophthalmoparesis, optic atrophy, peripheral neuropathy, intercurrent infection, cog-wheel rigidity, distal renal tubular acidosis, limb athetosis, seizures, carbohydrate intolerance, COX deficiency in muscle, lactic acidosis with hypoglycemia, kyphoscoliosis, short stature, brisk tendon reflexes, obesity, and high lactate levels in CSF. Clinical symptoms depend on which areas of the central nervous system are involved. The most common underlying cause is a defect in oxidative phosphorylation. Symptoms usually manifest between the ages of three months and two years and progress rapidly leading to death within two years of onset.

In addition to the observation of the various clinical signs of Leigh syndrome, diagnosis of LS typically involves several procedures. As noted above, a common laboratory finding is lactic acidemia and an increase of the lactate/pyruvate ratio in blood, urine, and cerebrospinal fluid. Diagnostic imaging with MRI may be used to detect the bilateral, symmetrical hyperintensities in T2-weighted images that are a characteristic finding in LS. Additional alterations may include necrotizing leukoencephalopathy, supratentorial stroke-like lesions, and cortical or cerebellar atrophy. Biochemical analysis of muscle biopsies can also serve as an important tool in diagnosing LS. In addition, genetic diagnostics can be used to screen particular candidate genes or whole exome sequencing in unclear cases.

Leigh syndrome mutations have been identified in both nuclear- and mitochondrial-encoded genes involved in energy metabolism, including mitochondrial respiratory chain complexes I, II, III, IV, and V, which are involved in oxidative phosphorylation and the generation of ATP, and components of the pyruvate dehydrogenase complex.

Mutations in complex I genes include mitochondrial-encoded *MTND2*, *MTND3, MTND5,* and *MTND6,* the nuclear-encoded *NDUFS1, NDUFS3, NDUFS4, NDUFS7, NDUFS8, NDUFA2, NDUFA9, NDUFA10, NDUFA12, NDUFAF6, FOXRED1, COXPD15* and *C20ORF7,* and the complex I assembly factor *NDUFAF2.* A mutation in the *MTFMT* gene, which is involved in mitochondrial translation, has also been reported with complex I deficiency.

Leigh syndrome is also associated with mutations in complex I (C8ORF38), complex II (the flavoprotein subunit A (*SDHA*)); complex III (*BCSIL*); complex IV (*MTCO3, COX10, COX15, SCO2, SURF1, TACO1,* and *PET100*); complex V (*MTATP6*); mitochondrial tRNA proteins (*MTTV, MTTS2, MTTK, MTTW,* and *MTTL1*); components of the pyruvate dehydrogenase complex (*e.g., DLD* and *PDHA1*); the *LRPPRC* gene; and coenzyme Q₁₀.

Mutations in *NDUFV1, NDUFV2, MTND2, MTND5,* and *MTND6* can result in Leigh syndrome due to mitochondrial complex I deficiency. Clinical symptoms include reduced Complex I activity, hypertrophic cardiomyopathy, developmental delay, cerebral atrophy, hypoplasia of the corpus callosum, acidosis, seizures, coma, cardiovascular arrest, demyelinization of corticospinal tracts, subacute necrotizing encephalomyelopathy, progressive encephalopathy, respiratory failure, exercise intolerance, weakness, mitochondrial proliferation in muscle, motor retardation, hypotonia, deafness, dystonia, pyramidal features, brainstem events with oculomotor palsies, strabismus and recurrent apnea, lactic acidemia, and basal ganglia lesions.

Mutations in *MTCO3* can result in Leigh syndrome that usually presents at 4 years of age. Clinical symptoms include spastic paraparesis with ophthalmoplegia, high serum lactic acid, Leigh-like lesions in putamen, and reduced COX activity in muscle. Mutations also found in *MTCO3* cause Leber's hereditary optic neuropathy (LHON), myopathy with exercise intolerance, rhabdomyolysis, episodic encephalopathy, and nonarteritic ischemic optic neuropathy (NAION)-Myoclonic epilepsy.

Mutations in 3-hydroxyisobutyryl-CoA hydrolase (HIBCH) results in β-Hydroxyisobutyryl CoA Deacylase (HIBCH) deficiency, a Leigh-like syndrome that usually manifests at neonatal to 6 months of age. Symptoms include hypotonia, regression, poor feeding, dystonia, ataxia, seizures, dysmorphic facies, vertebral anomalies, tetralogy of fallot, progressive or acute encephalopathy, respiratory chain deficiencies, high CSF lactate, basal ganglia abnormalities, brain agenesis and accumulation of metabolites (e.g., methacrylyl-CoA, acryloyl-CoA, hydroxy-C4-carnitine).

Mutations in enoyl-CoA hydratase, short-chain, 1 (*ECHS1*) results in short chain enoyl-CoA hydratase (ECHS1) deficiency, a Leigh-like syndrome that presents at the neonatal stage. ECHS1 catalyzes the second step in mitochondrial fatty acid β-oxidation. Clinical symptoms include hypotonia, respiratory insufficiency or apnea, bradycardia, developmental delay, high serum lactate, white matter atrophy, and accumulation of metabolites (e.g., methacrylyl-CoA, acryloyl-CoA).

Mutations in ATP synthase 6 (*MTATP6*) result in Maternal Inheritance Leigh Syndrome (MILS). Clinical symptoms include hypotonia, developmental delay, peripheral neuropathy, seizures, retinitis pigmentosa or optic atrophy, ataxia, respiratory failure, bilateral striatal necrosis, hereditary spastic paraparesis, myelopathy, limb spasticity, weakness, and sensory loss or pain.

### MEGDEL Syndrome

MEGDEL (3-methylglutaconic aciduria, deafness, encephalopathy, and Leigh-like disease) syndrome is an autosomal recessive disorder characterized by childhood onset of delayed psychomotor development or psychomotor regression, sensorineural deafness, spasticity or dystonia, and increased excretion of 3-methylglutaconic acid. MEGDEL is caused by homozygous or compound heterozygous mutations in the SERAC1 gene, which plays a role in phospholipid exchange and intracellular cholesterol trafficking. Brain imaging of affected subjects shows cerebral and cerebellar atrophy as well as lesions in the basal ganglia reminiscent of Leigh Syndrome. Clinical symptoms include hypotonia, encephalopathy (Leigh-like Syndrome), mental retardation, sensorineural deafness, spasticity, dystonia, hepatopathy, increased serum lactate and alanine, hyperammonemia, 3-Methylglutaconic aciduria, high transaminases, coagulopathy, high serum α-fetoprotein, mitochondrial oxidative phosphorylation defects, abnormal mitochondria, abnormal phosphatidylglycerol and cardiolipin profiles in fibroblasts, and abnormal accumulation of unesterified cholesterol within cells.

### Mitochondrial DNA Depletion Syndrome (MDS)

Mitochondrial DNA Depletion (or Deletion) Syndrome (MDS) refers to a group of autosomal recessive disorders that cause the affected tissues to exhibit a significant reduction in mitochondrial DNA (mtDNA) or a mutation (e.g., a deletion) in the mtDNA. Symptoms may manifest as myopathic, hepatopathic, and/or encephalomyopathic. The myopathic form of MDS, also known as mitochondrial DNA depletion syndrome-2 (MTDPS2), is characterized primarily by childhood onset of muscle weakness associated with depletion of mtDNA in skeletal muscle. MTDPS2 is caused by homozygous or compound heterozygous mutations in the nuclear-encoded mitochondrial thymidine kinase gene (*TK2*). Because *TK2* plays a key role in the mitochondrial salvage pathways of several deoxyribonucleoside triphosphates (dNTPs), a lowered activity would lead to less cycling of nucleotides. This lack of nucleotide recycling is detrimental since the mitochondria cannot synthesize entirely new deoxynucleotides, and the inner membrane of the mitochondria prevents the negatively charged nucleotides of the cytosol from entering the organelle. There is wide clinical variability; some patients have onset in infancy and show a rapidly progressive course with early death due to respiratory failure, whereas others have later onset of a slowly progressive myopathy.

Clinical signs and symptoms of MTDPS2 include elevated serum creatine kinase levels, gait impairment, hypotonia, weakness, respiratory failure, paralysis, gynecomastia, myopathy, chronic partial denervation, mtDNA depletion, reduced Complex I, III, IV and V activity, and elevated plasma lactate.

Variant TK2 syndromes include spinal muscular atrophy syndrome, rigid spine syndrome, and severe myopathy with motor regression.

### Mitochondrial Myopathy, Encephalopathy, Lactic Acidosis, and Stroke-Like Episodes (MELAS)

MELAS syndrome, comprising mitochondrial myopathy, encephalopathy, lactic acidosis, and stroke-like episodes, is a genetically heterogeneous mitochondrial disorder with a variable clinical phenotype. MELAS syndrome can be caused by mutations in several genes, including *POLG, MTTL1, MTTQ, MTTH, MTTK, MTTF, MTTC, MTTS1, MTTV, MTTQ, MTND1, MTND3, MTND5, MTND6, MTCOI,* cytochrome b, and *MTTS2,* with mutations in *MTTL1* accounting for the majority of MELAS cases. In particular, it is estimated that approximately 80% of MELAS patients have an A3243G point mutation in the *MTTL1* gene. A distinctive feature of MELAS syndrome is the patient's normal birth and early development. Although the patient harbors the mtDNA mutation from birth, the individual initially remains symptom free because energy output is sufficient to meet the body's requirements. Symptoms develop once the metabolic demands of the body exceed the energy supply available from the defective mitochondria. The age of onset of MELAS is variable, ranging from younger than 2 years to older than 60 years, with the majority of patients developing symptoms between the ages of 5 and 15.

MELAS is a multisystem disorder with no single feature leading to a definitive diagnosis. However, 90% of all cases include six core signs and symptoms and at least two secondary criteria that are required to confirm diagnosis. The six core features include: age of symptom onset before 40 years; encephalopathy, often manifesting in seizures, dementia, or both; exercise intolerance; lactic acidosis; ragged-red fibers on biopsy; and stroke-like episodes. Additional features to confirm diagnosis include: normal early development; recurrent headache; and recurrent vomiting. One of the nearly absolute findings in MELAS syndrome is an elevated lactate level in both the cerebrospinal fluid and serum. Ragged-red fibers are seen in 80-100% of skeletal muscle biopsy specimens.

Additional clinical symptoms include distal arthrogryposis, headache and vomiting, sensorineural hearing loss, seizures, loss of consciousness, dementia, mental retardation, focal events (strokes), cortical visual defects, hemiplegia, neuronal hyperexcitability, basal ganglia calcifications, weakness, exercise intolerance, ptosis, external ophthalmoplegia, gait disorder, paresthesias and numbness, reduced tendon reflexes, sensory neuropathy, chorea, Parkinsonism, ataxia, pigmentary retinopathy, macular dystrophy, optic atrophy, visual field defects, hypertelorism, hypertrophic cardiomyopathy, left ventricular noncompaction, conduction defects (such as Wolff-Parkinson-White), hypertension, short stature, maternally inherited diabetes (MIDD), pancreatitis, constipation, diarrhea, intestinal pseudoobstruction (ileus), nausea, dysphagia, abdominal pain, epigastralgia, sialoadenitis focal segmental glomerulosclerosis, renal cysts, tubular dysfunction, nephrotic syndrome, multihormonal hypopituitarism, Hashimoto thyroiditis, goiter, hypoparathyroidism, Addison's disease, ovarian failure, miscarriage, lipoma, atopic dermatitis, local melanoderma, asymmetric vascular dilatation, lactic acidosis, white matter lesions, respiratory chain dysfunction, ragged red fibers, cortical atrophy, focal necrosis, Purkinje dendrite cactus formations with increased mitochondria, and mitochondrial capillary angiopathy.

### Mitochondrial Neurogastrointestestinal Encephalomyopathy (MNGIE)

Mitochondrial DNA Depletion Syndrome-1 (MTDPS1), which manifests as a mitochondrial neurogastrointestinal encephalopathy (MNGIE), is caused by homozygous or compound heterozygous mutations in the nuclear-encoded thymidine phosphorylase gene (*TYMP*). TYMP catalyzes phosphorolysis of thymidine to thymine and deoxyribose 1-phosphate, and plays a role in homeostasis of cellular nucleotide pools. Mitochondrial DNA Depletion Syndrome-1 (MTDPS1) is an autosomal recessive progressive multisystem disorder clinically characterized by onset between the second and fifth decades of life of ptosis, progressive external ophthalmoplegia (PEO), retinal degeneration, optic atrophy, gastrointestinal dysmotility (often pseudoobstruction, gastroparesis, obstipation, malabsorption, diarrhea, abdominal pain and cramps, nausea and vomiting), borborygmi, early satiety, cachexia, thin body habitus, short stature, diffuse leukoencephalopathy, myopathy (proximal weakness, exercise intolerance), peripheral neuropathy (sensory loss/pain/ataxia, weakness, tendon reflexes absent, axonal loss, demyelination), hearing loss, cognitive impairment or dementia, seizures, headaches, and mitochondrial dysfunction. Mitochondrial DNA abnormalities can include depletion, deletion, and point mutations. MNGIE usually presents at < 20 years of age. Additional symptoms include incomplete right bundle branch block (cardiac defect), diabetes or glucose intolerance, amylase increase, exocrine insufficiency, neoplasms, lactic acidosis, elevated plasma thymidine levels, elevated plasma deoxyuridine and deoxythymidine levels, tetany, cardiac arrhythmia, high CSF protein, brain atrophy, mitochondrial changes in muscle fibers and neurogenic changes.

Partial loss of thymidine phosphorylase activity can result in a variant MNGIE disorder that manifests around the fifth decade of life. Clinical symptoms include ophthalmoplegia, ptosis, gastrointestinal features, and axon loss with or without demyelination.

Mitochondrial DNA Depletion Syndrome-4B (MTDPS4B), which manifests as a mitochondrial neurogastrointestinal encephalopathy (MNGIE), is caused by compound heterozygous mutations in the nuclear-encoded *POLG* gene. Mitochondrial DNA Depletion Syndrome-4B is an autosomal recessive progressive multisystem disorder clinically characterized by chronic gastrointestinal dysmotility and pseudoobstruction, cachexia, progressive external ophthalmoplegia (PEO), axonal sensory ataxic neuropathy, and muscle weakness.

Another MNGIE variant is MNGIM Syndrome without encephalopathy, which is not associated with mutations in thymidine phosphorylase or dNT-2. Clinical features include gastrointestinal malabsorption, diarrhea, borborygmi, abdominal pain, GI pseudoobstruction, weight loss, ophthalmoplegia, ptosis, weakness, cachexia, polyneuropathy (pain, gait disorder, sensory ataxia, axonal loss), high CSF protein, ragged red fibers, and reduced Complex I-IV activities.

Mutations in *MTTW* can manifest as a mitochondrial neurogastrointestinal encephalopathy (MNGIE). Patients present at 1 year of age with recurrent vomiting and failure to thrive. Leg discomfort, cognitive regression, seizures, muscle wasting, and incontinence manifest later during childhood. Other features include sensorineural deafness, ptosis, ophthalmoplegia, pigmentary retinopathy, constricted visual fields, short stature, feeding difficulties with constipation, colitis and diarrhea, high lactate levels in blood and CSF, brain atrophy, and periventricular white matter changes. Muscle biopsies show COX-negative fibers and low activity of Complexes I and IV.

Mutations in *MTTV* can manifest as a neurogastrointestinal encephalopathy (MNGIE). Age of onset is usually during early childhood. Clinical symptoms include cachexia, headache, gastrointestinal motility problems (ileus, abdominal pain; megacolon), hearing loss, developmental delay, high serum lactate, COX-negative fibers and low activity of complexes I and IV. Disruption of *MTTV* function can also lead to ataxia, seizures and hearing loss, and learning difficulties, hemiplegia and movement disorder.

### Myoclonic Epilepsy Ragged Red Fibers (MERRF)

MERRF syndrome represents a maternally-inherited mitochondrial myopathy that can be produced by mutations in more than one mitochondrial gene, *e.g., MTTK, MTTL1, MTTH, MTTS1, MTTS2,* and *MTTF.* Features of MERRF syndrome have also been associated with mutations in the *MTND5* gene. In some embodiments, mutations in the mtDNA include, but are not limited to, m.8334A>G and m.8344A>G.

Clinical features of MERRF include myoclonus, epilepsy, cardiomyopathy, ataxia, gait disorder, dementia, optic atrophy, distal sensory loss, hearing loss, weakness, muscle pain, cramps, fatigue, short stature, lipomata, ragged-red fibers, vacuoles in small fibers, and reduced Complex I, III and IV activity.

Other *MTTK* syndromes include cardiomyopathy, progressive external ophthalmoplegia with myoclonus, deafness and diabetes (DD), multiple symmetric lipomatosis, Leigh syndrome, MELAS, MNGIE, Myopathy with Episodic high Creatine Kinase (MIMECK), Parkinson syndrome neuropathy and myopathy. Clinical features of MIMECK include weakness, dysphagia, and episodic myalgias.

Other *MTTS1* disorders include MELAS, Epilepsia Partialis Continua, HAM syndrome, myopathy, encephalopathy with cytochrome c oxidase deficiency, myoclonus, epilepsy, cerebellar ataxia and progressive hearing loss, exercise intolerance, keratoderma, palmoplantar, with deafness, and sensorineural hearing loss.

Mutations in *MTTP* can also result in myoclonic epilepsy, myopathy, sensorineural deafness, cerebellar ataxia, and pigmentary retinopathy.

### Neuropathy; Ataxia; and Retinitis Pigmentosa (NARP)

Neuropathy, ataxia, and retinitis pigmentosa (NARP) syndrome is caused by mutations in the mitochondrial gene encoding subunit 6 of mitochondrial H(+)-ATPase (*MTATP6*) with an onset ranging from infancy to adulthood. The MT-ATP6 protein forms one subunit of complex V (ATP synthase), which is responsible for the last step in ATP production. Mutations in MT-ATP6 alter the structure or function of ATP synthase, reducing the ability of mitochondria to produce ATP. Most individuals with NARP have a specific point mutation at nucleotide 8993, with a T8993G mutation causing more severe symptoms than a T8993C mutation. Some cases involve a G8989C point mutation.

Clinical features of NARP include sensory neuropathy, proximal and distal weakness, reduced tendon reflexes, retinitis pigmentosa, reduced night vision, bull's eye maculopathy, pigment in posterior pole and mid-periphery, small retinal scars, vascular narrowing, central and paracentral scotomas, gait disorder, dysarthria, dementia, seizures, tonic-clonic seizures, developmental delay, pyramidal signs, dystonia, hearing loss, cardiac hypertrophy, denervation, cerebral atrophy, cortical cerebellar atrophy, focal cystic necrosis, and rod or cone dysfunction.

### Mutations in OPA1 Gene

The *OPA1* gene encodes a dynamin-related GTPase, which is targeted to mitochondria by an N-terminus import sequence motif and is anchored to the inner mitochondrial membrane facing the intermembrane space. The OPA1 protein includes a transmembrane domain, a GTPase domain, a middle domain, and a coiled-coil domain. The heptad repeat domains represent structural protein motifs that are commonly found in coiled-coils. The C-terminal coiled-coil is necessary for mitochondrial fusion. The OPA1 protein is present in mammalian cells in eight isoforms resulting from alternative splicing. The OPA1 isoforms range from 960 amino acids to 1,105 amino acids in length. The OPA1 protein isoforms are variably expressed in different tissues, with the highest levels expressed in the retina, brain, testis, heart, and muscle.

Mutations in the *OPA1* gene can lead to progressive external ophthalmoplegia, ragged red fibres (RRF), cytochrome c oxydase negative fibres (COX), and/or mitochondrial myopathy. By way of example, but not by way of limitation, in some embodiments, the mutation in *OPA1* gene that causes one or more of the above conditions, include, but it not limited to, c.1316 G > T (p.G439V); c.2729 T > A (p.V910D), c.1334 G > A (p.R455H), c.1635 C > G (p.S545R), and c.1069 G > A (p.A357T).

### Pearson Syndrome

Pearson syndrome is caused by sporadic deletions in mitochondrial DNA and is characterized by sideroblastic anemia and exocrine pancreas dysfunction. In individuals with Pearson syndrome, the bone marrow fails to produce white blood cells called neutrophils. The syndrome causes severe anemia, low platelet count, and aplastic anemia. Sideroblastic anemia is defined by the presence of anemia and ringed sideroblasts in the bone marrow. Ringed sideroblasts are normoblasts with excessive deposits of iron in mitochondria and are detected by iron stains of bone marrow. Pearson syndrome causes exocrine pancreas dysfunction as a result of pancreatic scarring and atrophy. Exocrine pancreatic dysfunction is manifest clinically by steatorrhea, which can be documented qualitatively by Sudan staining of the feces or quantitatively by measuring fecal fat. The gold standard is the secretin stimulation test, which requires placing a catheter in the duodenum and is difficult to perform in infants. Individuals with this condition have difficulty absorbing nutrients from their diet which leads to malabsorption. Infants with this condition generally do not grow or gain weight.

Other clinical features of Pearson syndrome include failure to thrive, pancytopenic crises, pancreatic fibrosis with insulin-dependent diabetes and exocrine pancreatic deficiency, muscle and neurologic impairment, malabsorption, steatorrhea, metabolic and lactic acidosis, and early death. The few patients who survive into adulthood often develop symptoms of Kearns-Sayre Syndrome (KSS).

### Progressive External Ophthalmoplegia (PEO)

Progressive external ophthalmoplegia (PEO) is a slowly progressive disorder associated with slow eye movement speed, limited gaze in all directions, ptosis, extraocular muscle pathology, and variably severe oropharyngeal and proximal limb weakness. PEO may arise sporadically or as a consequence of autosomal dominant, autosomal recessive, or maternal inheritance.

### 1. Sporadic PEO

Syndromes with severe ophthalmoplegia include Kearns-Sayre, PEO + proximal myopathy, and PEO.

Chronic PEO is caused by a single large mtDNA deletion and usually manifests at > 20 years of age. Symptoms include ophthalmoplegia, and heart block in some patients.

PEO with sensory ataxic neuropathy usually manifests between 10 to 31 years of age. Clinical features include sensory loss, gait disorder, distal motor weakness, absent tendon reflexes, external ophthalmoplegia, ptosis, dysarthria, facial weakness, myopathy, and ragged-red muscle fibers.

Mutations in *MTTQ* can result in PEO that presents at five years of age. Clinical symptoms include weakness, ptosis, dysphonia, dysphagia, ophthalmoplegia, reduced tendon reflexes, ragged red fibers, COX negative muscle fibers, and impairment in mitochondrial protein synthesis.

Mutations in *MTTA* can result in PEO that presents in the sixth decade of life. Clinical symptoms include ptosis, weakness, decreased eye movements, dysphagia, COX negative muscle fibers, mitochondrial proliferation, and partial defect of Complex I.

Mutations in *MTTL* can result in PEO that presents in the fifth decade of life. Clinical symptoms include ptosis, migraines, decreased eye movements, exercise intolerance, short stature, COX negative, ragged red muscle fibers, and partial defects of Complex I and IV.

Mutations in *MTTY* can result in PEO that presents in the fourth decade of life. Clinical symptoms include ptosis, exercise intolerance, ophthalmoplegia, myopathy, COX negative muscle fibers with increased SDH staining, and partial defect of Complex I and IV. Other MTTY syndromes include exercise intolerance with Complex III deficiency, and focal segmental glomerulosclerosis and dilated cardiomyopathy.

### 2. Maternally-inherited PEO

Maternal PEO is caused by mtDNA point mutations in *MTTL, MTTN, MTTQ, MTTA,* and *MTTK.*

### 3. Autosomal Dominant PEO

Autosomal dominant progressive external ophthalmoplegia (adPEO) with mitochondrial DNA (mtDNA) deletions-3 (PEOA3) is caused by heterozygous mutations in the nuclear-encoded twinkle gene (*C10ORF2*), which binds to the β-subunit of polymerase-y (POLG). Progressive external ophthalmoplegia is characterized by multiple mitochondrial DNA deletions in skeletal muscle. The most common clinical features include adult onset of weakness of the external eye muscles and exercise intolerance. Patients with C10ORF2-linked adPEO may have other clinical features including proximal muscle weakness, muscle pain, cramps, respiratory failure, ataxia, peripheral neuropathy, cardiomyopathy, cataracts, depression, ptosis, dysarthria, dysphagia, dysphonia, hearing loss, memory loss, Parkinsonism, avoidant personality traits, SDH+ COX negative muscle fibers, ragged-red fibers, ketoacidosis, cortical atrophy or white matter lesions, and endocrine abnormalities. Variant syndromes involving Twinkle mutations include Infantile Onset Spinocerebellar Ataxia (IOSCA), SANDO, MTDPS7, PEO + Dementia, PEO + Parkinson, and Perrault.

Autosomal dominant progressive external ophthalmoplegia (adPEO) with mitochondrial DNA (mtDNA) deletions-2 (PEOA2) is caused by heterozygous mutations in the nuclear-encoded ANT1 gene (SLC25A4), which usually manifests at 20 to 35 years of age. Clinical symptoms include ophthalmoplegia, ptosis, dysphagia, dysphonia, face, proximal, and respiratory weakness, cataracts, sensorineural hypoacusia, goiter, dementia, bipolar affective disorder, high serum lactic acid, and multiple mtDNA deletions.

PEOA2 can also be caused by heterozygous mutations in the nuclear-encoded twinkle gene (C10ORF2). The most common mutation is an Ala359Thr missense mutation, the homozygous version producing more severe effects than the heterozygous version. In addition, adPEO is characterized by multiple mitochondrial DNA deletions in skeletal muscle. A severe CNS phenotype with polyneuropathy is associated with a 39-bp deletion. In general, the mutations tend to cluster in regions of the protein involved in subunit interactions (amino acids 303-508). The twinkle protein is involved in mtDNA metabolism and could function as an adenine nucleotide-dependent DNA helicase. The function of the twinkle protein is believed to be critical for lifetime maintenance of mtDNA integrity. The most common clinical features of adPEO include adult onset of weakness of the external eye muscles and exercise intolerance. Patients with C10ORF2-linked adPEO may have other clinical features including proximal muscle weakness, ataxia, peripheral neuropathy, cardiomyopathy, cataracts, depression, and endocrine abnormalities.

Autosomal dominant progressive external ophthalmoplegia (adPEO) with mitochondrial DNA (mtDNA) deletions-1 (PEOA1) is caused by mutations in the nuclear-encoded DNA polymerase-gamma gene (*POLG*). Autosomal recessive PEO (PEOB) is also caused by mutations in the *POLG* gene. PEO1 manifests at 16 to 39 years of age. Clinical features include PEO, muscle weakness, exercise intolerance, sensory loss, absent tendon reflexes, poorly formed secondary sexual characteristics, early menopause, testicular atrophy, Parkinsonism, proximal weakness and wasting, dysphagia, dysphonia, facial diplegia, abnormal gait, depression, extrapyramidal syndrome, ragged red fibers, COX negative and SDH + fibers, and proximal myopathy. Other clinical syndromes associated with dominant *POLG* mutations include PEO+ demyelinating neuropathy, PEO + distal myopathy, sensory neuropathy, PEO and tremor, and PEO + hypogonadism. Clinical syndromes associated with recessive *POLG* mutations include Alpers-Huttenlocher Syndrome (AHS), Childhood myocerebrohepatopathy spectrum (MCHS), myoclonic epilepsy, myopathy, sensory ataxia (MEMSA), SANDO, MIRAS, MNGIE, and Parkinsonism.

PEO+ demyelinating neuropathy manifests at the second decade of life and is characterized by weakness, sensory loss, absent tendon reflexes, PEO with ptosis, dysphonia, dysphagia, nerve pathology, ragged red fibers, COX negative fibers, and reduced Complex I, III & IV activity.

PEO + hypogonadism is characterized by delayed sexual maturation, primary amenorrhea, early menopause, testicular atrophy, cataracts, cerebellar ataxia, tremor, Parkinsonism, depression, mental retardation, polyneuropathy, PEO, dysarthria, dysphonia, proximal weakness, rhabdomyolysis, hypoacusis, Pes cavus, ragged red fibers, and cytochrome c oxidase negative muscle fibers.

Distal myopathy, cachexia and PEO is caused by dominant or sporadic mutations in *POLG1* and usually manifests between the third and fourth decade of life. Clinical features include weakness, dysarthria, dysphagia, cachexia, ptosis, ophthalmoplegia, cataracts, and ragged red and COX negative muscle fibers.

Autosomal dominant progressive external ophthalmoplegia (adPEO) with mitochondrial DNA (mtDNA) deletions-4 (PEOA4) is caused by heterozygous mutations in the nuclear-encoded DNA polymerase gamma-2 gene (*POLG2*). Progressive external ophthalmoplegia-4 is an autosomal dominant form of mitochondrial disease that variably affects skeletal muscle, the nervous system, the liver, and the gastrointestinal tract. The age of onset ranges from infancy to adulthood. The phenotype ranges from relatively mild, with adult-onset skeletal muscle weakness and weakness of the external eye muscles, to severe, with a multisystem disorder characterized by delayed psychomotor development, lactic acidosis, constipation, and liver involvement. Clinical features include ptosis, external ophthalmoplegia, exercise intolerance, pain, weakness, seizures, hypotonia, impaired glucose tolerance, high lactate, cerebellar atrophy, cardiac conduction defect, and abnormal mitochondrial morphology.

Autosomal dominant progressive external ophthalmoplegia-6 (PEOA6) is caused by heterozygous mutations in the *DNA2* gene. PEOA6 is characterized by muscle weakness, mainly affecting the lower limbs, external ophthalmoplegia, exercise intolerance, and mitochondrial DNA (mtDNA) deletions on muscle biopsy. Clinical features include hypotonia, myalgia, exertional dyspnea, ptosis or ophthalmoplegia, lordosis, and muscular atrophy. Symptoms may appear in childhood or adulthood and show slow progression.

In some embodiments, dominant *POLG* mutations may lead to sensory neuropathy, tremor and PEO.

### 4. Autosomal Recessive PEO

PEO + myopathy and Parkinsonism is an adult onset autosomal recessive disorder. Clinical features include extrapyramidal signs (*e.g.,* akinesia, rigidity, rest tremor), ptosis, ophthalmoplegia, proximal and facial weakness, occasional distal leg weakness, hearing loss, SDH + and COX negative muscle fibers, reduced complex III activity, and multiple mtDNA deletions.

Autosomal recessive progressive external ophthalmoplegia (PEOB) is caused by homozygous or compound heterozygous mutations in the nuclear-encoded DNA polymerase-gamma gene (*POLG*). Recessive mutations in the *POLG* gene can also cause sensory ataxic neuropathy, dysarthria, and ophthalmoparesis (SANDO), which shows overlapping features. Autosomal recessive PEO is usually more severe than autosomal dominant PEO.

SANDO usually manifests between the ages of 16 to 38 years and is characterized by exercise intolerance, ptosis, and paresthesias. Clinical symptoms include sensory loss, ataxic gait, pseudoathetosis, small fiber modality loss, weakness, reduced tendon reflexes, ptosis, ophthalmoplegia, dysarthria, myoclonic epilepsy, depression, high CSF and serum lactate, degeneration of spinocerebellar and dorsal column tracts, thalamic lesions, cerebellar atrophy or white matter lesions, ragged red fibers, loss of myelinated and unmyelinated axons, and reduced activity of Complex I and IV.

Mitochondrial DNA Depletion Syndrome-11 (MTDPS11) can be caused by homozygous mutations in the *MGME1* gene. Mitochondrial DNA Depletion Syndrome-11 is an autosomal recessive mitochondrial disorder characterized by onset in childhood or adulthood of progressive external ophthalmoplegia (PEO), ptosis, muscle weakness and atrophy, exercise intolerance, dysphonia, dysphagia, and respiratory insufficiency due to muscle weakness. More variable features include spinal deformity, emaciation, and cardiac abnormalities. Skeletal muscle biopsies show deletion and depletion of mitochondrial DNA (mtDNA) with variable defects in respiratory chain enzyme activities. Additional features include scapular winging, mental retardation, memory deficits, nausea, flatulence, abdominal fullness, diarrhea, loss of appetite, SDH+ and COX negative fibers, Complex I and/or IV deficiencies, and cerebellar atrophy.

PEO with cardiomyopathy is caused by recessive mutations in *POLG* and usually manifests at childhood. Clinical features include PEO, cardiomyopathy, proximal weakness, multiple mtDNA deletions, and ragged-red fibers.

### Mutations in tRNA

In some embodiments, mitochondrial myopathies are a result of point mutations in tRNA genes selected from the group consisting of: tRNA^{Leu} (T3250C; A3302G; A12320G, A3288G); tRNA^{Pro} (G15990A; A16002G; G15995A); tRNA^{Phe} (T618C; G622A); tRNA^{Met} (T4409C; T5543C); tRNA^{Ser} (G7497A; A7480G); tRNA^{Asp} (A7526G); tRNA^{Gln} (4366insA); tRNA^{Ala}; tRNA^{Glu} (T14709C); tRNA^{Trp} (G5521A); and tRNA^{Tyr}.

### Mutations in mtDNA

In some embodiments, mitochondrial myopathies are a result of one or more of the following point mutations in mtDNA: G15243A, T9185C, G3421A, G10197A, T12148C, and G6570A.

### Therapeutic Methods

The following discussion is presented by way of example only, and is not intended to be limiting.

One aspect of the present technology includes methods of treating mitochondrial myopathy in a subject diagnosed as having, suspected as having, or at risk of having mitochondrial myopathy. In therapeutic applications, compositions or medicaments comprising at least one aromatic-cationic peptide, such as D-Arg-2',6'-Dmt-Lys-Phe-NH₂, or a pharmaceutically acceptable salt thereof, such as acetate, tartrate, trifluoroacetate, or hydrochloride salt, are administered to a subject suspected of, or already suffering from such a disease (such as, *e.g.,* subjects suspected of, or already suffering from a mitochondrial mitomypathy described herein such as Kearns-Sayre syndrome (KSS); Leber's hereditary optic neuropathy (LHON); Leigh syndrome (LS); MEGDEL Syndrome; mitochondrial DNA depletion syndrome (MDS); mitochondrial myopathy, encephalomyopathy, lactic acidosis, and stroke-like episodes (MELAS); myoclonus epilepsy with ragged-red fibers (MERRF); mitochondrial neurogastrointestinal encephalomyopathy (MNGIE); neuropathy, ataxia and retinitis pigmentosa (NARP); *OPA1* mutations; Pearson syndrome; and progressive external ophthalmoplegia (PEO)) in an amount sufficient to cure, or at least partially arrest, the symptoms of the disease, including its complications and intermediate pathological phenotypes in development of the disease. In some embodiments, the mitochondrial myopathies are not a result of *POLG* or *SURF1* mutations.

Subjects suffering from mitochondrial myopathy can be identified by any or a combination of diagnostic or prognostic assays known in the art. For example, typical symptoms of mitochondrial myopathy include one or more symptoms selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy.

In some embodiments, mitochondrial myopathy subjects treated with the aromatic-cationic peptide will show amelioration or elimination of one or more symptoms selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy.

Subjects with mitochondrial myopathy may display abnormal levels of one or more energy biomarkers compared to a normal control subject. In some embodiments, the energy biomarker is selected from the group consisting of lactic acid (lactate) levels; pyruvic acid (pyruvate) levels; lactate/pyruvate ratios; phosphocreatine levels; NADH (NADH+H⁺) or NADPH (NADPH+H⁺) levels; NAD or NADP levels; ATP levels; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels; beta-hydroxy butyrate levels; acetoacetate/ beta-hydroxy butyrate ratio; 8-hydroxy-2'-deoxyguanosine (8-OHdG) levels; levels of reactive oxygen species; oxygen consumption (VO₂), carbon dioxide output (VCO₂), and respiratory quotient (VCO₂/VO₂).

In some embodiments, subjects treated with the aromatic-cationic peptide will show normalization of one or more of the following energy biomarkers: lactic acid (lactate) levels; pyruvic acid (pyruvate) levels; lactate/pyruvate ratios; phosphocreatine levels; NADH (NADH+H⁺) or NADPH (NADPH+H⁺) levels; NAD or NADP levels; ATP levels; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels; beta-hydroxy butyrate levels; acetoacetate/ beta-hydroxy butyrate ratio; 8-hydroxy-2'-deoxyguanosine (8-OHdG) levels; levels of reactive oxygen species; oxygen consumption (VO₂), carbon dioxide output (VCO₂), and respiratory quotient (VCO₂/VO₂).

Exercise intolerance is often accompanied by myoglobinuria, due to breakdown of muscle tissue and subsequent excretion of muscle myoglobin in the urine. Various measures of exercise intolerance can be used, such as time spent walking or running on a treadmill before exhaustion, time spent on an exercise bicycle (stationary bicycle) before exhaustion. In certain embodiments, mitochondrial myopathy subject treated with the aromatic-cationic peptide of the present technology will show about a 5% or greater improvement in exercise tolerance (e.g., about a 5% or greater increase in time to exhaustion), about a 10% or greater improvement in exercise tolerance, about a 20% or greater improvement in exercise tolerance, about a 30% or greater improvement in exercise tolerance, about a 40% or greater improvement in exercise tolerance, about a 50% or greater improvement in exercise tolerance, about a 75% or greater improvement in exercise tolerance, or about a 100% or greater improvement in exercise tolerance compared to untreated mitochondrial myopathy subj ects.

### Prophylactic Methods

In one aspect, the present technology provides a method for preventing or delaying the onset of mitochondrial myopathy or symptoms of mitochondrial myopathy in a subject at risk of having mitochondrial myopathy. In some embodiments, the subject may be at risk of having a mitochondrial myopathy disclosed herein, such as Kearns-Sayre syndrome (KSS); Leber's hereditary optic neuropathy (LHON); Leigh syndrome (LS); MEGDEL Syndrome; mitochondrial DNA depletion syndrome (MDS); mitochondrial myopathy, encephalomyopathy, lactic acidosis, and stroke-like episodes (MELAS); myoclonus epilepsy with ragged-red fibers (MERRF); mitochondrial neurogastrointestinal encephalomyopathy (MNGIE); neuropathy, ataxia and retinitis pigmentosa (NARP); *OPA1* mutations; Pearson syndrome; and progressive external ophthalmoplegia (PEO). In some embodiments, the mitochondrial myopathies are not a result of *POLG* or *SURF1* mutations.

Subjects at risk for mitochondrial myopathy can be identified by, *e.g.,* any or a combination of diagnostic or prognostic assays known in the art. In prophylactic applications, pharmaceutical compositions or medicaments of aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, are administered to a subject susceptible to, or otherwise at risk of a disease or condition such as *e.g.,* mitochondrial myopathy, in an amount sufficient to eliminate or reduce the risk, or delay the onset of the disease, including biochemical, histologic and/or behavioral symptoms of the disease, its complications and intermediate pathological phenotypes presenting during development of the disease. Administration of a prophylactic aromatic-cationic peptide can occur prior to the manifestation of symptoms characteristic of the disease or disorder, such that the disease or disorder is prevented or, alternatively, delayed in its progression.

For therapeutic and/or prophylactic applications, a composition comprising an aromatic-cationic peptide, such as D-Arg-2',6'-Dmt-Lys-Phe-NH₂, or a pharmaceutically acceptable salt thereof, such as acetate, tartrate, trifluoroacetate, or hydrochloride salt, is administered to the subject. In some embodiments, the peptide composition is administered one, two, three, four, or five times per day. In some embodiments, the peptide composition is administered more than five times per day. Additionally or alternatively, in some embodiments, the peptide composition is administered every day, every other day, every third day, every fourth day, every fifth day, or every sixth day. In some embodiments, the peptide composition is administered weekly, bi-weekly, tri-weekly, or monthly. In some embodiments, the peptide composition is administered for a period of one, two, three, four, or five weeks. In some embodiments, the peptide is administered for six weeks or more. In some embodiments, the peptide is administered for twelve weeks or more. In some embodiments, the peptide is administered for a period of less than one year. In some embodiments, the peptide is administered for a period of more than one year. In some embodiments of the methods of the present technology, the aromatic-cationic peptide is administered daily for 1 week or more. In some embodiments of the methods of the present technology, the aromatic-cationic peptide is administered daily for 2 weeks or more. In some embodiments of the methods of the present technology, the aromatic-cationic peptide is administered daily for 3 weeks or more. In some embodiments of the methods of the present technology, the aromatic-cationic peptide is administered daily for 4 weeks or more. In some embodiments of the methods of the present technology, the aromatic-cationic peptide is administered daily for 6 weeks or more. In some embodiments of the methods of the present technology, the aromatic-cationic peptide is administered daily for 12 weeks or more.

In some embodiments, treatment with the aromatic-cationic peptide will prevent or delay the onset of one or more symptoms selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy.

### Determination of the Biological Effect of the Aromatic-Cationic Peptide-Based Therapeutic

In various embodiments, suitable *in vitro* or *in vivo* assays are performed to determine the effect of a specific aromatic-cationic peptide-based therapeutic and whether its administration is indicated for treatment. In various embodiments, *in vitro* assays can be performed with representative animal models, to determine if a given aromatic-cationic peptide-based therapeutic exerts the desired effect on reducing or eliminating signs and/or symptoms of mitochondrial myopathy. Compounds for use in therapy can be tested in suitable animal model systems including, but not limited to rats, mice, chicken, cows, monkeys, rabbits, and the like, prior to testing in human subjects. Similarly, for *in vivo* testing, any of the animal model system known in the art can be used prior to administration to human subjects. In some embodiments, *in vitro* or *in vivo* testing is directed to the biological function of D-Arg-2',6'-Dmt-Lys-Phe-NH₂, or a pharmaceutically acceptable salt thereof, such as acetate, tartrate, trifluoroacetate, or hydrochloride salt.

Animal models of mitochondrial myopathy may be generated using techniques known in the art, including, for example by administering the NADH-CoQ reductase inhibitor, diphenyleneiodonium to rats (*see* Cooper et al., J. Neurol. Sci. 83(2-3):335-347 (1988); Cosgrove et al., Genes Dev. 10(23):2981-92 (1996)), or generating knockout mice deficient in the muscle isoform of the adenine nucleotide translocator (Ant1) (*see* Graham, et al., Nat. Genet. 16(3):226-234 (1997)). Such models may be used to demonstrate the biological effect of aromatic-cationic peptides of the present technology, such as D-Arg-2',6'-Dmt-Lys-Phe-NH₂, in the prevention and treatment of conditions arising from disruption of a particular gene, and for determining what comprises a therapeutically effective amount of peptide in a given context.

### Modes of Administration and Effective Dosages

Any method known to those in the art for contacting a cell, organ or tissue with an aromatic-cationic peptide of the present technology, such as D-Arg-2',6'-Dmt-Lys-Phe-NH₂, or a pharmaceutically acceptable salt thereof, such as acetate, tartrate, trifluoroacetate, or hydrochloride salt, may be employed. Suitable methods include *in vitro, ex vivo, or in vivo* methods.

*In vitro* methods typically include cultured samples. For example, a cell can be placed in a reservoir (*e.g.,* tissue culture plate), and incubated with a compound under appropriate conditions suitable for obtaining the desired result. Suitable incubation conditions can be readily determined by those skilled in the art.

*Ex vivo* methods typically include cells, organs or tissues removed from a mammal, such as a human. The cells, organs or tissues can, for example, be incubated with the compound under appropriate conditions. The contacted cells, organs or tissues are typically returned to the donor, placed in a recipient, or stored for future use. Thus, the compound is generally in a pharmaceutically acceptable carrier.

*In vivo* methods typically include the administration of an aromatic-cationic peptide, such as those described above, to a mammal, suitably a human. When used *in vivo* for therapy, the aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, are administered to the subject in effective amounts (*i.e*., amounts that have desired therapeutic effect). The dose and dosage regimen will depend upon the degree of the infection in the subject, the characteristics of the particular aromatic-cationic peptide used, *e.g.,* its therapeutic index, the subject, and the subject's history.

The effective amount may be determined during pre-clinical trials and clinical trials by methods familiar to physicians and clinicians. An effective amount of a peptide useful in the methods may be administered to a mammal in need thereof by any of a number of well-known methods for administering pharmaceutical compounds. The peptide may be administered systemically or locally.

The peptide may be formulated as a pharmaceutically acceptable salt. The term "pharmaceutically acceptable salt" means a salt prepared from a base or an acid which is acceptable for administration to a patient, such as a mammal (*e.g.,* salts having acceptable mammalian safety for a given dosage regime). However, it is understood that the salts are not required to be pharmaceutically acceptable salts, such as salts of intermediate compounds that are not intended for administration to a patient. Pharmaceutically acceptable salts can be derived from pharmaceutically acceptable inorganic or organic bases and from pharmaceutically acceptable inorganic or organic acids. In addition, when a peptide contains both a basic moiety, such as an amine, pyridine or imidazole, and an acidic moiety such as a carboxylic acid or tetrazole, zwitterions may be formed and are included within the term "salt" as used herein. Salts derived from pharmaceutically acceptable inorganic bases include ammonium, calcium, copper, ferric, ferrous, lithium, magnesium, manganic, manganous, potassium, sodium, and zinc salts, and the like. Salts derived from pharmaceutically acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperadine, polyamine resins, procaine, purines, theobromine, triethylamine, trimethylamine, tripropylamine, tromethamine and the like. Salts derived from pharmaceutically acceptable inorganic acids include salts of boric, carbonic, hydrohalic (hydrobromic, hydrochloric, hydrofluoric or hydroiodic), nitric, phosphoric, sulfamic and sulfuric acids. Salts derived from pharmaceutically acceptable organic acids include salts of aliphatic hydroxyl acids (*e.g.,* citric, gluconic, glycolic, lactic, lactobionic, malic, and tartaric acids), aliphatic monocarboxylic acids (*e.g.,* acetic, butyric, formic, propionic and trifluoroacetic acids), amino acids (*e.g.,* aspartic and glutamic acids), aromatic carboxylic acids (*e.g.,* benzoic, p-chlorobenzoic, diphenylacetic, gentisic, hippuric, and triphenylacetic acids), aromatic hydroxyl acids (*e.g.,* o-hydroxybenzoic, p-hydroxybenzoic, 1-hydroxynaphthalene-2-carboxylic and 3-hydroxynaphthalene-2-carboxylic acids), ascorbic, dicarboxylic acids (*e.g.,* fumaric, maleic, oxalic and succinic acids), glucuronic, mandelic, mucic, nicotinic, orotic, pamoic, pantothenic, sulfonic acids (*e.g.,* benzenesulfonic, camphosulfonic, edisylic, ethanesulfonic, isethionic, methanesulfonic, naphthalenesulfonic, naphthalene-1,5-disulfonic, naphthalene-2,6-disulfonic and p-toluenesulfonic acids), xinafoic acid, tosylate and the like. In some embodiments, the salt is an acetate, tartrate, trifluoroacetate, or hydrochloride salt.

The aromatic-cationic peptides described herein, such as D-Arg-2',6'-Dmt-Lys-Phe-NH₂, or a pharmaceutically acceptable salt thereof, such as acetate, tartrate, trifluoroacetate, or hydrochloride salt, can be incorporated into pharmaceutical compositions for administration, singly or in combination, to a subject for the treatment or prevention of a disorder described herein. Such compositions typically include the active agent and a pharmaceutically acceptable carrier. As used herein the term "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

Pharmaceutical compositions are typically formulated to be compatible with the intended route of administration. Routes of administration include, for example, parenteral (*e.g.,* intravenous, intradermal, intraperitoneal or subcutaneous), oral, nasal/respiratory (*e.g.,* inhalation), transdermal (topical), ocular and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates, and agents for the adjustment of tonicity, such as sodium chloride or dextrose; buffers, such as acetates, citrates or phosphates; and compounds for adjusting pH such as acids or bases, *e.g.,* hydrochloric acid or sodium hydroxide. Examples of agents that can be used to decrease pH include, but are not limited to, ZnCl₂, AlCl₃, phosphate esters (*e.g.,* fructose 1,6 diphosphate, glucose 1,6 diphosphate, phosphoglyceric acid, and diphosphoglyceric acid), Carbopol^{®}, polycarbophil, dicarboxylic or tricarboxylic acids, acid salts of amino acids (*e.g.,* amino acid hydrochlorides) or derivatives thereof. A dicarboxylic or tricarboxylic acid may include one or more of propionicadipic, benzoic, glyoxylic, isocitric, acetic, ascorbic, glycocolic, isovaleric, benzenesulfonic, p-toluenenesulfonic, acetylsalicylic, fumaric, citric, pyruvic, succinic, glyceric, lactic, glucuronic, glutaric, tartaric, valeric, maleic, oxaloacetic, phthalic, sorbic, oxalosuccinic, edetic, methansulfonic, boric, and saccharinic acid. Acid salts of amino acids include acid salts of hypotaurine, isoleucine, leucine, acetylglutamic acid, alanine, carnitine, carnosine, lysine, phenylalanine, tryptophan, methylhistidine, asparagine, aspartic acid, creatine, glutamic acid, glycine, arginine, betaine, hydroxyprolinenorleucine, ornithine, proline, sarcosine, citrulline, histidine, hydroxylysine, serine, taurine, threonine, tyrosine and valine.

Alginic acid and other naturally occurring or synthetic polysaccharide acids, such as, *e.g.,* substituted carboyxmethyl celluloses, xanthan gum, polymethacrylic acid and substituted derivatives, cellulose glycolic acid, pectins, and protease inhibitors (*e.g.,* trypsin inhibitors (TI)) may also be used to adjust (*e.g.,* lower) pH. TIs include, but are not limited to, soy protein *(e.g.,* SBTI, Kunitz inhibitor, or Glycine Max^{®}) of 20.1 kDa, bovine pancreas of 6.5 kDa, aprotonin, chicken or turkey ova-mucoid TI of 28 kDA, partially hydrolysed gelatin fractions, epsilon-aminocaproic acid, and di-Sodium EDTA.

The preparation can be enclosed in ampoules, disposable syringes or multiple-dose vials made of glass or plastic. For convenience of the patient or treating physician, the dosing formulation can be provided in a kit containing all necessary equipment (*e.g.,* vials of drug, vials of diluent, syringes and needles) for a course of treatment (*e.g.,* 7 days of treatment).

Pharmaceutical compositions suitable for injectable use can include sterile aqueous solutions (where water soluble) or dispersions and sterile powders for the extemporaneous preparation of sterile injectable solutions or dispersion. For intravenous administration, suitable carriers include physiological saline, bacteriostatic water, CREMOPHOR EL^{™} (BASF, Parsippany, N.J.) or phosphate buffered saline (PBS). In all cases, a composition for parenteral administration must be sterile and should be fluid to the extent that easy syringability exists. It should be stable under the conditions of manufacture and storage and must be preserved against the contaminating action of microorganisms such as bacteria and fungi.

In one embodiment, the aromatic-cationic peptides of the present technology are administered intravenously. For example, an aromatic-cationic peptide of the present technology may be administered *via* rapid intravenous bolus injection. In some embodiments, the aromatic-cationic peptide of the present technology is administered as a constant-rate intravenous infusion.

The aromatic-cationic peptides of the present technology may also be administered orally, topically, intranasally, intramuscularly, subcutaneously, or transdermally. In one embodiment, transdermal administration is by iontophoresis, in which the charged composition is delivered across the skin by an electric current.

Other routes of administration include intracerebroventricularly or intrathecally. Intracerebroventricularly refers to administration into the ventricular system of the brain. Intrathecally refers to administration into the space under the arachnoid membrane of the spinal cord. Thus, in some embodiments, intracerebroventricular or intrathecal administration is used for those diseases and conditions which affect the organs or tissues of the central nervous system.

The aromatic-cationic peptide of the present technology may also be administered to mammals by sustained release, as is known in the art. Sustained release administration is a method of drug delivery to achieve a certain level of the drug over a particular period of time. The level is typically measured by serum or plasma concentration. A description of methods for delivering a compound by controlled release can be found in international PCT Application No. WO 02/083106, which is incorporated herein by reference in its entirety.

Any formulation known in the art of pharmacy is suitable for administration of the aromatic-cationic peptide of the present technology. For oral administration, liquid or solid formulations may be used. Examples of formulations include tablets, gelatin capsules, pills, troches, elixirs, suspensions, syrups, wafers, chewing gum and the like. The aromatic-cationic peptides of the present technology can be mixed with a suitable pharmaceutical carrier (vehicle) or excipient as understood by practitioners in the art. Examples of carriers and excipients include starch, milk, sugar, certain types of clay, gelatin, lactic acid, stearic acid or salts thereof, including magnesium or calcium stearate, talc, vegetable fats or oils, gums and glycols.

For systemic, intracerebroventricular, intrathecal, topical, intranasal, subcutaneous, or transdermal administration, formulations of the aromatic-cationic peptides of the present technology may utilize conventional diluents, carriers, or excipients *etc.,* such as those known in the art to deliver the aromatic-cationic peptides of the present technology. For example, the formulations may comprise one or more of the following: a stabilizer, a surfactant (*e.g.,* a nonionic surfactant), and optionally a salt and/or a buffering agent. The aromatic-cationic peptides of the present technology may be delivered in the form of an aqueous solution, or in a lyophilized form.

The stabilizer may comprise, for example, an amino acid, such as for instance, glycine; an oligosaccharide, such as, sucrose, tetralose, lactose; or a dextran. Alternatively, the stabilizer may comprise a sugar alcohol, such as, mannitol. In some embodiments, the stabilizer or combination of stabilizers constitutes from about 0.1% to about 10% weight for weight of the formulated composition.

In some embodiments, the surfactant is a nonionic surfactant (such as a polysorbate), an anionic surfactant (such as dioctyl sodium sulfosuccinate), a cationic surfactant (such as cetylpyridinium chloride), or a combination thereof. Examples of suitable non-ionic surfactants include polyoxyethylene sorbitan esters (*e.g*,. Tween-20 and Tween-80), p-t-octyl phenol polyoxyethylenes (*e.g*., Triton X-45, Triton X-100, Triton X-114, and Triton X-305), a polyethylene glycol or a polyoxyethylene polyoxypropylene glycol, such as Pluronic F-68, nonylphenoxypoloxyethylenes (*e.g.,* Igepal CO series), and polyoxyethylene ethers (e.g. Brij 36T, Brij 52, Brij 56, Brij 76, Brij 96, Texaphor A6, Texaphor A14, and Texaphor A60), at from about 0.001% (w/v) to about 10% (w/v).

The salt or buffering agent may be any salt or buffering agent, such as for example, sodium chloride, or sodium/potassium phosphate, respectively. In some embodiments, the buffering agent maintains the pH of the pharmaceutical composition in the range of about 5.5 to about 7.5. The salt and/or buffering agent is also useful to maintain the osmolality at a level suitable for administration to a human or an animal. In some embodiments, the salt or buffering agent is present at a roughly isotonic concentration of about 150 mM to about 300 mM.

Formulations of aromatic-cationic peptides of the present technology may additionally contain one or more conventional additives. Examples of such additives include a solubilizer such as, for example, glycerol; an antioxidant such as for example, benzalkonium chloride (a mixture of quaternary ammonium compounds, known as "quats"), benzyl alcohol, chloretone or chlorobutanol; an anesthetic agent such as for example a morphine derivative; and an isotonic agent *etc.,* such as described herein. As a further precaution against oxidation or other spoilage, the pharmaceutical compositions may be stored under nitrogen gas in vials sealed with impermeable stoppers.

In some embodiments, compositions including aromatic-cationic peptides of the present technology may include one or more of salicylates such as, *e.g.,* sodium salicylate, 3-methoxysalicylate, 5-methoxysalicylate and homovanilate; cholesterol derivatives such as bile acids, *e.g.,* taurocholic, tauorodeoxycholic, deoxycholic, cholic, glycholic, lithocholate, chenodeoxycholic, ursodeoxycholic, ursocholic, dehydrocholic, and fusidic acid; cetyl pyridinium chloride; acylcarnitines, acylcholines and acyl amino acids such as lauroylcarnitine, myristoylcarnitine, palmitoylcarnitine, lauroylcholine, myristoylcholine, palmitoylcholine, hexadecyllysine, N-acylphenylalanine, N-acylglycine; phospholipids such as lysolecithin, lysophosphatidylethanolamine, diheptanoylphosphatidylcholine and dioctylphosphatidylcholine; ethylene-diaminetetraacetic acid; alkylsaccharides such as lauryl maltoside, lauroyl sucrose, myristoyl sucrose, and palmitoyl sucrose; fatty acid derivatives of PEG such as Labrasol, Labrafac; and mixtures of mono-, di- and triglycerides containing medium-chain-length fatty acids (caprylic, capric and lauric acids).

The aromatic-cationic peptide compositions can include a carrier, which can be a solvent or dispersion medium containing, for example, water, ethanol, polyol (*e.g.,* glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thiomerasol, and the like. Glutathione and other antioxidants can be included to prevent oxidation. In many cases, it will be advantageous to include isotonic agents, for example, sugars, polyalcohols such as mannitol, sorbitol, or sodium chloride in the composition. Prolonged absorption of the injectable compositions can be brought about by including in the composition an agent that delays absorption, for example, aluminum monostearate or gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, typical methods of preparation include vacuum drying and freeze drying, which can yield a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds can be delivered in the form of an aerosol spray from a pressurized container or dispenser, which contains a suitable propellant, *e.g.,* a gas such as carbon dioxide, or a nebulizer. Such methods include those described in U.S. Pat. No. 6,468,798.

Systemic administration of a therapeutic compound as described herein can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays. For transdermal administration, the active compounds are formulated into ointments, salves, gels, or creams as generally known in the art. In one embodiment, transdermal administration may be performed by iontophoresis.

A therapeutic protein or peptide can be formulated in a carrier system. The carrier can be a colloidal system. The colloidal system can be a liposome, a phospholipid bilayer vehicle. In one embodiment, the therapeutic peptide is encapsulated in a liposome while maintaining peptide integrity. One skilled in the art would appreciate that there are a variety of methods to prepare liposomes. *(See* Lichtenberg, et al., Methods Biochem. Anal., 33:337-462 (1988); Anselem, et al., Liposome Technology, CRC Press (1993)). Liposomal formulations can delay clearance and increase cellular uptake (*See* Reddy, Ann. Pharmacother., 34(7-8):915-923 (2000)). An active agent can also be loaded into a particle prepared from pharmaceutically acceptable ingredients including, but not limited to, soluble, insoluble, permeable, impermeable, biodegradable or gastroretentive polymers or liposomes. Such particles include, but are not limited to, nanoparticles, biodegradable nanoparticles, microparticles, biodegradable microparticles, nanospheres, biodegradable nanospheres, microspheres, biodegradable microspheres, capsules, emulsions, liposomes, micelles and viral vector systems.

The carrier can also be a polymer, *e.g.,* a biodegradable, biocompatible polymer matrix. In one embodiment, the therapeutic peptide can be embedded in the polymer matrix, while maintaining protein integrity. The polymer may be natural, such as polypeptides, proteins or polysaccharides, or synthetic, such as poly α-hydroxy acids. Examples include carriers made of, *e.g.,* collagen, fibronectin, elastin, cellulose acetate, cellulose nitrate, polysaccharide, fibrin, gelatin, and combinations thereof. In one embodiment, the polymer is poly-lactic acid (PLA) or copoly lactic/glycolic acid (PGLA). The polymeric matrices can be prepared and isolated in a variety of forms and sizes, including microspheres and nanospheres. Polymer formulations can lead to prolonged duration of therapeutic effect. *(See* Reddy, Ann. Pharmacother., 34(7-8):915-923 (2000)). A polymer formulation for human growth hormone (hGH) has been used in clinical trials. *(See* Kozarich and Rich, Chemical Biology, 2:548-552 (1998)).

Examples of polymer microsphere sustained release formulations are described in PCT publication WO 99/15154 (Tracy, et al.), U.S. Pat. Nos. 5,674,534 and 5,716,644 (both to Zale, et al.), PCT publication WO 96/40073 (Zale, et al.), and PCT publication WO 00/38651 (Shah, et al.). U.S. Pat. Nos. 5,674,534 and 5,716,644 and PCT publication WO 96/40073 describe a polymeric matrix containing particles of erythropoietin that are stabilized against aggregation with a salt.

In some embodiments, the therapeutic compounds are prepared with carriers that will protect the therapeutic compounds against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using known techniques. The materials can also be obtained commercially, e.g., from Alza Corporation and Nova Pharmaceuticals, Inc. Liposomal suspensions (including liposomes targeted to specific cells with monoclonal antibodies to cell-specific antigens) can also be used as pharmaceutically acceptable carriers. These can be prepared according to methods known to those skilled in the art, for example, as described in U.S. Pat. No. 4,522,811.

The therapeutic compounds can also be formulated to enhance intracellular delivery. For example, liposomal delivery systems are known in the art, see, *e.g.,* Chonn and Cullis, "Recent Advances in Liposome Drug Delivery Systems," Current Opinion in Biotechnology 6:698-708 (1995); Weiner, "Liposomes for Protein Delivery: Selecting Manufacture and Development Processes," Immunomethods, 4(3):201-9 (1994); and Gregoriadis, "Engineering Liposomes for Drug Delivery: Progress and Problems," Trends Biotechnol., 13(12):527-37 (1995). Mizguchi, et al., Cancer Lett., 100:63-69 (1996), describes the use of fusogenic liposomes to deliver a protein to cells both *in vivo* and *in vitro.*

Dosage, toxicity and therapeutic efficacy of any therapeutic agent can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit high therapeutic indices are advantageous. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds may be within a range of circulating concentrations that include the ED50 with little or no toxicity. The dosage may vary within this range depending upon the dosage form employed and the route of administration utilized. For any compound used in the methods, the therapeutically effective dose can be estimated initially from cell culture assays. A dose can be formulated in animal models to achieve a circulating plasma concentration range that includes the IC50 *(i.e.,* the concentration of the test compound which achieves a half-maximal inhibition of symptoms) as determined in cell culture. Such information can be used to determine useful doses in humans accurately. Levels in plasma may be measured, for example, by high performance liquid chromatography.

Typically, an effective amount of the aromatic-cationic peptides, sufficient for achieving a therapeutic or prophylactic effect, range from about 0.000001 mg per kilogram body weight per day to about 10,000 mg per kilogram body weight per day. Suitably, the dosage ranges are from about 0.0001 mg per kilogram body weight per day to about 100 mg per kilogram body weight per day. For example dosages can be 1 mg/kg body weight or 10 mg/kg body weight every day, every two days or every three days or within the range of 1-10 mg/kg every week, every two weeks or every three weeks. In one embodiment, a single dosage of peptide ranges from 0.001-10,000 micrograms per kg body weight. In one embodiment, aromatic-cationic peptide concentrations in a carrier range from 0.2 to 2000 micrograms per delivered milliliter. An exemplary treatment regime entails administration once per day or once a week. In therapeutic applications, a relatively high dosage at relatively short intervals is sometimes required until progression of the disease is reduced or terminated, or until the subject shows partial or complete amelioration of symptoms of disease. Thereafter, the patient can be administered a prophylactic regime.

In some embodiments, a therapeutically effective amount of an aromatic-cationic peptide may be defined as a concentration of peptide at the target tissue of 10⁻¹² to 10⁻⁶ molar, e.g., approximately 10⁻⁷ molar. This concentration may be delivered by systemic doses of 0.001 to 100 mg/kg or equivalent dose by body surface area. The schedule of doses would be optimized to maintain the therapeutic concentration at the target tissue, such as by single daily or weekly administration, but also including continuous administration (e.g., parenteral infusion or transdermal application).

The skilled artisan will appreciate that certain factors may influence the dosage and timing required to effectively treat a subject, including but not limited to, the severity of the disease or disorder, previous treatments, the general health and/or age of the subject, and other diseases present. Moreover, treatment of a subject with a therapeutically effective amount of the therapeutic compositions described herein can include a single treatment or a series of treatments.

The mammal treated in accordance with the present methods can be any mammal, including, for example, farm animals, such as sheep, pigs, cows, and horses; pet animals, such as dogs and cats; laboratory animals, such as rats, mice and rabbits. In some embodiments, the mammal is a human.

### Combination Therapy with Aromatic-Cationic Peptides

In some embodiments, the aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, may be combined with one or more additional therapies for the prevention or treatment of mitochondrial myopathy. Additional therapeutic agents include, but are not limited to, creatine, L-carnitine, coenzyme Q₁₀, L-arginine, biotin, cytochrome c, corticosteroids, idebenone, sodium dichloroacetate, thiamine, thiocitic acid, riboflavin, α-tocopherol, succinate, ascorbate, menadione, naphthoquinone, and nicotinamide.

In one embodiment, an additional therapeutic agent is administered to a subject in combination with an aromatic cationic peptide, such that a synergistic therapeutic effect is produced. For example, administration of the peptide with one or more additional therapeutic agents for the prevention or treatment of mitochondrial myopathy will have greater than additive effects in the prevention or treatment of the disease. Therefore, lower doses of one or more of any individual therapeutic agent may be used in treating or preventing mitochondrial myopathy, resulting in increased therapeutic efficacy and decreased side-effects. In some embodiments, the peptide is administered in combination with one or more creatine, L-carnitine, coenzyme Q₁₀, L-arginine, biotin, cytochrome c, corticosteroids, idebenone, sodium dichloroacetate, thiamine, thiocitic acid, riboflavin, α-tocopherol, succinate, ascorbate, menadione, naphthoquinone, and nicotinamide, such that a synergistic effect in the prevention or treatment of mitochondrial myopathy results.

In any case, the multiple therapeutic agents may be administered in any order or even simultaneously. If simultaneously, the multiple therapeutic agents may be provided in a single, unified form, or in multiple forms (by way of example only, either as a single pill or as two separate pills). One of the therapeutic agents may be given in multiple doses, or both may be given as multiple doses. If not simultaneous, the timing between the multiple doses may vary from more than zero weeks to less than four weeks. In addition, the combination methods, compositions and formulations are not to be limited to the use of only two agents.

### EXAMPLES

The present technology is further illustrated by the following examples, which should not be construed as limiting in any way. For each of the examples below, any aromatic-cationic peptide described herein could be used. By way of example, but not by limitation, the aromatic-cationic peptide used in the example below could be 2'6'-Dmt-D-Arg-Phe-Lys-NH₂, Phe-D-Arg-Phe-Lys-NH₂, or D-Arg-2'6'-Dmt-Lys-Phe-NH₂ (MTP-131) or one or more of any of the peptides shown in Tables A-E.

### Example 1 - Use of Aromatic-Cationic Peptides in the Treatment of Mitochondrial Myopathy in Humans

This example demonstrates the use of aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, in the treatment of mitochondrial myopathy.

### Methods

Subjects suspected of having or diagnosed as having mitochondrial myopathy receive daily administrations of 1 mg/kg body weight of aromatic-cationic peptide, such as D-Arg-2',6'-Dmt-Lys-Phe-NH₂, or a pharmaceutically acceptable salt thereof, such as acetate, tartrate, trifluoroacetate, or hydrochloride salt, alone or in combination with one or more additional therapeutic agents for the treatment or prevention of mitochondrial myopathy. Peptides and/or additional therapeutic agents are administered orally, topically, systemically, intravenously, subcutaneously, intraperitoneally, or intramuscularly according to methods known in the art. Subjects will be evaluated weekly for the presence and/or severity of signs and symptoms associated with mitochondrial myopathy, wherein the symptoms are selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, muscle atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy. Treatments are maintained until such a time as one or more signs or symptoms of mitochondrial myopathy are ameliorated or eliminated.

### Results

It is predicted that subjects suspected of having or diagnosed as having mitochondrial myopathy and receiving therapeutically effective amounts of aromatic-cationic peptide, such as D-Arg-2',6'-Dmt-Lys-Phe-NH₂, or a pharmaceutically acceptable salt thereof, such as acetate, tartrate, trifluoroacetate, or hydrochloride salt will display reduced severity or elimination of symptoms associated with mitochondrial myopathy. It is also expected that mitochondrial myopathy subjects treated with the aromatic-cationic peptide will show normalization of one or more of one or more energy biomarkers selected from the group consisting of: lactic acid (lactate) levels; pyruvic acid (pyruvate) levels; lactate/pyruvate ratios; phosphocreatine levels; NADH (NADH+H⁺) or NADPH (NADPH+H⁺) levels; NAD or NADP levels; ATP levels; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels; beta-hydroxy butyrate levels; acetoacetate/ beta-hydroxy butyrate ratio; 8-hydroxy-2'-deoxyguanosine (8-OHdG) levels; levels of reactive oxygen species; oxygen consumption (VO2), carbon dioxide output (VCO2), and respiratory quotient (VCO2/VO2) levels by at least 10% compared to the untreated mitochondrial myopathy controls. It is further expected that administration of D-Arg-2',6'-Dmt-Lys-Phe-NH₂ in combination with one or more additional therapeutic agents will have synergistic effects in this regard compared to that observed in subjects treated with the aromatic-cationic peptides or the additional therapeutic agents alone.

These results will show that aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt are useful in the treatment of mitochondrial myopathies. These results will show that aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt are useful in ameliorating one or more of symptoms selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, muscle atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy. Accordingly, the peptides are useful in methods comprising administering aromatic-cationic peptides to a subject in need thereof for the treatment of mitochondrial myopathy.

### Example 2 - Use of Aromatic-Cationic Peptides in the Prevention of Mitochondrial Myopathy in Humans

This example demonstrates the use of aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, in the prevention of mitochondrial myopathy.

### Methods

Subjects at risk of having mitochondrial myopathy receive daily administrations of 1 mg/kg body weight of aromatic-cationic peptide, such as D-Arg-2',6'-Dmt-Lys-Phe-NH₂, or a pharmaceutically acceptable salt thereof, such as acetate, tartrate, trifluoroacetate, or hydrochloride salt, alone or in combination with one or more additional therapeutic agents for the treatment or prevention of mitochondrial myopathy. Peptides and/or additional therapeutic agents are administered orally, topically, systemically, intravenously, subcutaneously, intraperitoneally, or intramuscularly according to methods known in the art. Subjects will be evaluated weekly for the presence and/or severity of signs and symptoms associated with mitochondrial myopathy, wherein the symptoms are selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, muscle atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy.

### Results

It is predicted that subjects at risk of having or diagnosed as having mitochondrial myopathy and receiving therapeutically effective amounts of aromatic-cationic peptide, such as D-Arg-2',6'-Dmt-Lys-Phe-NH₂, or a pharmaceutically acceptable salt thereof, such as acetate, tartrate, trifluoroacetate, or hydrochloride salt will display delayed onset of mitochondrial myopathy, or prevention of onset of mitochondrial myopathy. It is also expected that mitochondrial myopathy subjects treated with the aromatic-cationic peptide will show normalization of one or more of one or more energy biomarkers selected from the group consisting of: lactic acid (lactate) levels; pyruvic acid (pyruvate) levels; lactate/pyruvate ratios; phosphocreatine levels; NADH (NADH+H⁺) or NADPH (NADPH+H⁺) levels; NAD or NADP levels; ATP levels; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels; beta-hydroxy butyrate levels; acetoacetate/ beta-hydroxy butyrate ratio; 8-hydroxy-2'-deoxyguanosine (8-OHdG) levels; levels of reactive oxygen species; oxygen consumption (VO2), carbon dioxide output (VCO2), and respiratory quotient (VCO2/VO2) levels by at least 10% compared to the untreated mitochondrial myopathy controls. It is further expected that administration of D-Arg-2',6'-Dmt-Lys-Phe-NH₂ in combination with one or more additional therapeutic agents will have synergistic effects in this regard compared to that observed in subjects treated with aromatic-cationic peptides or the additional therapeutic agents alone.

These results will show that aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt are useful in the prevention of mitochondrial myopathy. These results will show that aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt are useful in preventing or delaying the onset of one or more symptoms selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cardiac arrhythmia, cardiac hypertrophy, cerebellar atrophy, cerebral atrophy, muscle atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hypertrophic cardiomyopathy, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced cardiopulmonary capacity, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy.

Accordingly, the peptides are useful in methods comprising administering aromatic-cationic peptides to a subject in need thereof for the prevention of mitochondrial myopathy.

### Example 3 - Use of Aromatic-Cationic Peptides in the Treatment of Mitochondrial Myopathy in a Mouse Model

This example demonstrates the *in vivo* efficacy of aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, in treating mitochondrial myopathy in a mouse model.

### Methods

Homozygous male adenine nucleotide translocator (*Ant1^{PGKneo}*) null mice (as described by Graham, et al. Nature Genetics 16:226-234 (1997)) and wild-type litter mates will be used in this study. Subjects are divided into the following groups with 16 subjects in each group:
(1) Healthy wild-type controls
*(2) Ant1^{PGKneo}* null mice treated with PBS
*(3) Ant1^{PGKneo}* null mice treated with 1 mg/kg D-Arg-2'6'-Dmt-Lys-Phe-NH₂
*(4) Ant1^{PGKneo}* null mice treated with 5 mg/kg D-Arg-2'6'-Dmt-Lys-Phe-NH₂
*(5) Ant1^{PGKneo}* null mice treated with 10 mg/kg creatine, L-carnitine, coenzyme Q₁₀, L-arginine, biotin, cytochrome c, corticosteroids, idebenone, sodium dichloroacetate, thiamine, thiocitic acid, riboflavin, α-tocopherol, succinate, ascorbate, menadione, naphthoquinone, or nicotinamide (administered *via* drinking water)
*(6) Ant1^{PGKneo}* null mice treated with 1 mg/kg D-Arg-2'6'-Dmt-Lys-Phe-NH₂ and 1 mg/kg creatine, L-carnitine, coenzyme Q₁₀, L-arginine, biotin, cytochrome c, corticosteroids, idebenone, sodium dichloroacetate, thiamine, thiocitic acid, riboflavin, α-tocopherol, succinate, ascorbate, menadione, naphthoquinone, or nicotinamide

D-Arg-2'6'-Dmt-Lys-Phe-NH₂ will be formulated in water and administered once daily by subcutaneous bolus injection at either 1 or 5 mg/kg starting from 5 weeks of ages. Urine and blood (serum) samples will be collected every 2 weeks at week 5, 7, 9, 11, 13, 15, and 17. Six animals from each experimental group will be sacrificed for tissue collection at week 17. The remaining 10 animals in each group will continue to be dosed to collect survival data.

*Histopathological analysis.* For histopathological analysis of skeletal muscle, the gastrocnemius muscle will be dissected and frozen fresh in isopentane supercooled by liquid nitrogen. Ten-micrometer frozen transverse sections are cut with a cryostat and stained with modified Gomori's trichrome, and for COX and succinate dehydrogenase (SDH) activities. The COX histochemical stain consists of DAB (3,3'-diaminobenzidine tetrahydrochloride), which gives a brown product with COX activity. The SDH histochemical stain contains NBT (nitroblue tetrazolium), which gives a blue product with SDH activity.

For electron microscopy, the gastrocnemiums muscle are dissected, cut into 0.5-mm cubes, fixed for 1 hour at room temperature in 1% glutaraldehyde in PBS, washed three times in PBS at room temperature and further processed for staining, embedding, sectioning and post-staining. Specimens are then examined and photographed with an electron microscope.

*Exercise stress testing.* Each animal is exercised on an enclosed treadmill (Columbus Instruments, Columbus, OH) supplied with an electrified grid at the rear of the belt to provide motivation. After an initial 5-minute baseline, the mice are subjected to a 20-minute exercise protocol, under constant supervision, during which the workload is increased every 2 minutes by increasing of the belt speed and/or the belt incline. The protocol is composed of the following: 0-5 minutes (at rest), 5-7 minutes (5m/min, 0° incline), 7-9 minutes (7m/min, 0° incline), 9-11 minutes (10m/min, 0° incline), 11-13 minutes (12m/min, 0° incline), 13-15 minutes (15m/min, 10° incline), 19-21 minutes (15m/min, 15° incline), 21-23 minutes (15m/min, 20° incline), and 23-25 minutes (15m/min, 25° incline). Each animal is subjected to a 10-minute run (belt speed = 5m/min, 0° incline) the day before the actual experiments to allow for acclimation. Gas measurements are made during the exercise protocol using an open-flow respirometry system (OXYMAX, Columbus Instruments). Data are collected by computer using OXYMAX software (v. 500). The measurement window is set to 30 second intervals. Each animal is subjected to the protocol at least twice on separate days.

### Results

It is anticipated that untreated *Ant1^{PGKneo}* null mice will exhibit the presence of ragged-red fibers (RRFs), increased succinate dehydrogenase (SDH) activity, increased cytochrome c oxidase (COX) activity, increased serum lactate, pyruvate, and alanine levels, and exercise intolerance as compared to healthy controls. It is anticipated that the *Ant1^{PGKneo}* null mice receiving D-Arg-2',6'-Dmt-Lys-Phe-NH₂ will show improvement in these parameters in a dose dependent manner, and will resemble those observed in healthy wild-type controls. It is further expected that administration of D-Arg-2',6'-Dmt-Lys-Phe-NH₂ in combination with creatine, L-carnitine, coenzyme Q₁₀, L-arginine, biotin, cytochrome c, corticosteroids, idebenone, sodium dichloroacetate, thiamine, thiocitic acid, riboflavin, α-tocopherol, succinate, ascorbate, menadione, naphthoquinone, or nicotinamide will have synergistic effects in this regard compared to that observed in *Ant1^{PGKneo}* null mice treated with D-Arg-2',6'-Dmt-Lys-Phe-NH₂ or creatine, L-carnitine, coenzyme Q₁₀, L-arginine, biotin, cytochrome c, corticosteroids, idebenone, sodium dichloroacetate, thiamine, thiocitic acid, riboflavin, α-tocopherol, succinate, ascorbate, menadione, naphthoquinone, or nicotinamide alone.

These results will show that aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt are useful in treating mitochondrial myopathy in the *Ant1^{PGKneo}* knockout mouse model. These results will show that aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt are useful in restoring exercise tolerance and skeletal muscle morphology in subjects that are at risk of developing mitochondrial myopathy.

Accordingly, the peptides are useful in methods comprising administering aromatic-cationic peptides to a subject in need thereof for the treatment of mitochondrial myopathy.

### Example 4 - Use of Aromatic-Cationic Peptides in the Treatment of Mitochondrial Myopathies in Humans

This example demonstrates the use of aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, in the treatment of mitochondrial myopathy.

### Methods

### Treatment

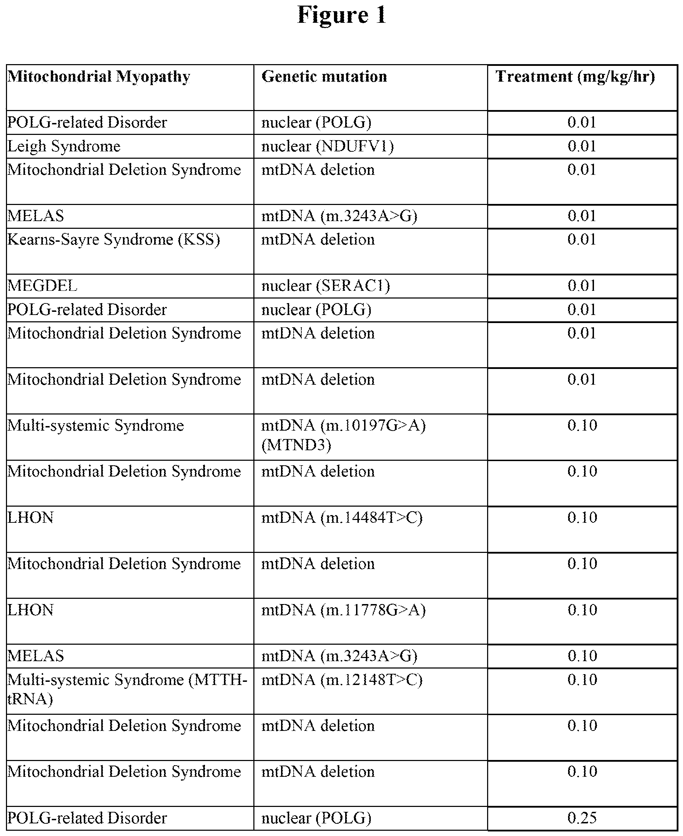

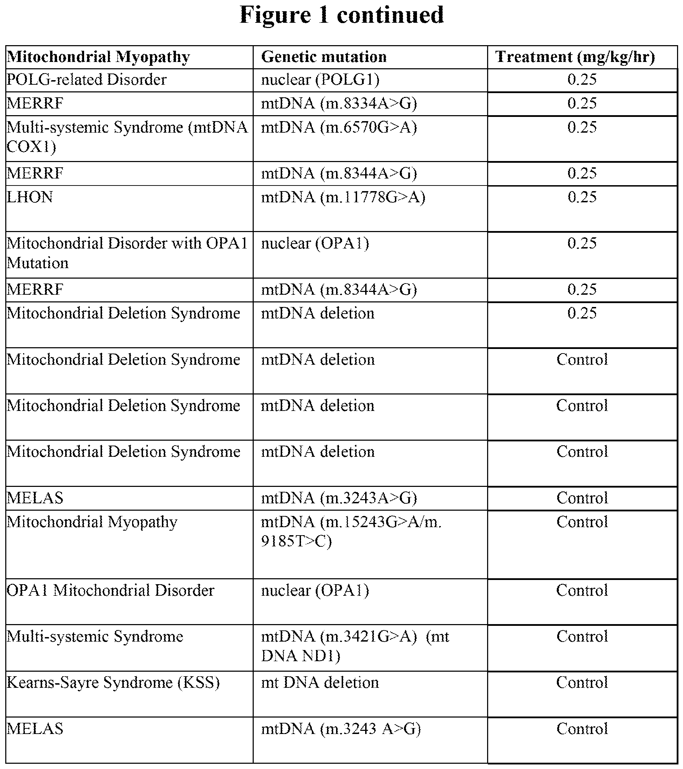

Subjects (ages 16-65 years of age) diagnosed as having mitochondrial myopathy (see Figure 1) were randomly divided into four groups (n=9), wherein Groups 1-3 received daily intravenous administration of D-Arg-2',6'-Dmt-Lys-Phe-NH₂ at 0.01 (Group 1), 0.10 (Group 2), or 0.25 (Group 3) mg/kg body weight/hr for two hours. Control mitochondrial myopathy subjects (Group 4) received equal volumes of saline solution administered for two hours. All groups were treated for five consecutive days. The demographics of the subjects are disclosed in Table 5.

| **Table 5: Subjects' demographics** | | | | | |
|---|---|---|---|---|---|
| | **0.01 mg (n=9)** | **0.1 mg (n=9)** | **0.25 mg (n=9)** | **Placebo (n=9)** | **All Subjects** |
| **Avg. Age** | 40.8 | 45.0 | 42.3 | 41.9 | 42.5 |
| **Gender** | -- | -- | -- | -- | 6 male 30 female |
| **Race** | 9 white | 9 white | 8 white 1 white/Asian | 9 white | 35 white |
| **Avg. BMI** | 25.5 | 24.3 | 21.5 | 21 | 23.1 |

Mitochondrial myopathy symptoms expressed by the subjects included: fatigue (n=35), exercise intolerance (n= 34), muscle weakness (n=31), muscle cramps (n=12), muscle atrophy (n=9), other symptoms (*e.g*., ptosis, ophthalmoplegia, and dysphagia) (n=6), and fasciculations (n=4).

### Six-Minute Walk Test (6MWT)

Treatment of mitochondrial myopathy with D-Arg-2',6'-Dmt-Lys-Phe-NH₂ was assessed by the 6MWT. The 6MWTtest measures the distance a subject walks on a hard flat surface within 6 minutes.

The distance walked in 6 minutes by each subject was measured on day 1 before the first treatment and on day 5 before the fifth (and final) treatment.

### Results

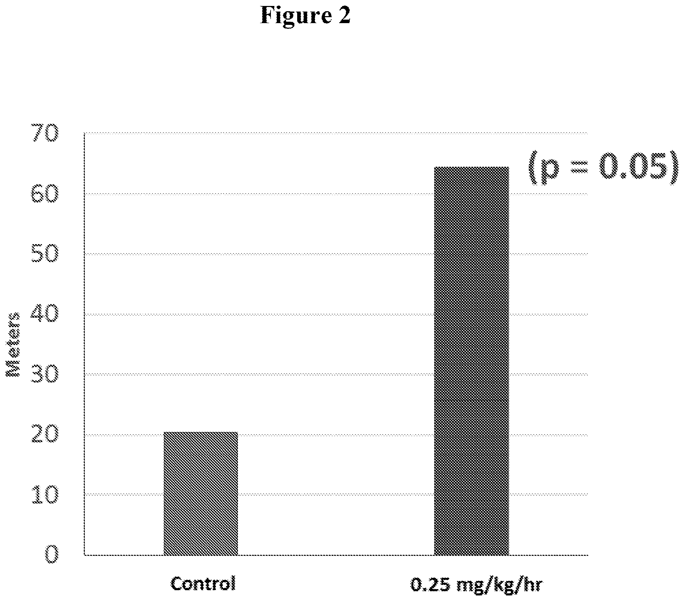

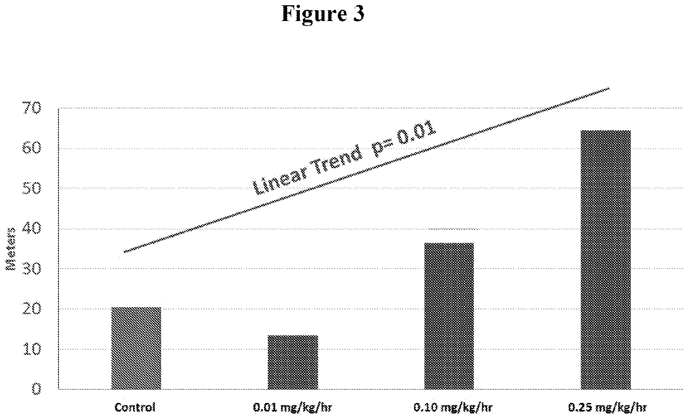

Figure 2 shows that subjects treated with 0.25 mg/kg /hr had a significant increase in the distance walked in the 6MWT as compared to the control group. Figure 3 shows that there was a significant dose dependent increase in the distance walked in the 6MWT in the D-Arg-2',6'-Dmt-Lys-Phe-NH₂ treated groups as compared to the control group.

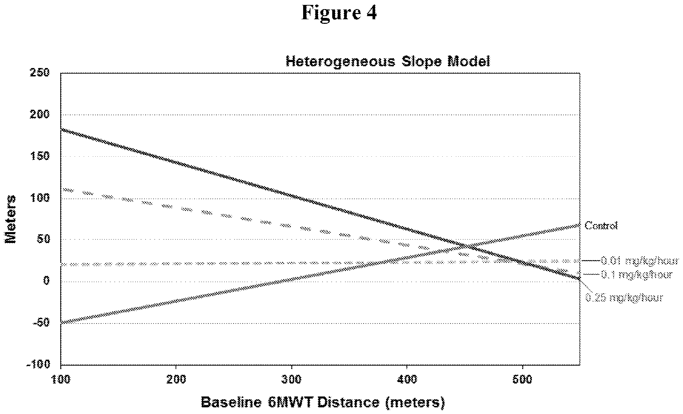

Figure 4 shows a heterogeneous slope model for the female subjects enrolled in the trial. The model indicates the amount of treatment benefit with respect to improvement in 6 minute walk distance at Day 5 as a function of the baseline distance walked. Figure 4 suggests a dose-dependent response (greatest benefit for high dose (0.25 mg/kg/hr), then 0.10 mg/kg/hr, followed by 0.01 mg/kg/hr, and lastly placebo). The slopes of the lines which estimate the amount of improvement as a function of baseline distance walked show a statistically significant difference between the high dose cohort (0.25 mg/kg/hr) and placebo (p=0.005).

None of the test subjects exhibited any serious adverse events from treatment with D-Arg-2',6'-Dmt-Lys-Phe-NH₂. Adverse event rates were similar between treatment and control groups. Adverse events with >1 subject incidence included: headaches (n=4), dizziness (n=3), dyspnea (n=2), and abdominal pain (n=2). There were no significant trends changes in vital signs, electrocardiograms, or clinical laboratory findings

These results show that aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt are useful in the treatment of mitochondrial myopathies.

### Example 5 - Use of Aromatic-Cationic Peptides in the Treatment of Mitochondrial Myopathy in Humans

This example demonstrates the use of aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, in the treatment of mitochondrial myopathy.

### Methods

Subjects diagnosed with mitochondrial myopathy are treated as described in Table 6 (sc=subcutaneous injection).

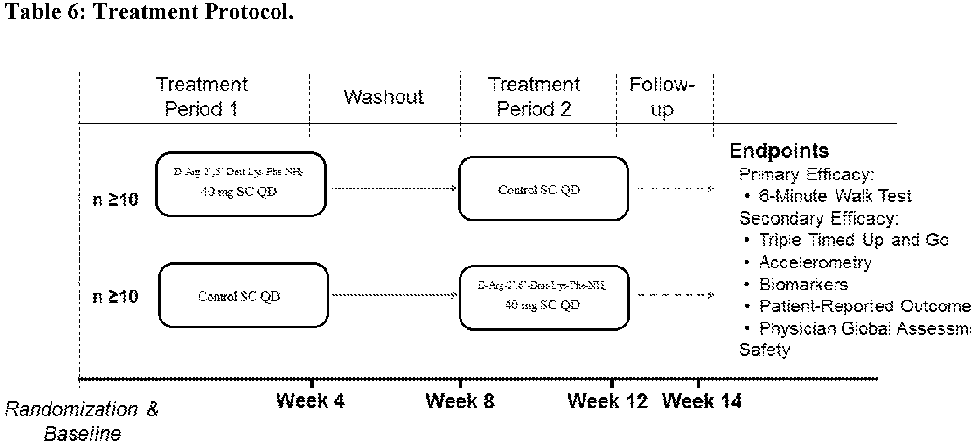

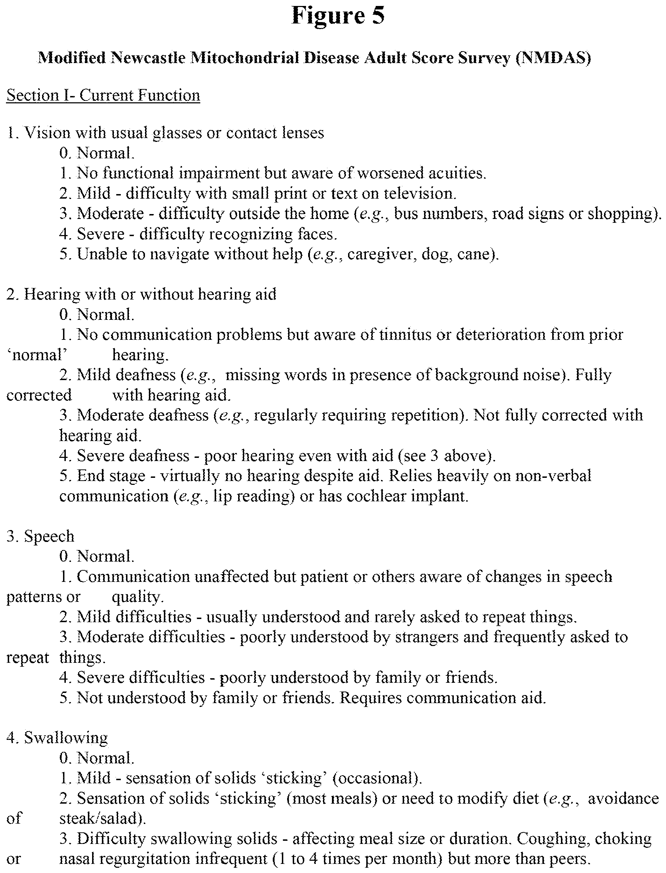

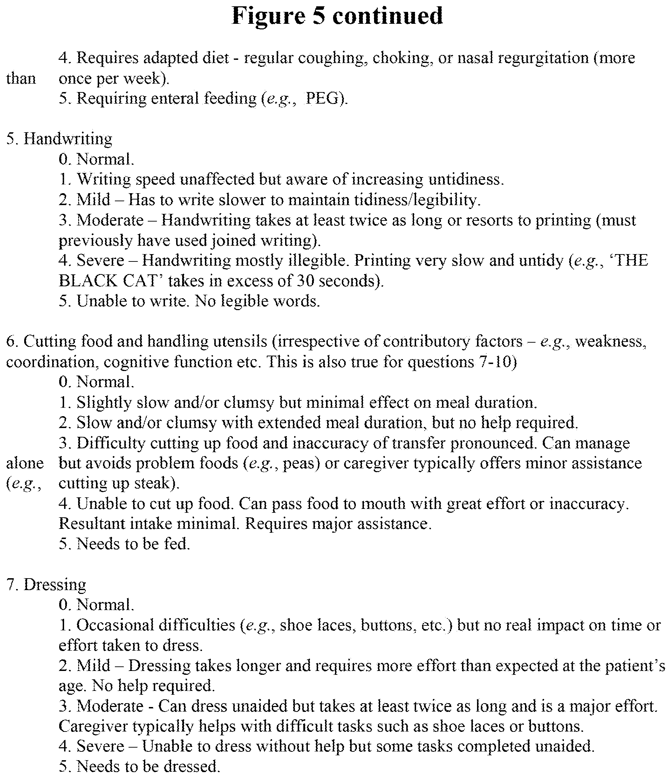

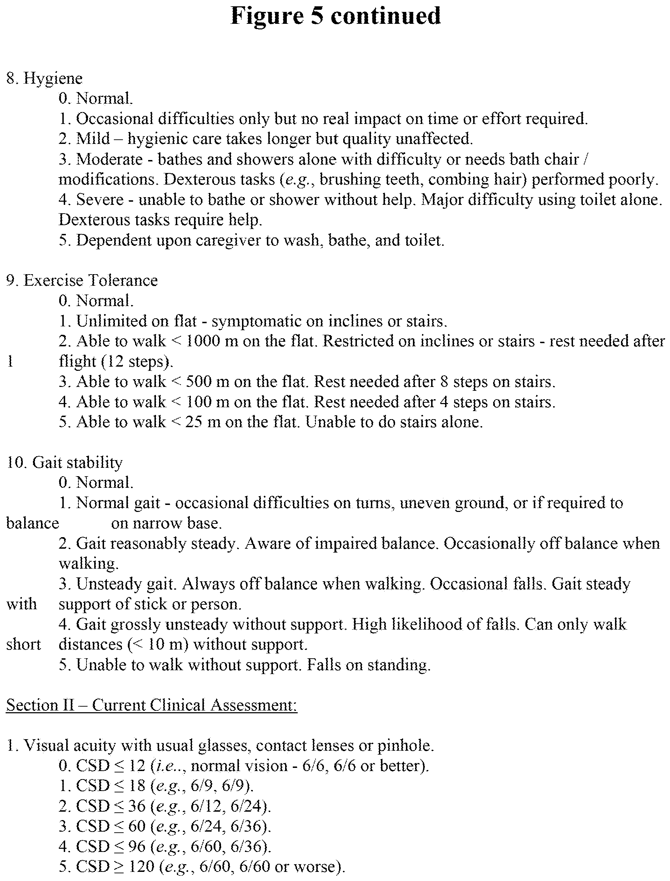

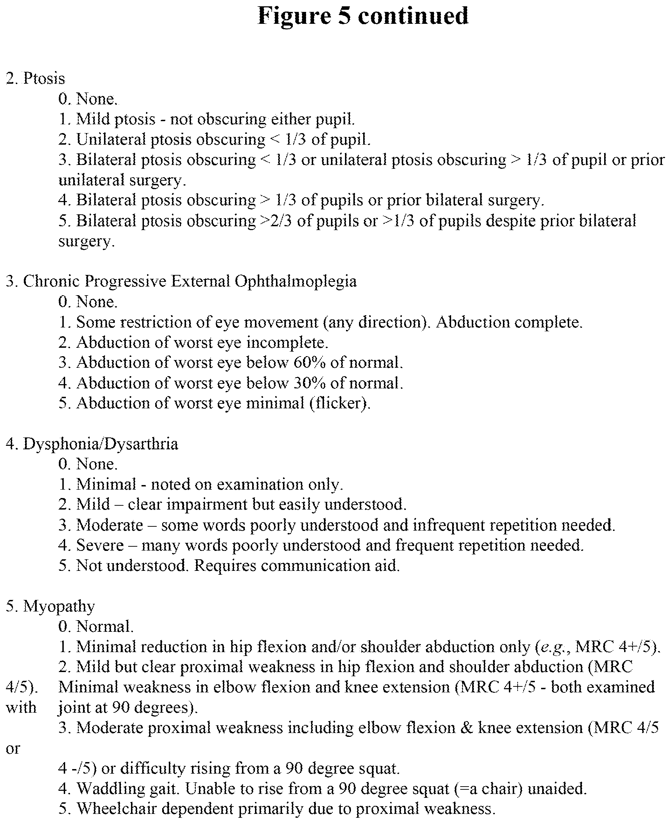

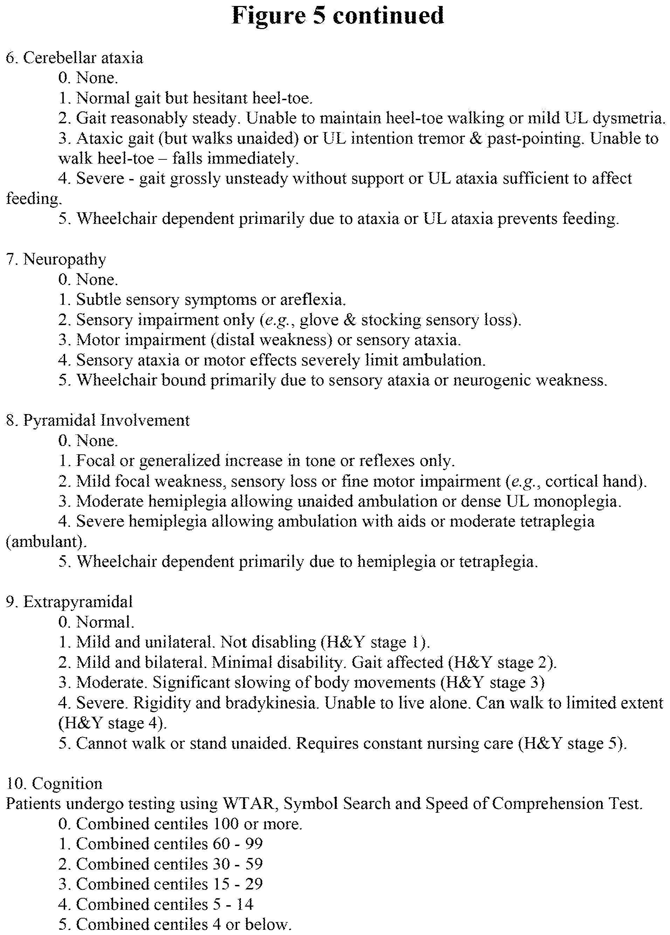

Each subject is administered a cardiopulmonary exercise test (CPET) and answers a modified Newcastle Mitochondrial Disease Adult Score survey (NMDAS) (see Figure 5) on day 1 before the first treatment and on day 5 before the fifth (and final) treatment. Additionally, each subject answers a Daily Symptom Questionnaire (DSQ)(see Table 7) from day 1 to day 5 of treatment.

| **Table 7: Daily Symptom Questionnaire** | |
|---|---|
| Rate the following on a scale from 0-10, where 0 = none/no symptoms and 10 = very severe symptoms. | |
| Symptom | Rate: 0-10 |
| Abdominal pain | |
| Limitation on activities (such as walking or climbing stairs) | |
| Muscle pain | |
| Muscle weakness | |
| Mental fatigue | |
| Physical fatigue | |

### Results

It is predicted that subjects treated with D-Arg-2',6'-Dmt-Lys-Phe-NH₂ will show an increase in peak oxygen uptake (VO₂) (*e.g.,* an increase in aerobic capacity) and/or an increase of VO₂ at anaerobic threshold (as measured by CPET) as compared to untreated subjects. Alternatively, or additionally, it is also predicted that subjects treated with D-Arg-2',6'-Dmt-Lys-Phe-NH₂ will normalize VO₂ levels as compared to a healthy control subject without mitochondrial myopathy. It is also anticipated that subjects treated with D-Arg-2',6'-Dmt-Lys-Phe-NH₂ will show improved scores on the modified NMDAS and DSQ surveys as compared to untreated subjects.

These results will show that aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt are useful in the treatment of mitochondrial myopathy. Accordingly, the peptides are useful in methods comprising administering aromatic-cationic peptides to a subject in need thereof for the treatment of mitochondrial myopathy.

### Example 6 - Use of Aromatic-Cationic Peptides to Increase ATPₘₐₓ and Improve Skeletal Muscle Function in Elderly Subjects

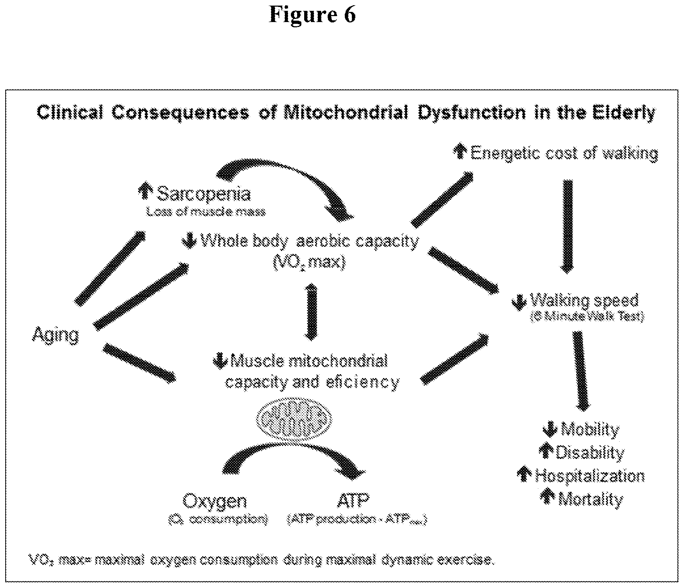

This example demonstrates the use of aromatic-cationic peptides, such as D-Arg-2',6'-Dmt-Lys-Phe-NH₂ (MTP-131), or a pharmaceutically acceptable salt thereof, such as acetate, tartrate, trifluoroacetate, or hydrochloride salt, to elevate ATPₘₐₓ and improve muscle function in elderly subjects. Exemplary clinical consequences of mitochondrial dysfunction as related to muscle physiology in the elderly are shown in Figure 6.

### Methods

### Study Overview

The study was a randomized, double-blind placebo-controlled, single-center study in male and female elderly subjects with evidence of skeletal muscle mitochondrial dysfunction. The elderly subjects in the study were administered a single double-blind dose of either D-Arg-2',6'-Dmt-Lys-Phe-NH₂ or placebo. A sufficient number of subjects were screened in order to have 40 subjects (20 treated with D-Arg-2',6'-Dmt-Lys-Phe-NH₂ and 20 treated with placebo) complete the study. The duration of the study included a Screening Period of up to 28 days, a 1-day Treatment Period and a 7-day Observation Period.

### Diagnosis and Main Inclusion Criteria

Adults enrolled in this study were ≥ 60 and ≤ 85 years-of-age, with a body mass index (BMI) between 16 and 35 kg/m² with mitochondrial dysfunction defined as *in vivo* ³¹P MRS- and OPS determined by maximal adenosine triphosphate (ATP) synthetic rate (phosphorylation capacity per unit muscle volume [ATP]) max < 0.70 mM/sec and *in vivo* ³¹P MRS- and OPS determined mitochondrial coupling Phosphate/Oxygen (P/O) of < 1.9.

### Dosage and Administration

D-Arg-2',6'-Dmt-Lys-Phe-NH₂ or placebo was administered as an intravenous infusion at 0.25 mg/kg/hour and at a rate of 60 mL/hour for 2 hours.

### Criteria for Evaluation

Primary endpoint: The primary efficacy endpoint was the change from baseline after treatment with D-Arg-2',6'-Dmt-Lys-Phe-NH₂ or placebo in the maximum ATP synthetic rate (ATPₘₐₓ).

Secondary endpoints: Secondary analyses included comparisons in change from baseline between treatment groups for the following hand skeletal muscle energetics and functional properties that were analyzed in the same manner as the primary efficacy measures:
- P/O
- Nicotinamide adenine dinucleotide (NAD) levels; and
- Muscle force-time-integral

The mean change from baseline between treatment groups with respect to muscle performance, measured as muscle work rate and maximum integrated force generated, was calculated from the results of the hand muscle fatigue test. Change from baseline for the above measures was assessed at Hour 2 (2 hours after the start of infusion or end of infusion) and Day 7, except for muscle performance, which was assessed at Day 3. The mean change between treatment groups was compared in an analysis of covariance (ANCOVA) framework, with baseline as a covariate.

### Magnetic Resonance Spectroscopy, Optical Spectroscopy, and Muscle Fatigue Testing

The following procedures were used in magnetic resonance spectroscopy (MRS), optical spectroscopy, and muscle fatigue testing of the elderly subjects.

³¹P MRS Procedures:
a. Positioning/Scanning (∼20 minutes):
   i. Hand Positioning: Fit right hand into magnetic resonance (MR) cradle to position index finger for force measurement, secure MR coil to hand with tape and secure arm/hand to cradle with Velcro, and fit the blood pressure (BP) cuff on upper right arm.
   ii. Subject Positioning: Move subject into magnet room, position subject in supine position on gurney, place MR holder in magnet and adjust subject's position on gurney to ensure comfort.
   iii. Cradle Positioning in Magnet: Adjust position of MR cradle in magnet bore to center of magnet and attach all electronic connections.
   iv. Hand Muscle Force Measure: Request the subject to push index finger against force transducer to generate a muscle maximum voluntary contraction (MVC). Follow force by the number of lights activated on a Light Emitting Diode (LED) panel. Instruct the subject to activate half the number of lights (70% MVC) during the exercise period.
   v. Scanning: Provide subject with earplugs, inform about MR generated noises, tune MR probe, close door, optimize MR measures, and take fully relaxed spectrum.
b. Ischemia (∼40 minutes):
   i. Pre-ischemia Measures: Instruct subject about timing, start experimental scans (5 minutes), and warn subject 1 minute before ischemia/exercise.
   ii. Ischemic Protocol: Start scan of resting first dorsal interosseous (FDI) muscle (Minute 0); warn subject about onset of ischemia (Minute 7); inflate BP cuff on right arm and instruct subject to start exercise (Minute 8); contract muscle with each beat of a metronome (40 beats/minute) until instructed to stop (∼30 sec; Minute 8.5), ischemia is maintained until Minute 23 (total ischemia = 15 minutes), release cuff and allow muscle to recover until Minute 35 (total recovery = 12 minutes).

Optical Spectroscopy Procedures:
a. Hand Positioning: Fit right hand into optics cradle with optics probe positioned over FDI muscle, fit the BP cuff on upper right arm, fit subject with mask for 100% O₂.
b. Optics/Ischemia Measures: Collect optical spectra for 3 minutes, turn on O₂ supply for 5 more minutes and continue throughout protocol; inflate BP cuff (Minute 8) and keep inflated for 15 minutes; release BP cuff at Minute 23 and follow recovery for 12 minutes (to Minute 38).

Muscle Fatigue Procedures (Hand Fatigue):
a. Exercise Instruction: Review exercise protocol to re-familiarize subject.
   i. ATPmax Test: Instruct subject to exercise FDI muscle as fast as possible when instructed and to stop immediately when instructed (∼30 seconds)
   ii. Sustained Hand Fatigue Test: Review exercise protocol. Request the subject to push index finger against force transducer to generate a muscle MVC. Instruct subject to exercise FDI muscle at 70% MVC (as shown by the light box) at rate set by a metronome for 2 minutes and to increase rate with a metronome every 2 minutes. Practice with hand in MR cradle outside magnet. Practice tests with right hand in MR cradle outside magnet.
b. Positioning/Scanning:
   i. Hand Positioning: Fit right hand into MRS cradle to position finger for force measurement, secure MR coil to hand with tape and secure arm/hand to cradle with Velcro, and fit the BP cuff on upper right arm.
   ii. Subject Positioning: Move subject into magnet room, position subject in supine position on gurney, place MR holder in magnet and adjust subject's position on gurney to ensure comfort.
   iii. Cradle Positioning in Magnet: Adjust position of MR cradle in magnet bore to center of magnet and attach all electronic connections.
   iv. Hand Muscle Force Measure: Request the subject to push index finger against force transducer to generate a muscle MVC. Follow force by the number of lights activated on a LED panel. Instruct the subject to activate half the number of lights (70% MVC) during the exercise period.
   v. Scanning: Provide subject with earplugs, inform about MR generated noises, tune MR probe, close door, optimize MR measures, and take fully relaxed spectrum.
c. ATPₘₐₓ Test: Start scan of resting FDI muscle (Minute 0); warn subject about onset of exercise (Minute 7); instruct subject to start exercise (Minute 8); contract muscle as fast as possible until instructed to stop (∼30 seconds; Minute 8.5), allow muscle to recover until Minute 20.5 (total recovery = 12 minutes).
d. Fatigue Test: Start scan of resting muscle (Minute 0); warn subject about onset of exercise (Minute 7); instruct subject to start exercise at 70% MVC (Minute 8) at the rate set by a metronome and to increase exercise rate with the faster beat of the metronome, which occurs in 2 minute increments until 70% MVC cannot be maintained (~8 minutes; Minute 16); allow muscle to recover for 12 minutes.

### Results

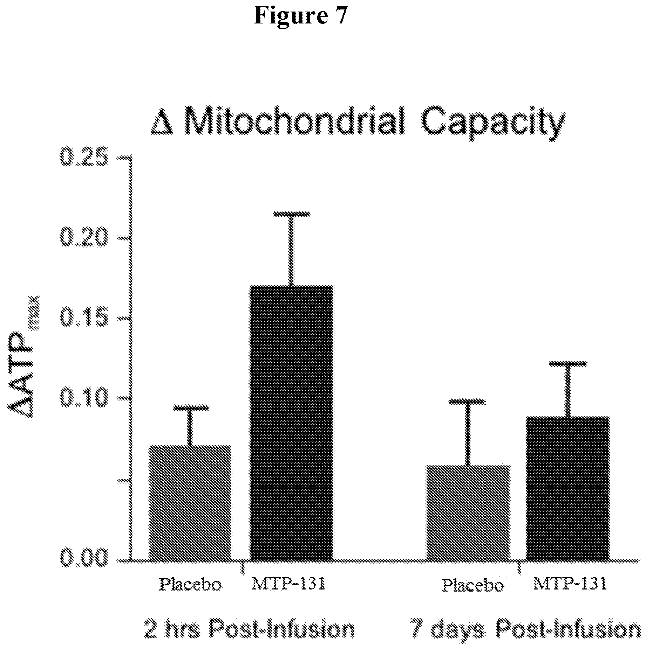

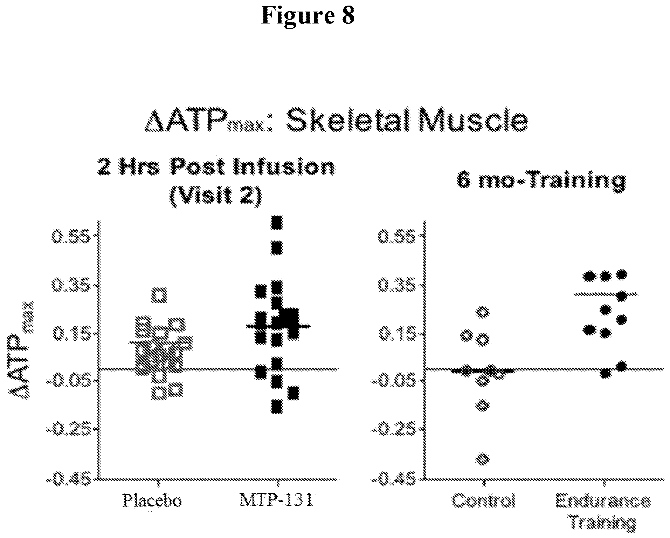

Elderly subjects treated with D-Arg-2',6'-Dmt-Lys-Phe-NH₂ showed an increase in ATPₘₐₓ as compared to placebo treated subjects at 2 hours post infusion and at day 7 post-infusion (Figure 7). Additionally, the observed increase in ATPₘₐₓ in treated elderly subjects as compared to untreated elderly subjects at 2 hours post-infusion was comparable to a previous study (Conley et al., J Physiol., 526(Pt 1): 203-210 (July 1, 2000)) that showed increased ATPₘₐₓ in elderly subjects 6 months post endurance training as compared to control elderly subjects (*i.e.,* no endurance training) (Figure 8).

The higher ATPₘₐₓ values were associated with greater function as measured by Force Time Integral (r=0.267, p=0.0041) (data not shown).

Elderly subjects treated with D-Arg-2',6'-Dmt-Lys-Phe-NH₂ showed improved mitochondrial function, as indicated by increased ATPₘₐₓ, after a single infusion as compared to placebo treated elderly subjects.

These results show that treatment with D-Arg-2',6'-Dmt-Lys-Phe-NH₂ increases ATPₘₐₓ in muscle and improves sustained exercise performance in the muscle of elderly subjects. Accordingly, the peptides disclosed herein are useful in methods for the treatment of mitochondrial myopathy and improving skeletal muscle function of elderly subjects.

### Example 7 - Use of Aromatic-Cationic Peptides to Increase ATPₘₐₓ in Aged Mice

This example demonstrates the use of aromatic-cationic peptides, such as D-Arg-2',6'-Dmt-Lys-Phe-NH₂ (MTP-131), or a pharmaceutically acceptable salt thereof, such as acetate, tartrate, trifluoroacetate, or hydrochloride salt, to elevate ATPₘₐₓ in aged mice (see complete study at Siegel etal., Aging Cell, 12: 763-771 (2013)).

### Methods

Five month (young) and 27 month (aged) old female C57BL/6 mice were purchased from the NIA aged mouse colony. All mice were exposed to a 12 hour light/dark cycle in a fixed-temperature environment with free access to water and standard mouse chow until immediately prior to experimentation. Treatments were administered by intraperitoneal injection of isotonic saline or 3 mg/kg D-Arg-2',6'-Dmt-Lys-Phe-NH₂ dissolved in isotonic saline at a concentration of 0.3 mg/mL. Mouse body temperatures were maintained at 36°C ± 1°C throughout *in vivo* and *in situ* experiments.

*In Vivo* Metabolic Spectroscopy: Mice were anesthetized by intraperitoneal injection of 0.01 ml/g of 2.5% tribromoethanol ("Avertin", Sigma), the distal hindlimb was shaved, and the mouse was suspended by flexible straps within a custom built combined MR/optics probe for use with a 7T vertical bore spectrometer (Varian, Palo Alto, CA). The distal hindlimb was centered within a horizontal MR solenoid coil tunable to both 1H and 31P with fiber optic bundles positioned on either side to simultaneously collect MR and optical spectra from intact skeletal muscle. After positioning the mouse, MR signal was optimized by shimming the 1H of tissue water and optical signal was optimized by adjusting acquisition time. Next, a high signal to noise 31P spectrum was acquired under fully relaxed conditions (32 transients, 4096 complex points, 10 kHz sweep width, 25 sec interpulse delay). Finally, dynamic optical (0.5 sec delay) and MR (45° flip angle, 4 transients, 4096 complex points, 10 kHz sweep width, 1.5 sec interpulse delay) spectra were acquired continuously through periods of rest (2 min), ischemia (11 min), and recovery (7 min). After the first minute of rest mice breathed 100% O₂ for the remainder of each dynamic experiment. *In vivo* spectroscopy data were acquired approximately one hour after a single injection with either saline or D-Arg-2',6'-Dmt-Lys-Phe-NH₂.

Tissue Preparation: Immediately following *in vivo* spectroscopy the skeletal muscles of the distal hindlimb were dissected and flash-frozen in liquid nitrogen. From the left leg, extensor digitorum longus, gastrocnemius, soleus, and tibialis anterior muscles were pooled and pulverized over liquid nitrogen for measurement of mixed muscle metabolite, hemoglobin, and myoglobin concentrations. From the right leg, gastrocnemius was pulverized over liquid nitrogen and prepared for western blotting. All muscle samples were stored at -80°C until the day of assay.

*In vivo* Spectroscopy Data Analysis: ³¹P MR spectra were exponentially multiplied, Fourier transformed, and manually phase corrected using Varian VNMR (7T) software. The resulting spectra were taken to custom written MATLAB software (Mathworks, Natick, MA) for the remainder of analysis. Raw optical spectra files, collected using WinSpec (Princeton Instruments), were taken directly to custom written MATLAB software for analysis. Relative peak integrals from fully relaxed 31P MR spectra were used to calculate the resting inorganic phosphate (Pi)/ATP and PCr/ATP ratios. Three consecutive dynamic spectra were summed to improve signal-to noise ratio and then the Fit-to-Standard algorithm was used to determine PCr and Pi peak magnitudes throughout dynamic acquisition. After correcting for variable relaxation, the ATP concentration from HPLC analysis of mixed muscle was used as an internal reference to calculate absolute PCr and Pi concentrations over time. pH was determined using the chemical shift between Pi and PCr peaks, and ADP and AMP concentrations were calculated using the known kinetics of the creatine kinase and adenylate kinase reactions, assuming equilibrium conditions and a Mg²⁺ concentration of 0.6 mM.

Optical spectra were analyzed using a partial-least squares routine to determine the O₂ saturations of Hb and Mb throughout dynamic spectral acquisition. Second derivatives of optical spectra were used to minimize the influence of tissue scattering. The concentrations and known O₂ binding kinetics of Hb and Mb were then used to calculate net O₂ flux in the closed system of the ischemic hindlimb.

The resting rates of mitochondrial ATP production (ATPase) and O₂ consumption were calculated during ischemia from least-squares linear approximations of the decline in PCr and O₂, respectively, during the initial phase of ischemia. The maximum rate of oxidative phosphorylation (ATPmax) was calculated using a leastsquares monoexponential approximation of PCr recovery during recovery from ischemia.

### Results

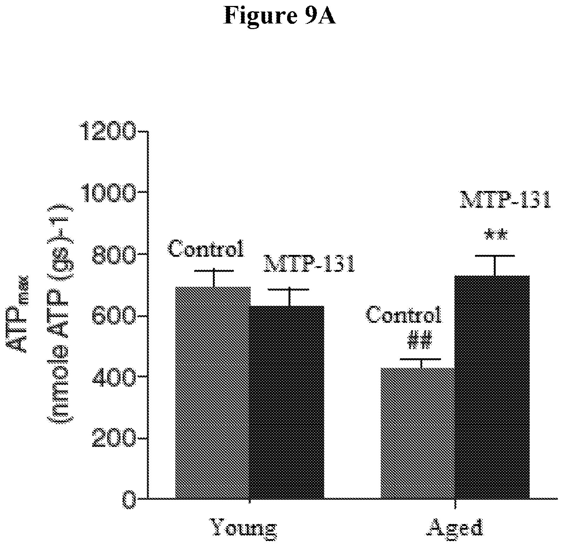

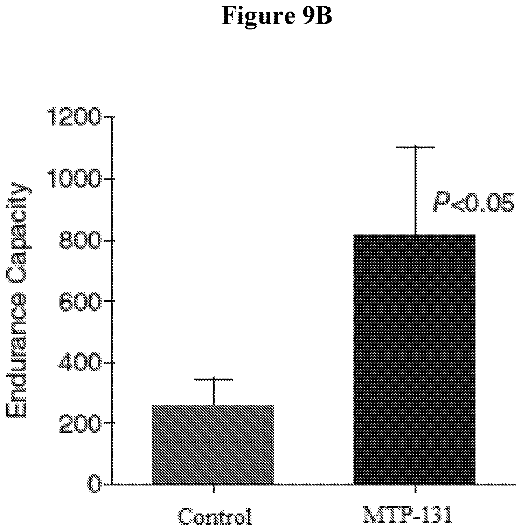

Aged mice treated with D-Arg-2',6'-Dmt-Lys-Phe-NH₂ showed an increase in ATPₘₐₓ as compared to aged untreated mice (Figure 9A). Additionally, aged mice treated with D-Arg-2',6'-Dmt-Lys-Phe-NH₂ showed an increase in endurance capacity as compared to untreated aged mice (Figure 9B).

This data shows that treatment with D-Arg-2',6'-Dmt-Lys-Phe-NH₂ increases ATPₘₐₓ in muscle and improves endurance capacity in the muscles of aged mice. Accordingly, the peptides disclosed herein are useful in methods for the treatment of mitochondrial myopathy and improving skeletal muscle function of elderly subjects.

### Example 8- Use of Aromatic-Cationic Peptides in the Treatment of Mitochondrial Myopathy in Humans

This example demonstrates the use of aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt, in the treatment of mitochondrial myopathy.

### Methods

A randomized, double-blind, placebo-controlled crossover study was performed. The study enrolled 30 subjects with genetically confirmed mitochondrial disease, who completed participation in the SPIMM-201 study.

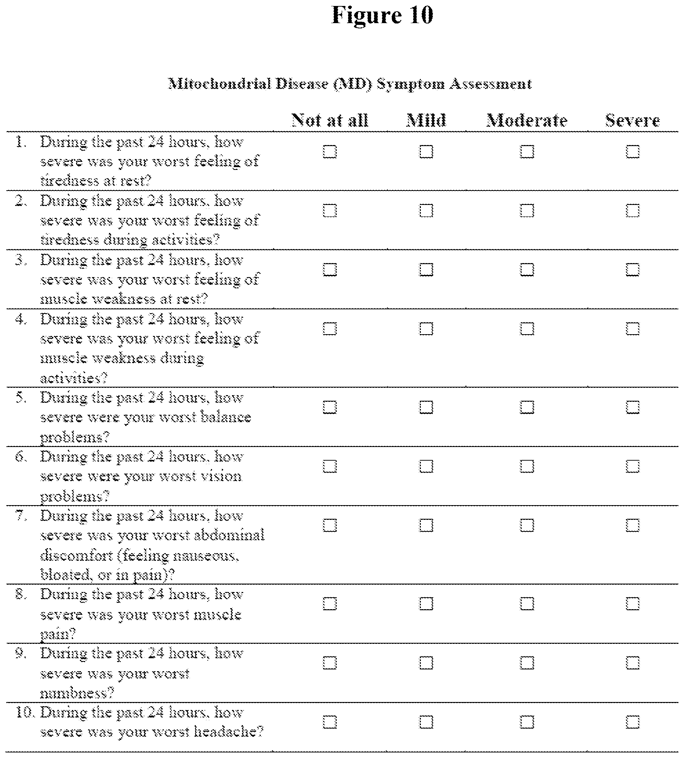

Subjects were randomized (1:1) into one of two sequence groups: Group 1: 4-weeks of treatment with 40 mg D-Arg-2',6'-Dmt-Lys-Phe-NH₂ administered once daily SC or Group 2: 4-weeks of treatment with placebo administered once daily SC. On Day 1 of study, baseline measurements were taken before treatments began. *See* Figure 10.

All subjects completed daily Mitochondrial Disease Symptom Assessments (MDSA) (*see* Figure 10). All subjects took part in a 6-minute walk test (6MWT) before treatments (predose) and at the end of treatment.

### Results

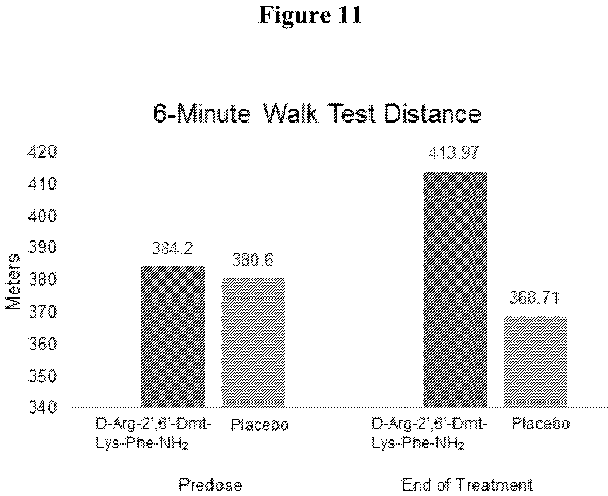

Subjects treated with D-Arg-2',6'-Dmt-Lys-Phe-NH₂ showed an increase in their distance traveled in the 6MWT after treatment as compared to their distance travel in the 6MWT before treatment (Figure 11). Subjects treated with placebo showed a decrease in their distance traveled in the 6MWT after treatment as compared to their distance travel in the 6MWT before treatment (Figure 11).

Subjects treated with D-Arg-2',6'-Dmt-Lys-Phe-NH₂ also traveled further in the 6MWT as compared to untreated subjects (Figure 11) at the end of treatment.

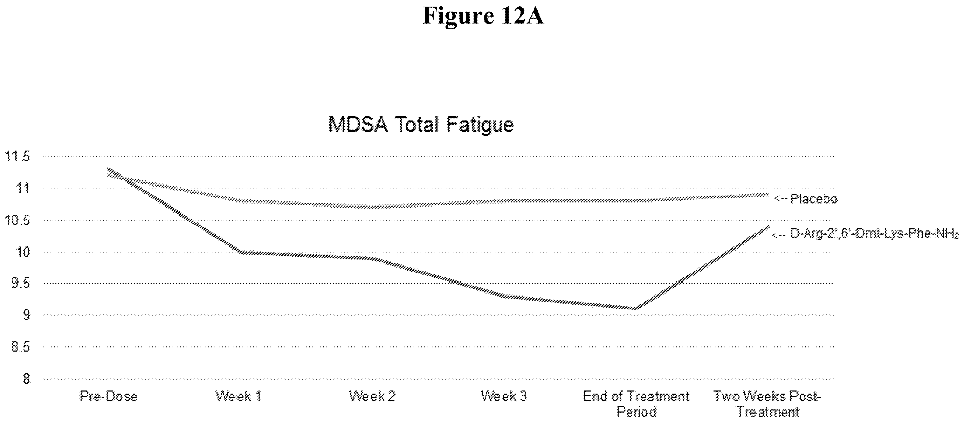

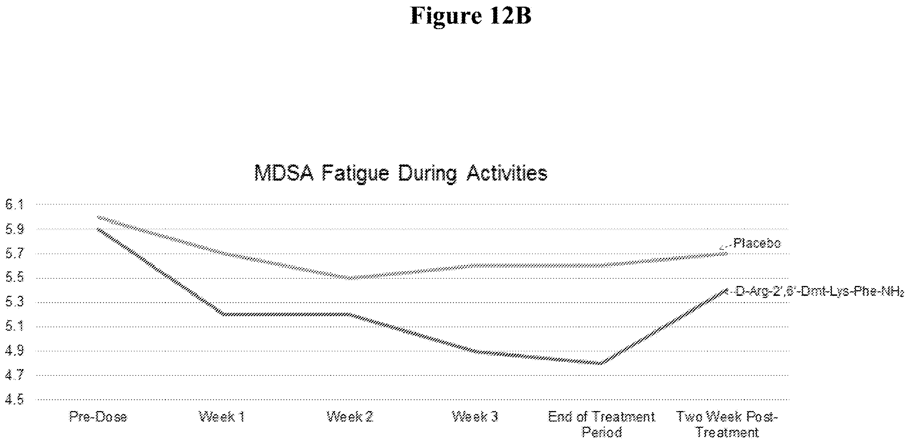

Additionally, subjects treated with D-Arg-2',6'-Dmt-Lys-Phe-NH₂ showed a reduction in Total Fatigue and Total Fatigue during Activites as measure by the MDSA. (Figures 12A and 12B).

The results also show that D-Arg-2',6'-Dmt-Lys-Phe-NH₂ treated subjects showed an increase in Total Fatigue and Total Fatigue during Activites once they were no longer receiving treatment (*see* Two Weeks Post-Treatment in Figures 12A and 12B). Subjects that were treated with placebo did not show a similar increase in Total Fatigue and Total Fatigue during Activites. As such, the increase of fatigue in the D-Arg-2',6'-Dmt-Lys-Phe-NH₂ treated subjects no longer receiving treatment appears to be the result of no longer receiving D-Arg-2',6'-Dmt-Lys-Phe-NH₂ treatment.

These results show that aromatic-cationic peptides, such as D-Arg-2',6'-Dmt-Lys-Phe-NH₂, or a pharmaceutically acceptable salt thereof, such as acetate, tartrate, trifluoroacetate, or hydrochloride salt are useful in the treatment of mitochondrial myopathies.

### EQUIVALENTS

The present technology is not to be limited in terms of the particular embodiments described in this application, which are intended as single illustrations of individual aspects of the present technology. Many modifications and variations of this present technology can be made without departing from its spirit and scope, as will be apparent to those skilled in the art. Functionally equivalent methods and apparatuses within the scope of the present technology, in addition to those enumerated herein, will be apparent to those skilled in the art from the foregoing descriptions. Such modifications and variations are intended to fall within the scope of the appended claims. The present technology is to be limited only by the terms of the appended claims, along with the full scope of equivalents to which such claims are entitled. It is to be understood that this present technology is not limited to particular methods, reagents, compounds compositions or biological systems, which can, of course, vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member. Thus, for example, a group having 1-3 cells refers to groups having 1, 2, or 3 cells. Similarly, a group having 1-5 cells refers to groups having 1, 2, 3, 4, or 5 cells, and so forth.

All patents, patent applications, provisional applications, and publications referred to or cited herein are incorporated by reference in their entirety, including all figures and tables, to the extent they are not inconsistent with the explicit teachings of this specification.

Other embodiments are set forth within the following claims.

## Claims

1. A peptide for use in treating or preventing mitochondrial myopathy in a human subject in need thereof, wherein the peptide is D-Arg-2',6'-Dmt-Lys-Phe-NH₂ or a pharmaceutically acceptable salt thereof, wherein the mitochondrial myopathy is selected from the group consisting of Kearns-Sayre syndrome (KSS); MEGDEL Syndrome; mitochondrial DNA depletion syndrome (MDS); mitochondrial myopathy, encephalomyopathy, lactic acidosis, and stroke-like episodes (MELAS); myoclonus epilepsy with ragged-red fibers (MERRF); mitochondrial neurogastrointestinal encephalomyopathy (MNGIE); neuropathy, ataxia and retinitis pigmentosa (NARP); *OPA1* mutations; Pearson syndrome; and progressive external ophthalmoplegia (PEO), wherein the peptide is administered subcutaneously, daily for four weeks or more.

2. The peptide for use according to claim 1, wherein the subject has been diagnosed as having mitochondrial myopathy.

3. The peptide for use according to claim 1, wherein the signs or symptoms of mitochondrial myopathy comprise one or more symptoms selected from the group consisting of abnormal breathing rhythm, abnormal choroid plexus function, accumulation of metabolites, acidosis, asymmetric vascular dilatation, ataxia, basal ganglia calcifications, basal ganglia lesions, bilateral striatal necrosis, borborygmi, brainstem events with oculomotor palsies, brisk tendon reflexes, cachexia, carbohydrate intolerance, cerebellar atrophy, cerebral atrophy, muscle atrophy, chorea, choreoathetosis, chronic partial denervation, constipation, COX deficiency in muscle, dementia, demyelinization of corticospinal tracts, developmental delay, diarrhea, diffuse leukoencephalopathy, distal arthrogryposis, distal renal tubular acidosis, dysarthria, dysmorphic facies, dysphagia, dystonia, elevated plasma deoxyuridine and deoxythymidine levels, elevated plasma thymidine levels, elevated serum creatine kinase levels, encephalopathy, epigastralgia, episodic encephalopathy, exercise intolerance, exocrine insufficiency, gait impairment, gastrointestinal dysmotility, glucose intolerance, heart block, hemiplegia, hereditary spastic paraparesis, high CSF protein levels, high homovanillic acid (HVA) in CSF, high lactate levels in CSF, hypertelorism, hypertension, hyperventilation, hypoacusis, hypoplasia of the corpus callosum, hypotonia, incomplete right bundle branch block, increased tendon reflexes, lactic acidosis, limb athetosis, limb spasticity, limitation or absence of movement in all fields of gaze, lordosis, loss verbal milestones, low 5-methyltetrahydrofolate (5-MTHF) in CSF, mental retardation, mitochondrial capillary angiopathy, mitochondrial proliferation in muscle, motor retardation, motor spasticity, mtDNA depletion, myelopathy, nausea, nephrotic syndrome, neuronal hyperexcitability, nystagmus, occasional fatigue or pain on exertion, pancreatitis, paralysis, paresthesias, Parkinsonism, peripheral neuropathy, Pes cavus, pigmentary degeneration of retina (retinitis pigmentosa), progressive encephalopathy, progressive or acute encephalopathy, proximal renal tubular acidosis, pseudoathetosis, ptosis, Purkinje dendrite cactus formations with increased mitochondria, pyramidal features, ragged-red fibers, reduced respiratory drive, renal cysts, respiratory failure, rhabdomyolysis, reduced maximal whole body oxygen consumption (VO₂ max), seizures, sensory neuropathy, sensory-motor polyneuropathy, sialoadenitis focal segmental glomerulosclerosis, small fiber modality loss, spasticity, status spongiosis in gray and white matter, recurrent apnea, stroke, subacute necrotizing encephalomyelopathy, tetany, tonic-clonic seizures, tubular dysfunction, variation in muscle fiber size, vascular narrowing, vertebral anomalies, vomiting, weakness, weight loss, and white matter atrophy.

4. The peptide for use according to claim 1, wherein the subject displays abnormal levels of one or more energy biomarkers compared to a normal control subject.

5. The peptide for use according to claim 4, wherein the energy biomarker is selected from the group consisting of lactic acid (lactate) levels; pyruvic acid (pyruvate) levels; lactate/pyruvate ratios; phosphocreatine levels; NADH (NADH+H⁺) or NADPH (NADPH+H⁺) levels; NAD or NADP levels; ATP levels; reduced coenzyme Q (CoQ^{red}) levels; oxidized coenzyme Q (CoQ^{ox}) levels; total coenzyme Q (CoQ^{tot}) levels; oxidized cytochrome C levels; reduced cytochrome C levels; oxidized cytochrome C/reduced cytochrome C ratio; acetoacetate levels; beta-hydroxy butyrate levels; acetoacetate/ beta-hydroxy butyrate ratio; 8-hydroxy-2'-deoxyguanosine (8-OHdG) levels; levels of reactive oxygen species; oxygen consumption (VO₂), carbon dioxide output (VCO₂), and respiratory quotient (VCO₂/VO₂).

6. The peptide for use according to any one of claims 1-5, wherein the peptide is intended to be administered separately, sequentially, or simultaneously with an additional therapeutic agent.

7. The peptide for use according to claim 6, wherein the additional therapeutic agent is selected from the group consisting of: creatine, L-carnitine, coenzyme Q₁₀, L-arginine, biotin, cytochrome c, corticosteroids, idebenone, sodium dichloroacetate, thiamine, thiocitic acid, riboflavin, α-tocopherol, succinate, ascorbate, menadione, naphthoquinone, and nicotinamide.

8. The peptide for use according to any one of claims 1-7, wherein the pharmaceutically acceptable salt comprises acetate, tartrate, trifluoroacetate, or hydrochloride salt.
